# EUROPEAN PATENT SPECIFICATION

(11) **EP 1 082 322 B1**
(45) Date of publication and mention of the grant of the patent: **28.01.2004**
(21) Application number: 99925181.2
(22) Date of filing: 03.06.1999
(51) Int. Cl.: C07D 413/14, C07D 413/12, A61K 31/42, A61K 31/44, A61K 31/50, A61K 31/505

(54) **OXAZOLIDINONE DERIVATIVES AS ANTIBIOTIC COMPOUNDS, PROCESS FOR THEIR PREPARATION AND PHARMACEUTICAL COMPOSITIONS CONTAINING THEM**
OXAZOLIDINON-DERIVATE ALS ANTIBIOTIKA, VERFAHREN ZU IHRER HERSTELLUNG UND DIESE DERIVATE ENTHALTENDE PHARMAZEUTISCHE ZUSAMMENSETZUNGEN
DERIVES D'OXAZOLIDINONE COMME ANTIBIOTIQUES, LEUR PROCEDE DE PREPARATION ET COMPOSITIONS PHARMACEUTIQUES CONTENANT CEUX-CI

(30) Priority: 05.06.1998 GB 9812019
(43) Date of publication of application: 14.03.2001
(60) Divisional application: 03012958.9
(73) Proprietor: AstraZeneca AB, 151 85 Södertälje (SE)
(72) Inventor: GRAVESTOCK, Michael Barry, Macclesfield, Cheshire SK10 4TG (GB)
(74) Representative: Bryant, Tracey
(86) International application number: PCT/GB1999/001737
(87) International publication number: WO 1999/064416

(56) References cited:
- EP-A- 0 645 376
- EP-A- 0 710 657
- WO-A-93/22298
- WO-A-93/23384
- WO-A-94/22857
- WO-A-97/06791
- WO-A-97/09328
- CHUNG-HO PARK ET AL: "ANTIBACTERIALS. SYNTHESIS AND STRUCTURE-ACTIVITY STUDIES OF 3-ARYL-2-OXOOXAZOLIDINES. 4 MULTIPLY-SUBSTITUTED ARYL DERIVATIVES" JOURNAL OF MEDICINAL CHEMISTRY,US,AMERICAN CHEMICAL SOCIETY. WASHINGTON, vol. 35, no. 6, 1992, page 1156-1165 XP000567006 ISSN: 0022-2623 cited in the application
- W A GREGORY ET AL: "Antibacterials. Synthesis and Structure-Activity Studies of 3-Aryl-2-oxooxazolidines. 2. The A Group" JOURNAL OF MEDICINAL CHEMISTRY,US,AMERICAN CHEMICAL SOCIETY. WASHINGTON, vol. 33, 1990, page 2569-2578 XP002082548 ISSN: 0022-2623 cited in the application

## Description

The present invention relates to antibiotic compounds and in particular to antibiotic compounds containing a substituted oxazolidinone ring. This invention further relates to processes for their preparation, to intermediates useful in their preparation, to their use as therapeutic agents and to pharmaceutical compositions containing them.

The international microbiological community continues to express serious concern that the evolution of antibiotic resistance could result in strains against which currently available antibacterial agents will be ineffective. In general, bacterial pathogens may be classified as either Gram-positive or Gram-negative pathogens. Antibiotic compounds with effective activity against both Gram-positive and Gram-negative pathogens are generally regarded as having a broad spectrum of activity. The compounds of the present invention are regarded primarily as effective against Gram-positive pathogens because of their particularly good activity against such pathogens.

Gram-positive pathogens, for example Staphylococci, Enterococci, Streptococci and mycobacteria, are particularly important because of the development of resistant strains which are both difficult to treat and difficult to eradicate from the hospital environment once established. Examples of such strains are methicillin resistant staphylococcus (MRSA), methicillin resistant coagulase negative staphylococci (MRCNS), penicillin resistant Streptococcus pneumoniae and multiply resistant Enterococcus faecium.

The major clinically effective antibiotic for treatment of such resistant Gram-positive pathogens is vancomycin. Vancomycin is a glycopeptide and is associated with nephrotoxicity and ototoxicity. Furthermore, and most importantly, antibacterial resistance to vancomycin and other glycopeptides is also appearing. This resistance is increasing at a steady rate rendering these agents less and less effective in the treatment of Gram-positive pathogens.

Certain antibacterial compounds containing an oxazolidinone ring have been described in the art (for example, Walter A. Gregory et al in J.Med.Chem. 1990, 33, 2569-2578 and Chung-Ho Park et al in J.Med.Chem. 1992, 35, 1156-1165). Such antibacterial oxazolidinone compounds with a 5-methylacetamide sidechain may be subject to mammalian peptidase metabolism. Furthermore, bacterial resistance to known antibacterial agents may develop, for example, by (i) the evolution of active binding sites in the bacteria rendering a previously active pharmacophore less effective or redundant, and/or (ii) the evolution of means to chemically deactivate a given pharmacophore. Therefore, there remains an ongoing need to find new antibacterial agents with a favourable pharmacological profile, in particular for compounds containing new pharmacophores.

EP 0645376A discloses adhesion receptor antagonists which are 1-phenyloxazolidin-2-one compounds, substituted at the 5-position of the oxazolidinone ring with phenyl or heteroaryl rings substituted with carboxylic acid derivatives.

We have discovered a class of antibiotic compounds containing a new class of substituted oxazolidinone ring which has useful activity against Gram-positive pathogens including MRSA and MRCNS and, in particular, against various strains exhibiting resistance to vancomycin and against E. faecium strains resistant to both aminoglycosides and clinically used β-lactams.

Accordingly the present invention provides a compound of the formula (I), or a pharmaceutically-acceptable salt thereof, 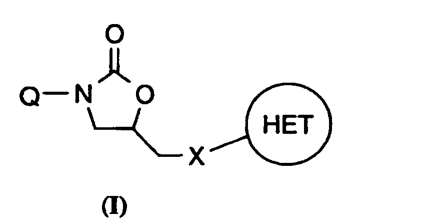 wherein X is -O- or -S-;
HET is a C-linked 6-membered heteroaryl ring containing 1 or 2 N, which ring is optionally substituted on any available C atom (provided that when the N atom is adjacent to the X-link, there is no substitution on any C atom that is adjacent to this N atom) by 1, 2 or 3 substituents independently selected from (1-4C)alkyl, amino, (1-4C)alkylamino, (1-4C)alkoxy, (1-4C)alkoxycarbonyl and halogen;
Q is Q1:- 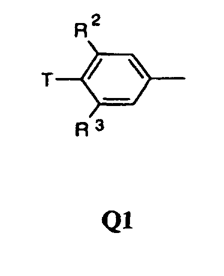 wherein R² and R³ are independently hydrogen or fluoro;
wherein T is selected from the groups in (TA) to (TC) below (wherein AR1, AR2, AR2a, AR2b, CY1 and CY2 are defined hereinbelow);
**(TA)** T is selected from the following groups :-
**(TAf)** an optionally substituted N-linked (fully unsaturated) 5-membered heteroaryl ring system containing 1, 2 or 3 nitrogen atoms selected from a group of formula (TAf1) to (TAf6) as set out hereinafter; or
**(TB)** T is selected from the following groups :-
**(TBa)** halo or (1-4C)alkyl
   {optionally substituted by one or more groups each independently selected from hydroxy, (1-4C)alkoxy, (1-4C)alkanoyl, cyano, halo, trifluoromethyl, (1-4C)alkoxycarbonyl, -NRvRw, (1-6C)alkanoylamino, (1-4C)alkoxycarbonylamino, N-(1-4C)alkyl-N-(1-6C)alkanoylamino, (1-4C)alkylS(O)_{q}- (q is 0, 1 or 2), CY1, CY2 or AR1};
**(TBb)** -NRv¹Rw¹ ;
**(TBc)** ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl;
**(TBd)** R¹⁰CO- , R¹⁰S(O)_{q}- (q is 0, 1 or 2) or R¹⁰CS-
   wherein R¹⁰ is selected from the following groups :-
   ***(TBda)*** CY1 or CY2;
   ***(TBdb)*** hydrogen, (1-4C)alkoxycarbonyl, trifluoromethyl, -NRvRw, ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl or 2-(AR2)ethenyl; or
   ***(TBdc)*** (1-4C)alkyl {optionally substituted as defined in (TBa) above, or by (1-4C)alkylS(O)ₚNH- or (1-4C)alkylS(O)ₚ-((1-4C)alkyl)N- (p is 1 or 2)};
wherein Rv is hydrogen or (1-4C)alkyl; Rw is hydrogen or (1-4C)alkyl; Rv¹ is hydrogen, (1-4C)alkyl or (3-8C)cycloalkyl; Rw¹ is hydrogen, (1-4C)alkyl, (3-8C)cycloalkyl, (1-4C)alkyl-CO- or (1-4C)alkylS(O)_{q}- (q is 1 or 2); or
(TC) T is selected from the groups (TC1) to (TC4) as set out hereinafter: or
wherein Rc is selected from groups (Rc1) to (Rc4) :-
***(Rc1)*** (1-6C)alkyl {optionally substituted by one or more (1-4C)alkanoyl groups (including geminal disubstitution) and/or optionally monosubstituted by cyano, (1-4C)alkoxy, trifluoromethyl, (1-4C)alkoxycarbonyl, phenyl (optionally substituted as for AR defined hereinafter), (1-4C)alkylS(O)_{q}- (q is 0, 1 or 2); or, on any but the first carbon atom of the (1-6C)alkyl chain, optionally substituted by one or more groups (including geminal disubstitution) each independently selected from hydroxy and fluoro, and/or optionally monosubstituted by oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is 1 or 2)};
***(Rc2)*** R¹³CO- , R¹³SO₂- or R¹³CS-
   wherein R¹³ is selected from (Rc2a) to (Rc2e) :-
   ***(Rc2a)*** AR1, AR2, AR2a, AR2b, AR3, AR3a, AR3b, AR4, AR4a, CY1, CY2;
   ***(Rc2b)*** hydrogen, (1-4C)alkoxycarbonyl, trifluoromethyl, -NRvRw [wherein Rv is hydrogen or (1-4C)alkyl; Rw is hydrogen or (1-4C)alkyl), ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, 2-(AR2a)ethenyl;
   ***(Rc2c)*** (1-10C)alkyl
      {optionally substituted by one or more groups (including geminal disubstitution) each independently selected from hydroxy, (1-10C)alkoxy, (1-4C)alkoxy-(1-4C)alkoxy, (1-4C)alkoxy-(1-4C)alkoxy-(1-4C)alkoxy, (1-4C)alkanoyl and amino; and/or optionally substituted by one group selected from phosphonate [phosphono, -P(O)(OH)₂, and mono- and di-(1-4C)alkoxy derivatives thereof], phosphinate [-P(OH)₂ and mono- and di-(1-4C)alkoxy derivatives thereof], phosphoryl [-O-P(O)(OH)₂, and mono- and di-(1-4C)alkoxy derivatives thereof], phosphiryl [-O-P(OH)₂ and mono- and di-(1-4C)alkoxy derivatives thereof], cyano, halo, trifluoromethyl, (1-4C)alkoxycarbonyl, (1-4C)alkoxy-(1-4C)alkoxycarbonyl, (1-4C)alkoxy-(1-4C)alkoxy-(1-4C)alkoxycarbonyl, (1-4C)alkylamino, di((1-4C)alkyl)amino, (1-6C)alkanoylamino, (1-4C)alkoxycarbonylamino, N-(1-4C)alkyl-N-(1-6C)alkanoylamino, (1-4C)alkylaminocarbonyl, di((1-4C)alkyl)aminocarbonyl, (1-4C)alkylS(O)ₚNH-, (1-4C)alkylS(O)ₚ-((1-4C)alkyl)N-, fluoro(1-4C)alkylS(O)ₚNH-, fluoro(1-4C)alkylS(O)ₚ((1-4C)alkyl)N-, (1-4C)alkylS(O)_{q}-, CY1, CY2, AR1, AR2, AR1-O-, AR2-O-, AR1-S(O)_{q}- , AR2-S(O)_{q}-, AR1-NH-, AR2-NH- (p is 1 or 2 and q is 0, 1 or 2), and also AR2a, AR2b versions of AR2 containing groups};
   ***(Rc2d)*** R¹⁴C(O)O(1-6C)alkyl wherein R¹⁴ is AR1, AR2, (1-4C)alkylamino, benzyloxy(1-4C)alkyl or (1-10C)alkyl {optionally substituted as defined for (Rc2c)};
   ***(Rc2e)*** R¹⁵O- wherein R¹⁵ is benzyl, (1-6C)alkyl {optionally substituted as defined for (Rc2c)}, CY1, CY2 or AR2b;
   ***(Rc4)*** trityl, AR1, AR2, AR2a, AR2b;
wherein
**AR1** is an optionally substituted phenyl or optionally substituted naphthyl;
**AR2** is as defined hereinafter;
**AR2a** is as defined hereinafter;
**AR2b** is as defined hereinafter;
**CY1** is an optionally substituted cyclobutyl, cyclopentyl or cyclohexyl ring;
**CY2** is an optionally substituted cyclopentenyl or cyclohexenyl ring.

In this specification, where it is stated that a ring may be linked via an sp² carbon atom, which ring is fully saturated other than (where appropriate) at a linking sp² carbon atom, it is to be understood that the ring is linked via a C=C double bond.

In another emdodiment, (Rc1) is as defined above other than the optional phenyl substituent on (1-6C)alkyl is optionally substituted as for AR1 defined hereinafter; and (Rc2c), is as defined above and further includes carboxy as an optional substituent on R¹³ as (1-10C)alkyl.
**(TAf)** is an optionally substituted N-linked (fully unsaturated) 5-membered heteroaryl ring system containing 1, 2 or 3 nitrogen atoms selected from a group of formula (TAf1) to (TAf6) below: 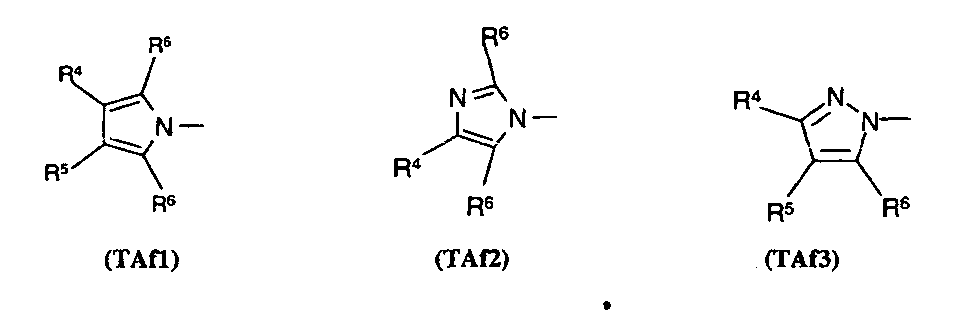 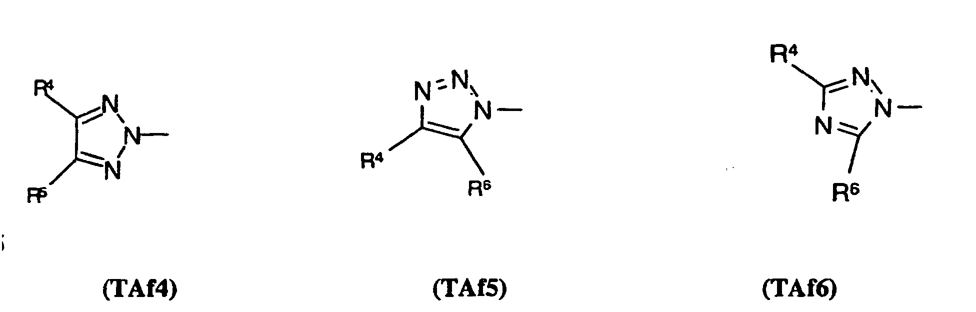 wherein :
R⁶ is selected (independently where appropriate) from hydrogen, (1-4C)alkyl, (1-4C)alkoxycarbonyl, (1-4C)alkanoyl, carbamoyl and cyano;
R⁴ and R⁵ are independently selected from hydrogen, halo, trifluoromethyl, cyano, nitro, (1-4C)alkoxy, (1-4C)alkylS(O)_{q}- (q is 0, 1 or 2), (1-4C)alkanoyl, (1-4C)alkoxycarbonyl, (2-4C)alkanoyloxy-(1-4C)alkyl, benzoxy-(1-4C)alkyl, (2-4C)alkanoylamino, -CONRvRw, -NRvRw and (1-4C)alkyl {optionally substituted by hydroxy, trifluoromethyl, cyano, nitro, (1-4C)alkoxy, (1-4C)alkylS(O)_{q}- (q is 0, 1 or 2), (1-4C)alkoxycarbonyl, (1-4C)alkanoylamino, - CONRvRw, -NRvRw; wherein RvRw is hydrogen or (1-4C)alkyl; Rw is hydrogen or (1-4C)alkyl}.

Preferably R⁶ is hydrogen or (1-4C)alkyl, and R⁴ and R⁵ are independently selected from hydrogen, (1-4C)alkyl. Other preferable substituents on the (TAf1) to (TAf6) are illustrated in the accompanying Examples.
(TC) T is selected from the groups (TC1) to (TC4) :- 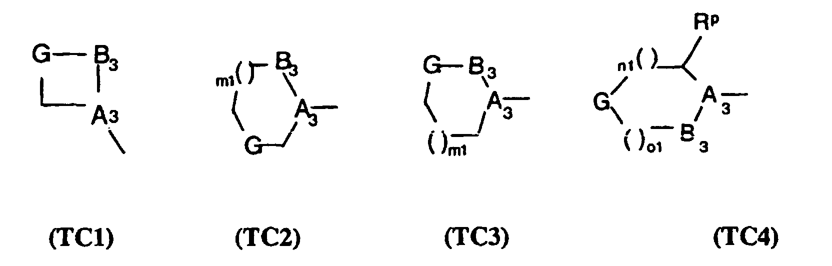 wherein in (TC1) : >A₃-B₃- is >C(Rq)-CH(Rr)- and G is -O-, -S-, -SO-, -SO₂- or >N(Rc);
wherein in (TC2) : m1 is 0, 1 or 2; >A₃-B₃- is >C=C(Rr)- or >C(Rq)-CH(Rr)- and G is -O-, - S-, -SO-, -SO₂- or >N(Rc);
wherein in (TC3) : m1 is 0, 1 or 2; >A₃-B₃- is >C(Rq)-CH(Rr)- (other than when Rq and Rr are both together hydrogen) and G is -O-, -S-, -SO-, -SO₂- or >N(Rc);
wherein in (TC4) : n1 is 1 or 2; o1 is 1 or 2 and n1 + o1 = 2 or 3; >A₃-B₃- is >C=C(Rr)- or >C(Rq)-CH(Rr)- or >N-CH₂- and G is -O-, -S-, -SO-, -SO₂- or >N(Rc); Rp is hydrogen, (1-4C)alkyl (other than when such substitution is defined by >A₃-B₃-), hydroxy, (1-4C)alkoxy or (1-4C)alkanoyloxy;
wherein in (TC1), (TC2) and (TC4); m1, n1 and o1 are as defined hereinbefore :
>A₃-B₃- is >N-CH₂- and G is >C(R¹¹)(R¹²), >C=O, >C-OH, >C-(1-4C)alkoxy, >C=N-OH, >C=N-(1-4C)alkoxy, >C=N-NH-(1-4C)alkyl, >C=N-N((1-4C)alkyl)₂ (the last two (1-4C)alkyl groups above in G being optionally substituted by hydroxy) or >C=N-N-CO-(1-4C)alkoxy; wherein > represents two single bonds;
Rq is hydrogen, hydroxy, halo, (1-4C)alkyl or (1-4C)alkanoyloxy;
Rr is (independently where appropriate) hydrogen or (1-4C)alkyl;
R¹¹ is hydrogen, (1-4C)alkyl, fluoro(1-4C)alkyl, (1-4C)alkyl-thio-(1-4C)alkyl or hydroxy-(1-4C)alkyl and R¹² is -[C(Rr)(Rr)]ₘ₂-N(Rr)(Rc) wherein m2 is 0, 1 or 2;
   and, other than the ring substitution defined by G, >A₃-B₃- and Rp, each ring system may be optionally further substituted on a carbon atom not adjacent to the link at >A₃- by up to two substituents independently selected from (1-4C)alkyl, fluoro(1-4C)alkyl (including trifluoromethyl), (1-4C)alkyl-thio-(1-4C)alkyl, hydroxy-(1-4C)alkyl, amino, amino-(1-4C)alkyl, (1-4C)alkanoylamino, (1-4C)alkanoylamino-(1-4C)alkyl, carboxy, (1-4C)alkoxycarbonyl, AR-oxymethyl, AR-thiomethyl, oxo (=O) (other than when G is >N-Rc and Rc is group (Rc2) defined hereinbefore) or independently selected from Rc; and also hydroxy or halo (the last two optional substituents only when G is -O- or -S-);
wherein AR is as defined hereinafter; Rc is selected from groups (Rc1) to (Rc5) defined hereinbefore.

For the avoidance of doubt, ( )ₘ₁, ( )ₙ₁ and ( )ₒ₁ indicate (-CH₂-)ₘ₁, (-CH₂-)ₙ₁ and (-CH₂-)ₒ₁ respectively (optionally substituted as described above).

In the above definition of (TC1) to (TC4) and of the further optional substituents, AR is preferably AR2, and the further optional substituents are preferably not selected from the values listed for Rc. A preferred value for G is >N(Rc) or >C(R¹¹)(R¹²).

Particularly preferred values for the optional substituents and groups defined in (TC1) to (TC4) are contained in the following definitions (TC5) to (TC11) :- 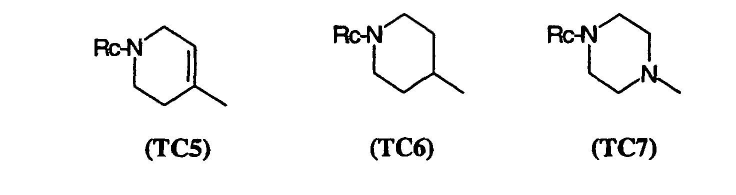 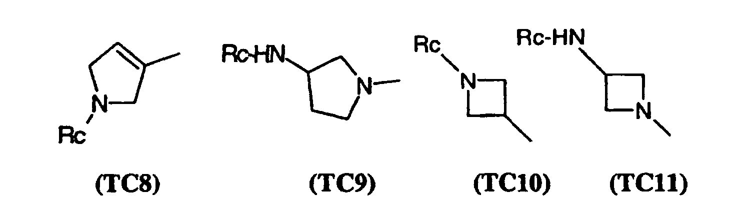 wherein Rc has any of the values listed hereinbefore or hereinafter.

Especially preferred are (TC5), (TC6), (TC7) and (TC9), most especially (TC5) in which Rc has any of the values listed hereinbefore or hereinafter (especially R¹³CO- with the preferable R¹³ values given hereinafter). In (TCS) Rc is preferably selected from the group (Rc2), especially R¹³CO- with the preferable R¹³ values given hereinafter. In (TC7) Rc is preferably selected from group (Rc3) or (Rc4).

The above preferred values of TC are particularly preferred when present in Q1 and when X is -O-.

AR is optionally substituted phenyl, optionally substituted phenyl(1-4C)alkyl, optionally substituted naphthyl, optionally substituted 5- or 6-membered heteroaryl, wherein 1, 2, or 3 of the ring atoms are selected from nitorgen, oxygen and sulfur and the rings are fully aromatic; an optionally substituted 5/6 or 6/6 bicyclic heteroaryl ring system, comprising a 6-membered ring fused to either a 5-membered ring or another 6-membered ring, the bicyclcic ring system containing 1 to 4 heteroatoms selected from nitrogen, oxygen and sulfur, in which the bicyclic heteroaryl ring systems may be linked via an atom in either of the rings comprising the bicyclic system, and wherein both the mono- and bicyclic heteroaryl ring systems are linked via a ring carbon atom and may be (partially) hydrogenated.

For the avoidance of doubt, phosphono is -P(O)(OH)₂; (1-4C)alkoxy(hydroxy)-phosphoryl is a mono-(1-4C)alkoxy derivative of -O-P(O)(OH)₂; and di-(1-4C)alkoxyphosphoryl is a di-(1-4C)alkoxy derivative of -O-P(O)(OH)₂.

Particular examples of 5- or 6-membered heteroaryl ring systems are furan, pyrrole, pyrazole, imidazole, triazole, pyrimidine, pyridazine, pyridine, isoxazole, oxazole, isothiazole, thiazole and thiophene.

Particular examples of 5/6 and 6/6 bicyclic ring systems are indole, benzofuran, benzimidazole, benzothiophene, benzisothiazole, benzoxazole, benzisoxazole, pyridoimidazole, pyrimidoimidazole, quinoline, quinoxaline, quinazoline, phthalazine, cinnoline and naphthyridine.

AR is optionally mono- or di-substituted with halo, (1-4C)alkyl , hydroxy, nitro, carbamoyl, (1-4C)alkylcarbamoyl, di-((1-4C)alkyl)carbamoyl, cyano, trifluoromethyl, trifluoromethoxy, amino, (1-4C)alkylamino, di((1-4C)alkyl)amino, (1-4C)alkyl S(O)_{q}- (q is 0, 1 or 2), carboxy, (1-4C)alkoxycarbonyl, (2-4C)alkenyl, (2-4C)alkynyl, (1-4C)alkanoyl, (1-4C)alkoxy, (1-4C)alkylS(O)₂amino, (1-4C)alkanoylamino, benzoylamino, benzoyl, phenyl (optionally substituted by up to three substituents selected from halo, (1-4C)alkoxy or cyano), furan, pyrrole, pyrazole, imidazole, triazole, pyrimidine, pyridazine, pyridine, isoxazole, oxazole, isothiazole, thiazole, thiophene, hydroxyimino(1-4C)alkyl, (1-4C)alkoxyimino(1-4C)alkyl, hydroxy-(1-4C)alkyl, halo-(1-4C)alkyl, nitro(1-4C)alkyl, amino(1-4C)alkyl, cyano(1-4C)alkyl, (1-4C)alkanesulfonamido, aminosulfonyl, (1-4C)alkylaminosulfonyl and di-((1-4C)alkyl)aminosulfonyl.

In this specification the term 'alkyl' includes straight chained and branched structures. For example, (1-6C)alkyl includes propyl, isopropyl and tert-butyl. However, references to individual alkyl groups such as "propyl" are specific for the straight chained version only, and references to individual branched chain alkyl groups such as "isopropyl" are specific for the branched chain version only. A similar convention applies to other radicals, for example halo(1-4C)alkyl includes 1-bromoethyl and 2-bromoethyl.

There follow particular and suitable values for certain substituents and groups referred to in this specification. These values may be used where appropriate with any of the definitions and embodiments disclosed hereinbefore, or hereinafter.

Examples of **(1-4C)alkyl** and **(1-5C)alkyl** include methyl, ethyl, and propyl and isopropyl; examples of **(1-6C)alkyl** include methyl, ethyl, propyl, isopropyl, pentyl and hexyl; examples of **(1-10C)alkyl** include methyl, ethyl, propyl, isopropyl, pentyl, hexyl, heptyl, octyl and nonyl; examples of **(1-4C)alkanoylamino-(1-4C)alkyl** include formamidomethyl, acetamidomethyl and acetamidoethyl; examples of **hydroxy(1-4C)alkyl** and **hydroxy(1-6C)alkyl** include hydroxymethyl, 1-hydroxyethyl, 2-hydroxyethyl and 3-hydroxypropyl; examples of **(1-4C)alkoxycarbonyl** include methoxycarbonyl, ethoxycarbonyl and propoxycarbonyl; examples of **2-((1-4C)alkoxycarbonyl)ethenyl** include 2-(methoxycarbonyl)ethenyl and 2-(ethoxycarbonyl)ethenyl; examples of **2-cyano-2-((1-4C)alkyl)ethenyl** include 2-cyano-2-methylethenyl and 2-cyano-2-ethylethenyl; examples of **2-nitro-2-((1-4C)alkyl)ethenyl** include 2-nitro-2-methylethenyl and 2-nitro-2-ethylethenyl; examples of **2-((1-4C)alkylaminocarbonyl)ethenyl** include 2-(methylaminocarbonyl)ethenyl and 2-(ethylaminocarbonyl)ethenyl; examples of **(2-4C)alkenyl** include allyl and vinyl; examples of **(2-4C)alkynyl** include ethynyl and 2-propynyl; examples of **(1-4C)alkanoyl** include formyl, acetyl and propionyl; examples of **(1-4C)alkoxy** include methoxy, ethoxy and propoxy; examples of **(1-6C)alkoxy** and **(1-10C)alkoxy** include methoxy, ethoxy, propoxy and pentoxy; examples of **(1-4C)alkylthio** include methylthio and ethylthio; examples **of (1-4C)alkylamino** include methylamino, ethylamino and propylamino; examples of **di-((1-4C)alkyl)amino** include dimethylamino, N-ethyl-N-methylamino, diethylamino, N-methyl-N-propylamino and dipropylamino; examples of **halo** groups include fluoro, chloro and bromo; examples of **(1-4C)alkylsulfonyl** include methylsulfonyl and ethylsulfonyl; examples of **(1-4C)alkoxy-(1-4C)alkoxy** and **(1-6C)alkoxy-(1-6C)alkoxy** include methoxymethoxy, 2-methoxyethoxy, 2-ethoxyethoxy and 3-methoxypropoxy; examples of **(1-4C)alkoxy-(1-4C)alkoxy-(1-4C)alkoxy** include 2-(methoxymethoxy)ethoxy, 2-(2-methoxyethoxy)ethoxy; 3-(2-methoxyethoxy)propoxy and 2-(2-ethoxyethoxy)ethoxy; examples of **(1-4C)alkylS(O)**_{**2**}**amino** include methylsulfonylamino and ethylsulfonylamino; examples of **(1-4C)alkanoylamino** and **(1-6C)alkanoylamino** include formamido, acetamido and propionylamino; examples of **(1-4C)alkoxycarbonylamino** include methoxycarbonylamino and ethoxycarbonylamino; examples of **N-(1-4C)alkyl-N-(1-6C)alkanoylamino** include N-methylacetamido, N-ethylacetamido and N-methylpropionamido; examples of **(1-4C)alkylS(O)**_{**p**}**NH-** wherein p is 1 or 2 include methylsulfinylamino, methylsulfonylamino, ethylsulfinylamino and ethylsulfonylamino; examples of **(1-4C)alkylS(O)**_{**p**}**((1-4C)alkyl)N-** wherein p is 1 or 2 include methylsulfinylmethylamino, methylsulfonylmethylamino, 2-(ethylsulfinyl)ethylamino and 2-(ethylsulfonyl)ethylamino; examples of **fluoro(1-4C)alkylS(O)**_{**p**}**NH-** wherein p is 1 or 2 include trifluoromethylsulfinylamino and trifluoromethylsulfonylamino; examples of **fluoro(1-4C)alkylS(O)**_{**p**}**((1-4C)alkyl)NH-** wherein p is 1 or 2 include trifluoromethylsulfinylmethylamino and trifluoromethylsulfonylmethylamino examples of (1-**4C)alkoxy(hydroxy)phosphoryl** include methoxy(hydroxy)phosphoryl and ethoxy(hydroxy)phosphoryl; examples of **di-(1-4C)alkoxyphosphoryl** include dimethoxyphosphoryl, di-ethoxyphosphoryl and ethoxy(methoxy)phosphoryl; examples of **(1-4C)alkylS(O)**_{**q**}**-** wherein q is 0, 1 or 2 include methylthio, ethylthio, methylsulfinyl, ethylsulfinyl, methylsulfonyl and ethylsulfonyl; examples of **phenylS(O)**_{**q**} and **naphthylS(O)**_{**q**}**-** wherein q is 0, 1 or 2 are phenylthio, phenylsulfinyl, phenylsulfonyl and naphthylthio, naphthylsulfinyl and naphthylsulfonyl respectively; examples of **benzyloxy-(1-4C)alkyl** include benzyloxymethyl and benzyloxyethyl; examples of a **(3-4C)alkylene** chain are trimethylene or tetramethylene; examples of **(1-6C)alkoxy-(1-6C)alkyl** include methoxymethyl, ethoxymethyl and 2-methoxyethyl; examples of **hydroxy-(2-6C)alkoxy** include 2-hydroxyethoxy and 3-hydroxypropoxy; examples of **(1-4C)alkylamino-(2-6C)alkoxy** include 2-methylaminoethoxy and 2-ethylarninoethoxy; examples of **di-(1-4C)alkylamino-(2-6C)alkoxy** include 2-dimethylaminoethoxy and 2-diethylaminoethoxy; examples of **phenyl(1-4C)alkyl** include benzyl and phenethyl; examples of **(1-4C)alkylcarbamoyl** include methylcarbamoyl and ethylcarbamoyl; examples of **di((1-4C)alkyl)carbamoyl** include di(methyl)carbamoyl and di(ethyl)carbamoyl; examples of **hydroxyimino(1-4C)alkyl** include hydroxyiminomethyl, 2-(hydroxyimino)ethyl and 1-(hydroxyimino)ethyl; examples of **(1-4C)alkoxyimino-(1-4C)alkyl** include methoxyiminomethyl, ethoxyiminomethyl, 1-(methoxyimino)ethyl and 2-(methoxyimino)ethyl; examples of **halo(1-4C)alkyl** include, halomethyl, 1-haloethyl, 2-haloethyl, and 3-halopropyl; examples of **nitro(1-4C)alkyl** include nitromethyl, 1-nitroethyl, 2-nitroethyl and 3-nitropropyl; examples of **amino(1-4C)alkyl** include aminomethyl, 1-aminoethyl, 2-aminoethyl and 3-aminopropyl; examples of **cyano(1-4C)alkyl** include cyanomethyl, 1-cyanoethyl, 2-cyanoethyl and 3-cyanopropyl; examples of **(1-4C)alkanesulfonamido** include methanesulfonamido and ethanesulfonamido; examples of **(1-4C)alkylaminosulfonyl** include methylaminosulfonyl and ethylaminosulfonyl; and examples of **di-(1-4C)alkylaminosulfonyl** include dimethylaminosulfonyl, diethylaminosulfonyl and N-methyl-N-ethylaminosulfonyl; examples of **(1-4C)alkanesulfonyloxy** include methylsulfonyloxy, ethylsulfonyloxy and propylsulfonyloxy; examples of **(1-4C)alkanoyloxy** include acetoxy; examples of **(1-4C)alkylaminocarbonyl** include methylaminocarbonyl and ethylaminocarbonyl; examples of **di((1-4C)alkyl)aminocarbonyl** include dimethylaminocarbonyl and diethylaminocarbonyl; examples of **(3-8C)cycloalkyl** include cyclopropyl, cyclobutyl, cyclopentyl and cyclohexyl; examples of **(4-7C)cycloalkyl** include cyclobutyl, cyclopentyl and cyclohexyl; examples of **di(N-(1-4C)alkyl)aminomethylimino** include dimethylaminomethylimino and diethylaminomethylimino.

AR2 is selected from furan, pyrrole, thiophene, pyrazole, imidazole, pyridine, pyrimidine, pyrazine, pyridazine, 1,2,3- & 1,2,4-triazole, tetrazole, oxazole, isoxazole, oxazine, thiazole, isothiazole, 1,2,4- and 1,3,4-thiadiazole.

AR2a is selected from dihydropyrrole and tetrahydropyridine.

AR2b is selected from tetrahydrofuran, pyrrolidine, morpholine, thiomorpholine, piperazine, imidazoline, piperidine, 1,3-dioxolan-4-yl, 1,3-dioxan-4-yl, 1,3-dioxan-5-yl and 1,4-dioxan-2-yl.

The nomenclature used is that found in, for example, "Heterocyclic Compounds (Systems with bridgehead nitrogen), W.L.Mosby (Intercsience Publishers Inc., New York), 1961, Parts 1 and 2.

Where optional substituents are listed such substitution is preferably not geminal disubstitution unless stated otherwise. If not stated elsewhere suitable optional substituents for a particular group are those as stated for similar groups herein.

Suitable substituents on AR1, AR2, AR2a, AR2b, CY1 and CY2 are (on an available carbon atom) up to three substituents independently selected from (1-4C)alkyl {optionally substituted by (preferably one) substituents selected independently from hydroxy, trifluoromethyl, (1-4C)alkyl S(O)_{q}- (q is 0, 1 or 2) (this last substituent preferably on AR1 only), (1-4C)alkoxy, (1-4C)alkoxycarbonyl, cyano, nitro, (1-4C)alkanoylamino, -CONRvRw or -NRvRw}, trifluoromethyl, hydroxy, halo, nitro, cyano, thiol, (1-4C)alkoxy, (1-4C)alkanoyloxy, dimethylaminomethyleneaminocarbonyl, di(N-(1-4C)alkyl)aminomethylimino, carboxy, (1-4C)alkoxycarbonyl, (1-4C)alkanoyl, (1-4C)alkylSO₂amino, (2-4C)alkenyl {optionally substituted by carboxy or (1-4C)alkoxycarbonyl}, (2-4C)alkynyl, (1-4C)alkanoylamino, oxo (=O), thioxo (=S), (1-4C)alkanoylamino {the (1-4C)alkanoyl group being optionally substituted by hydroxy}, (1-4C)alkyl S(O)_{q}- (q is 0, 1 or 2) {the (1-4C)alkyl group being optionally substituted by one or more groups independently selected from cyano, hydroxy and (1-4C)alkoxy}, -CONRvRw or - NRvRw [wherein Rv is hydrogen or (1-4C)alkyl; Rw is hydrogen or (1-4C)alkyl].

Further suitable substituents on AR1, AR2, AR2a, AR2b, CY1 and CY2 (on an available carbon atom), and also on alkyl groups (unless indicated otherwise) are up to three substituents independently selected from trifluoromethoxy, benzoylamino, benzoyl, phenyl {optionally substituted by up to three substituents independently selected from halo, (1-4C)alkoxy or cyano}, furan, pyrrole, pyrazole, imidazole, triazole, pyrimidine, pyridazine, pyridine, isoxazole, oxazole, isothiazole, thiazole, thiophene, hydroxyimino(1-4C)alkyl, (1-4C)alkoxyimino(1-4C)alkyl, halo-(1-4C)alkyl, (1-4C)alkanesulfonamido, -SO₂NRvRw [wherein Rv is hydrogen or (1-4C)alkyl; Rw is hydrogen or (1-4C)alkyl].

Preferable optional substituents on Ar2b as 1,3-dioxolan-4-yl, 1,3-dioxan-4-yl, 1,3-dioxan-5-yl or 1,4-dioxan-2-yl are mono- or disubstitution by substituents independently selected from (1-4C)alkyl (including geminal disubstitution), (1-4C)alkoxy, (1-4C)alkylthio, acetamido, (1-4C)alkanoyl, cyano, trifluoromethyl and phenyl]._

Preferable optional substituents on CY1 & CY2 are mono- or disubstitution by substituents independently selected from (1-4C)alkyl (including geminal disubstitution), hydroxy, (1-4C)alkoxy, (1-4C)alkylthio, acetamido, (1-4C)alkanoyl, cyano, and trifluoromethyl.

Suitable substituents on AR2, AR2a, AR2b are (on an available nitrogen atom, where such substitution does not result in quatemization) (1-4C)alkyl, (1-4C)alkanoyl {wherein the (1-4C)alkyl and (1-4C)alkanoyl groups are optionally substituted by (preferably one) substituents independently selected from cyano, hydroxy, nitro, trifluoromethyl, (1-4C)alkyl S(O)_{q}- (q is 0, 1 or 2), (1-4C)alkoxy, (1-4C)alkoxycarbonyl, (1-4C)alkanoylamino, -CONRvRw or -NRvRw [wherein Rv is hydrogen or (1-4C)alkyl; Rw is hydrogen or (1-4C)alkyl]}, (2-4C)alkenyl, (2-4C)alkynyl, (1-4C)alkoxycarbonyl or oxo (to form an N-oxide).

Suitable pharmaceutically-acceptable salts include acid addition salts such as methanesulfonate, fumarate, hydrochloride, citrate, maleate, tartrate and (less preferably) hydrobromide. Also suitable are salts formed with phosphoric and sulfuric acid. In another aspect suitable salts are base salts such as an alkali metal salt for example sodium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine, tris-(2-hydroxyethyl)amine, N-methyl d-glucamine and amino acids such as lysine. There may be more than one cation or anion depending on the number of charged functions and the valency of the cations or anions. A preferred pharmaceutically-acceptable salt is the sodium salt.

However, to facilitate isolation of the salt during preparation, salts which are less soluble in the chosen solvent may be preferred whether pharmaceutically-acceptable or not.

The compounds of the present invention have a chiral centre at the C-5 position of the oxazolidinone ring. The pharmaceutically active enantiomer is of the formula (IA):

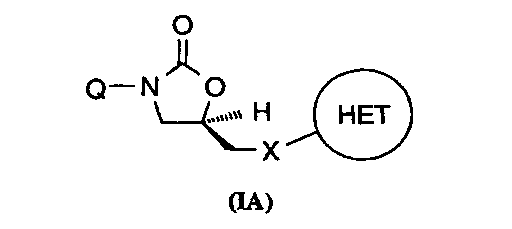

The present invention includes the pure enantiomer depicted above or mixtures of the 5R and 5S enantiomers, for example a racemic mixture. If a mixture of enantiomers is used, a larger amount (depending upon the ratio of the enantiomers) will be required to achieve the same effect as the same weight of the pharmaceutically active enantiomer. For the avoidance of doubt the enantiomer depicted above is the 5R enantiomer.

Furthermore, some compounds of the formula (I) may have other chiral centres. It is to be understood that the invention encompasses all such optical and diastereo-isomers, and racemic mixtures, that possess antibacterial activity. It is well known in the art how to prepare optically-active forms (for example by resolution of the racemic form by recrystallisation techniques, by chiral synthesis, by enzymatic resolution, by biotransformation or by chromatographic separation) and how to determine antibacterial activity as described hereinafter.

The invention relates to all tautomeric forms of the compounds of the formula (I) that possess antibacterial activity.

It is also to be understood that certain compounds of the formula (I) can exist in solvated as well as unsolvated forms such as, for example, hydrated forms. It is to be understood that the invention encompasses all such solvated forms which possess antibacterial activity.

It is also to be understood that certain compounds of the formula (I) may exhibit polymorphism, and that the invention encompasses all such forms which possess antibacterial activity.

As stated before, we have discovered a range of compounds that have good activity against a broad range of Gram-positive pathogens including organisms known to be resistant to most commonly used antibiotics. Physical and/or pharmacokinetic properties, for example increased stability to mammalian peptidase metabolism and a favourable toxicological profile are important features. The following compounds possess particularly favourable physical and/or pharmacokinetic properties and are preferred.

Particularly preferred compounds of the invention comprise a compound of formula (I), or a pharmaceutically-acceptable salt, wherein the substituents Q, X, HET, T and other substituents mentioned above have values disclosed hereinbefore, or any of the following values (which may be used where appropriate with any of the definitions and embodiments disclosed hereinbefore or hereinafter):

Preferably T is selected from (TAf), or (TC); more particularly (TC2), (TC3) and (TC4); and most preferably (TC5), (TC7) or (TC9), and most particularly (TC5).

Preferable values for other substituents (which may be used where appropriate with any of the definitions and embodiments disclosed hereinbefore or hereinafter) are :-
(a) Preferably X is -O-;
   (a1) In another aspect X is -S-;
   (a2) In another embodiment X can also be -SO- or -SO₂-;
(b) Preferably HET is pyridine, pyridazine or pyrazine; more preferably HET is pyridin-2-yl, pyridazin-3-yl or pyrazin-2-yl;
   (b1) Preferably HET is unsubstituted;
(c) Preferably one of R² and R³ is hydrogen and the other fluoro;
(d) In another aspect both R² and R³ are fluoro;
(e) Preferably AR is 5- or 6-membered heteroaryl; more preferably AR is 6-membered heteroaryl, such as pyridinyl;
(f) Preferred substituents for phenyl and carbon atoms in heteroaryl (mono- and bicyclic) ring systems in AR include halo, (1-4C)alkyl, hydroxy, nitro, amino, cyano, (1-4C)alkylS(O)ₚ- and (1-4C)alkoxy;
(g) Preferably the optionally substituted ring systems in AR are unsubstituted;

In the definition of (Rc2c) the AR2a, AR2b versions of AR2 containing groups are preferably excluded.

Especially preferred compounds of the present invention are of the formula (IB): 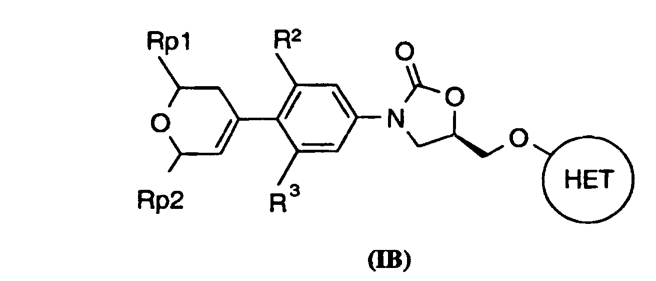 wherein HET is pyridin-2-yl or pyrazin-2-yl (especially pyridin-2-yl); R² and R³ are independently hydrogen or fluoro; and Rp1 and Rp2 are independently hydrogen, hydroxy, bromo, (1-4C)alkyl, carboxy, (1-4C)alkoxycarbonyl, hydroxymethyl, (1-4C)alkoxymethyl or carbamoyl; or pharmaceutically-acceptable salts thereof.

Further especially preferred compounds of the invention are of the formula (IB) wherein HET is pyridin-2-yl or pyrazin-2-yl (especially pyridin-2-yl); R² and R³ are independently hydrogen or fluoro; and Rp1 and Rp2 are independently hydrogen, AR-oxymethyl or AR-thiomethyl (wherein AR is phenyl, phenyl-(1-4C)alkyl, naphthyl, furan, pyrrole, pyrazole, imidazole, triazole, pyrimidine, pyridazine, pyridine, isoxazole, oxazole, isothiazole, thiazole or thiophene); or pharmaceutically-acceptable salts thereof.

Of the above especially preferred compounds of the invention of the formula (IB), particularly preferred compounds are those wherein Rp1 and Rp2 are hydrogen are particularly preferred.

Further, especially preferred compounds of the invention are of the formula (IC): 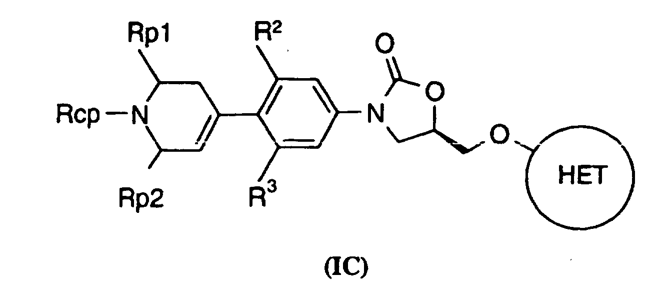 wherein HET is pyridin-2-yl or pyrazin-2-yl (especially pyridin-2-yl); R² and R³ are independently hydrogen or fluoro; Rp1 and Rp2 are independently hydrogen, AR-oxymethyl or AR-thiomethyl (wherein AR is phenyl, phenyl-(1-4C)alkyl, naphthyl, furan, pyrrole, pyrazole, imidazole, triazole, pyrimidine, pyridazine, pyridine, isoxazole, oxazole, isothiazole, thiazole or thiophene), (1-4C)alkyl, carboxy, (1-4C)alkoxycarbonyl, hydroxymethyl, (1-4C)alkoxymethyl or carbamoyl and Rcp is cyano, pyrimidin-2-yl, 2-cyanoethenyl, 2-cyano-2-((1-4C)alkyl)ethenyl or Rcp is of the formula R^{13p}CO-, R^{13p}SO₂- or R^{13p}CS- (wherein R^{13p} is hydrogen, (1-5C)alkyl [optionally substituted by one or more groups each independently selected from hydroxy and amino, or optionally monosubstituted by (1-4C)alkoxy, (1-4C)alkylS(O)_{q}-, (1-4C)alkylamino, (1-4C)alkanoyl, naphthoxy, (2-6C)alkanoylamino or (1-4C)alkylS(O)ₚNH- wherein p is 1 or 2 and q is 0, 1 or 2], imidazole, triazole, pyrimidine, pyridazine, pyridine, isoxazole, oxazole, isothiazole, thiazole, pyridoimidazole, pyrimidoimidazole, quinoxaline, quinazoline, phthalazine, cinnoline or naphthyridine, or R^{13p} is of the formula R^{14p}C(O)O(1-6C)alkyl wherein R^{14p} is (1-6C)alkyl), or Rcp is of the formula RfC(=O)C(=O)- wherein Rf is (1-6C)alkoxy; or pharmaceutically-acceptable salts thereof.

Of the above especially preferred compounds of the invention of the formula (IC), those wherein HET is pyridin-2-yl or pyrazin-2-yl (especially pyridin-2-yl); R² and R³ are independently hydrogen or fluoro; Rp1 and Rp2 are independently hydrogen, AR-oxymethyl or AR-thiomethyl (wherein AR is phenyl, phenyl-(1-4C)alkyl, naphthyl, furan, pyrrole, pyrazole, imidazole, triazole, pyrimidine, pyridazine, pyridine, isoxazole, oxazole, isothiazole, thiazole or thiophene), (1-4C)alkyl, carboxy, (1-4C)alkoxycarbonyl, hydroxymethyl, (1-4C)alkoxymethyl or carbamoyl and Rcp is cyano, pyrimidin-2-yl, 2-cyanoethenyl, 2-cyano-2-((1-4C)alkyl)ethenyl or Rcp is of the formula R^{13p}CO-, R^{13p}SO₂- or R^{13p}CS- (wherein R^{13p} is hydrogen, (1-5C)alkyl [optionally substituted by one or more groups each independently selected from hydroxy and amino, or optionally monosubstituted by (1-4C)alkoxy, (1-4C)alkylS(O)_{q} , (1-4C)alkylamino, (1-4C)alkanoyl, (2-6C)alkanoylamino or (1-4C)alkylS(O)ₚNH- wherein p is 1 or 2 and q is 0, 1 or 2], pyridine, or R^{13p} is of the formula R^{14p}C(O)O(1-6C)alkyl wherein R^{14p} is (1-6C)alkyl), or Rcp is of the formula RfC(=O)C(=O)-wherein Rf is (1-6C)alkoxy; or pharmaceutically-acceptable salts thereof are further preferred.

Of the above especially preferred compounds of the invention of the formula (IC), particularly preferred compounds are those wherein HET is pyridin-2-yl or pyrazin-2-yl (especially pyridin-2-yl); R² and R³ are independently hydrogen or fluoro; Rp1 and Rp2 are hydrogen, and Rcp is pyridin-2-yl (optionally substituted with cyano) or Rcp is of the formula R^{13p}CO- (wherein R^{13p} is hydrogen, 1,3-dioxolan-4-yl (optionally disubstituted with (1-4C)alkyl) or (1-5C)alkyl [optionally substituted by one or more hydroxy groups] or R^{13p} is of the formula R^{14p}C(O)O(1-6C)alkyl wherein R^{14p} is (1-6C)alkyl)); or pharmaceutically-acceptable salts thereof.

Of the above especially preferred compounds of the invention of the formula (IC), particularly preferred compounds are those wherein Rcp is of the formula R^{13p}CO- (wherein R^{13p} is hydrogen, 1,3-dioxolan-4-yl (optionally disubstituted with (1-4C)alkyl) or (1-5C)alkyl [substituted by two hydroxy groups]; or pharmaceutically-acceptable salts thereof.

In all of the above definitions the preferred compounds are as shown in formula (IA), i.e. the pharmaceutically active (5(R)) enantiomer.

Particular compounds of the present invention include the following (and the individual isomers where a mixture of isomers is possible) :-
Particular compounds of the present invention include :-
5(R)-Pyrid-2-yloxymethyl-3-(4-(4-(5-cyanopyrid-2-yl)piperazin-1-yl)-3-fluorophenyl)oxazolidin-2-one;
5(R)-(6-Chloropyridazin-3-yloxymethyl)-3-(4-(4-(5-cyanopyrid-2-yl)piperazin-1-yl)-3-fluorophenyl)oxazolidin-2-one;
5(R)-Pyrazin-2-yloxymethyl-3-(4-(4-(5-cyanopyrid-2-yl)piperazin-1-yl)-3-fluorophenyl)oxazolidin-2-one;
5(R)-Pyrimidin-4-yloxymethyl-3-(4-(4-(5-cyanopyrid-2-yl)piperazin-1-yl)-3-fluorophenyl)oxazolidin-2-one;
5(R)-Pyridazin-3-yloxymethyl-3-(4-(4-(5-cyanopyrid-2-yl)piperazin-1-yl)-3-fluorophenyl)oxazolidin-2-one;
5(R)-Pyrid-2-yloxymethyl-3-(3-fluoro-4-(3,6-dihydro-(2H)-pyran-4-yl)phenyl)oxazolidin-2-one;
5(R)-(4-Methylpyrid-2-yloxymethyl)-3-(3-fluoro-4-(3,6-dihydro-(2H)-pyran-4-yl)phenyl)oxazolidin-2-one;
5(R)-(3-Methylpyrid-2-yloxymethyl)-3-(3-fluoro-4-(3,6-dihydro-(2H)-pyran-4-yl)phenyl)oxazolidin-2-one;
5(R)-(6-Chloropyridazin-3-yloxymethyl)-3-(3-fluoro-4-(3,6-dihydro-(2H)-pyran-4-yl)phenyl)oxazolidin-2-one;
5(R)-Pyridazin-3-yloxymethyl-3-(3-fluoro-4-(3,6-dihydro-(2H)-pyran-4-yl)phenyl)oxazolidin-2-one;
5(R)-Pyrimidin-4-yloxymethyl-3-(3-fluoro-4-(3,6-dihydro-(2H)-pyran-4-yl)phenyl)oxazolidin-2-one;
5(R)-Pyrazin-2-yloxymethyl-3-(3-fluoro-4-(3,6-dihydro-(2H)-pyran-4-yl)phenyl)oxazolidin-2-one;
5(R)-(5-Chloropyridin-2-yloxymethyl)-3-(3-fluoro-4-(3,6-dihydro-(2H)-pyran-4-yl)phenyl)oxazolidin-2-one; and pharmaceutically-acceptable salts thereof.

Of the above particular compounds, especially preferred are 5(R)-Pyrid-2-yloxymethyl-3-(3-fluoro-4-(3,6-dihydro-(2H)-pyran-4-yl)phenyl)oxazolidin-2-one;
5(R)-Pyrazin-2-yloxymethyl-3-(3-fluoro-4-(3,6-dihydro-(2H)-pyran-4-yl)phenyl)oxazolidin-2-one; and pharmaceutically-acceptable salts thereof.

Other preferred compounds are those described in the Examples, and the 3,5-difluorophenyl analogues of the 3-fluorophenyl compounds described in the Examples.

### Process section :

In a further aspect the present invention provides a process for preparing a compound of formula (I) or a pharmaceutically-acceptable salt thereof. It will be appreciated that during certain of the following processes certain substituents may require protection to prevent their undesired reaction. The skilled chemist will appreciate when such protection is required, and how such protecting groups may be put in place, and later removed.

For examples of protecting groups see one of the many general texts on the subject, for example, 'Protective Groups in Organic Synthesis' by Theodora Green (publisher: John Wiley & Sons). Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Thus, if reactants include, for example, groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or *t*-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a t-butyl group which may be removed, for example, by treatment with an acid, for example an organic acid such as trifluoroacetic acid, or for example a benzyl group which may be removed, for example, by hydrogenation over a catalyst such as palladium-on-carbon.

Examples of the use of resins as a protecting group are illustrated in Examples 135 & 136 herein.

The protecting groups may be removed at any convenient stage in the synthesis using conventional techniques well known in the chemical art.

A compound of the formula (I), or a pharmaceutically-acceptable salt thereof, may be prepared by any process known to be applicable to the preparation of chemically-related compounds. Such processes, when used to prepare a compound of the formula (I), or a pharmaceutically-acceptable salt thereof, are provided as a further feature of the invention and are illustrated by the following representative examples. Necessary starting materials may be obtained by standard procedures of organic chemistry (see, for example, Advanced Organic Chemistry (Wiley-Interscience), Jerry March). The preparation of such starting materials is described within the accompanying non-limiting Examples. Alternatively, necessary starting materials are obtainable by analogous procedures to those illustrated which are within the ordinary skill of an organic chemist. Information on the preparation of necessary starting materials or related compounds (which may be adapted to form necessary starting materials) may also be found in the following Patent and Application Publications, the contents of the relevant process sections of which are hereby incorporated herein by reference : WO99/02525; WO98/54161; WO97/37980; WO97/30981 (& US5,736,545); WO97/21708 (& US5,719,154); WO97/10223; WO97/09328; WO96/35691; WO96/23788; WO96/15130; WO96/13502; WO95/25106 (& US5,668,286); WO95/14684 (& US5,652,238); WO95/07271 (& US5,688,792); WO94/13649; WO94/01110; WO93/23384 (& US5,547,950 & US 5,700,799); WO93/09103 (& US5,565,571, US5,654,428, US5,654,435, US5,756,732 & US5,801,246); US5,231,188; US5,247,090; US5,523,403; WO97/27188; WO97/30995; WO97/31917; WO98/01447; WO98/01446; WO99/10342; WO99/10343; WO99/11642; European Patent Application Nos. 0,359,418 and 0,609,905; 0,693,491 A1 (& US5,698,574); 0,694,543 A1 (& AU 24985/95); 0,694,544 A1 (& CA 2,154,024); 0,697,412 A1 (& US5,529,998); 0,738,726 A1 (& AU 50735/96); 0,785,201 A1 (& AU 10123/97); German Patent Application Nos. DE 195 14 313 A1 (& US5,529,998); DE 196 01 264 A1 (& AU 10098/97); DE 196 01 265 A1 (& AU 10097/97); DE 196 04 223 A1 (& AU 12516/97); DE 196 49 095 A1 (& AU 12517/97).

The following Patent and Application Publications may also provide useful information and the contents of the relevant process sections are hereby incorporated herein by reference :
FR 2458547; FR 2500450(& GB 2094299, GB 2141716 & US 4,476,136); DE 2923295 (& GB 2028306, GB 2054575, US4,287,351, US4,348,393, US4,413,001, US4,435,415 & US4,526,786), DE 3017499 (& GB 2053196, US4,346,102 & US4,372,967);
US4,705,799; European Patent Application Nos. 0,312,000; 0,127,902; 0,184,170; 0,352,781; 0,316,594;

The skilled organic chemist will be able to use and adapt the information contained and referenced within the above references to obtain necessary starting materials.

Thus, the present invention also provides that the compounds of the formulae **(I)** and pharmaceutically-acceptable salts thereof, can be prepared by a process **(a)** to **(g)** as follows (wherein the variables are as defined above unless otherwise stated) :
**(a)** by modifying a substituent in or introducing a substituent into another compound of formula (I);
**(b)** by reaction of a compound of formula (II) 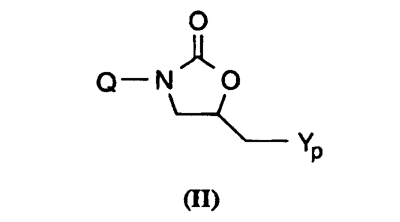 wherein Yp is hydroxy with a compound of the formula (b1) HET-OH or (b2) HET-Lg, wherein Lg is a suitable leaving group;
**(c)** by reaction of a compound of formula (II) wherein Yp is a leaving group, for example halogen, mesylate or tosylate, with a metal alkoxide compound of the formula HET-OM where M is an alkali metal, or another metal, such as silver, known to promote O-alkylation;
**(d)** by reaction of a compound of the formula Q-Zp wherein Zp is an isocyanate or amine group with an epoxide of the formula CH₂(O)CH-CH₂O-HET;
**(e)** when X is -S- by a process analogous to process (c) wherein (e1) a metal thioxide compound of the formula HET-SM where M is an alkali metal, or another metal, such as silver, known to promote S-alkylation; or (e2) alternatively by a process analogous to process (c) using HET-SH and a compound of formula (II) in which Yp is a suitable leaving group;
**(f)** when X is -SO- or -SO₂- by oxidation of a compound wherein X is -S-;
**(g)** by reaction of a urethane compound of formula (III) with a compound of formula (IV) 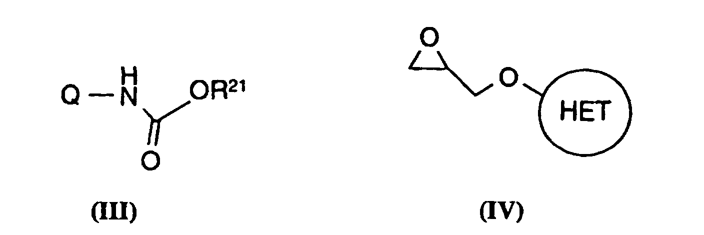
wherein R²¹ is (1-6C)alkyl or benzyl; and thereafter if necessary :
(i) removing any protecting groups; (ii) forming a pharmaceutically-acceptable salt.

General guidance on reaction conditions and reagents may be obtained in Advanced Organic Chemistry, 4^{th} Edition, Jerry March (publisher : J.Wiley & Sons), 1992. Necessary starting materials may be obtained by standard procedures of organic chemistry, such as described in this process section, in the Examples section or by analogous procedures within the ordinary skill of an organic chemist. Certain references are also provided (see above) which describe the preparation of certain suitable starting materials, for particular example see International Patent Application Publication No. WO 97/37980, the contents of which are incorporated here by reference. Processes analogous to those described in the references may also be used by the ordinary organic chemist to obtain necessary starting materials.
**(a)** Methods for converting substituents into other substituents are known in the art. For example an alkylthio group may be oxidised to an alkylsulfinyl or alkylsulfonyl group, a cyano group reduced to an amino group, a nitro group reduced to an amino group, a hydroxy group alkylated to a methoxy group, a hydroxy group thiomethylated to an arylthiomethyl or a heteroarylthiomethyl group (see, for example, Tet.Lett., 585, 1972), a carbonyl group converted to a thiocarbonyl group (eg. using Lawsson's reagent) or a bromo group converted to an alkylthio group. It is also possible to convert one Rc group into another Rc group as a final step in the preparation of a compound of the formula (I).

One compound of formula (I) may be converted into another compound of formula (I) by reacting a compound of formula (I) in which T is halo with a suitable compound to form another value of T. Thus, for example, T as halo may be displaced by suitable vinyl, aromatic, tropolone and nitrogen-linked systems as T by reaction using known Pd(0) coupling techniques.

Further examples of converting substituents into other substituents are contained in the accompanying non-limiting Examples.
**(b1)** When HET-OH is used reaction (b1) is performed under Mitsunobu conditions, for example, in the presence of tri-n-butylphosphine and diethyl azodicarboxylate (DEAD) in an organic solvent such as THF, and in the temperature range 0°C - 60°C, but preferably at ambient temperature. Details of Mitsunobu reactions are contained in Tet. Letts., 31, 699, (1990); The Mitsunobu Reaction, D.L.Hughes, Organic Reactions, 1992, Vol.42, 335-656 and Progress in the Mitsunobu Reaction, D.L.Hughes, Organic Preparations and Procedures International, 1996, Vol.28, 127-164.
**(b2)** When HET-Lg is used reaction (b2) is performed using a suitably reactive HET and under basic conditions (using a base such as 1,8-diazabicyclo[5,4,0]undec-7-ene) which are sufficiently mild not to destroy the oxazolidinone ring structure. The skilled organic chemist will appreciate which suitable leaving group Lg (such as chloro or bromo) and reaction conditions to use.

Compounds of the formula (II) wherein Yp is hydroxy may be obtained by reacting a compound of the formula (III) with a compound of formula (V): 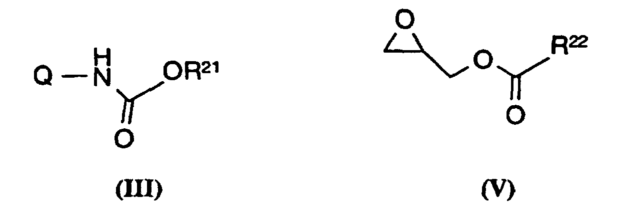 wherein R²¹ is (1-6C)alkyl or benzyl and R²² is (1-4C)alkyl or -S(O)_{q}(1-4C)alkyl where q is 0, 1 or 2. Preferably R²² is (1-4C)alkyl.

Compounds of the formula (II), (III) and (V) may be prepared by the skilled chemist, for example as described in International Patent Application Publication Nos. WO95/07271, WO97/27188, WO 97/30995, WO 98/01446 and WO 98/01446, the contents of which are hereby incorporated by reference, and by analogous processes.

If not commercially available, compounds of the formula HET-OH and HET-Lg may be prepared by procedures which are selected from standard chemical techniques, techniques which are analogous to the synthesis of known, structurally similar compounds, or techniques which are analogous to the procedures described in the Examples. For example, standard chemical techniques are as described in Houben Weyl, Methoden der Organische Chemie. **(c)** & **(e)** Reactions (c) and (e) are performed conveniently at a temperature in the range 25-60°C in a solvent such as NMP or DMF.

A compound of the formula (II) wherein Yp is fluoro may be prepared by reacting a compound of the formula (II) wherein Yp is hydroxy (hydroxy compound) with a fluorinating agent such as diethylaminosulfur trifluoride in an organic solvent such as dichloromethane in the temperature range of 0°C to ambient temperature.

When Yp is chloro, the compound of the formula (II) may be formed by reacting the hydroxy compound with a chlorinating agent. For example, by reacting the hydroxy compound with thionyl chloride, in a temperature range of ambient temperature to reflux, optionally in a chlorinated solvent such as dichloromethane or by reacting the hydroxy compound with carbon tetrachloride/triphenyl phosphine in dichloromethane, in a temperature range of 0°C to ambient temperature. A compound of the formula (II) wherein Yp is chloro or iodo may also be prepared from a compound of the formula (II) wherein Yp is mesylate or tosylate, by reacting the latter compound with lithium chloride or lithium iodide and crown ether, in a suitable organic solvent such as THF, in a temperature range of ambient temperature to reflux

When Yp is (1-4C)alkanesulfonyloxy or tosylate the compound (II) may be prepared by reacting the hydroxy compound with (1-4C)alkanesulfonyl chloride or tosyl chloride in the presence of a mild base such as triethylamine or pyridine.

Compounds of the formula HET-OM and HET-SM may be prepared by the skilled chemist from the corresponding HET-OH or HET-SH compound, using a suitable base, such as sodium hydride, silver carbonate, sodium carbonate or an alkoxide.

When X is -S- and a process is used that is analogous to process (c) but using HET-SH and a compound of formula (II) in which Yp is a suitable leaving group, a suitable leaving group is, for example, mesylate and a suitable base for the reaction is a base such as 1,8-diazabicyclo[5,4,0]undec-7-ene (see for example, Example 153).
**(d)** Reaction (d) is performed under conditions analogous to those described in the following references which disclose how suitable and analogous starting materials may be obtained.

Compounds of the formula Q-Zp wherein Zp is an isocyanate may be prepared by the skilled chemist, for example by analogous processes to those described in Walter A. Gregory et al in J.Med.Chem. 1990, 33, 2569-2578 and Chung-Ho Park et al in J.Med.Chem. 1992, 35, 1156-1165. Compounds of the formula Q-Zp wherein Zp is a urethane (see process (i)) may be prepared by the skilled chemist, for example by analogous processes to those described in International Patent Application Publication Nos. WO 97/30995 and WO 97/37980.

A similar reaction to reaction (d) may be performed in which Q-Zp wherein Zp is a amine group is reacted with the epoxide (optionally in the presence of an organic base), and the product is reacted with, for example, phosgene to form the oxazolidinone ring. Such reactions and the preparation of starting materials in within the skill of the ordinary chemist with reference to the above-cited documents disclosing analogous reactions and preparations.

Epoxides of the formula CH₂(O)CH-CH₂O-HET may be prepared from the corresponding CH₂=CH-CH₂-O-HET compound. Certain such epoxide and alkene intermediates are novel and are provided as a further feature of the invention. Asymmetric epoxidation may be used to give the desired optical isomer.
**(f)** When X is -SO- or -SO₂- the oxidation of a compound wherein X is -S- may be achieved by oxidising with standard reagents known in the art for the oxidation of a thio group to a sulfinyl or sulfonyl group. For example, a thio group may be oxidised to a sulfinyl group with a peracid such as m-chloroperoxybenzoic acid and oxidising agents such as potassium permanganate can be used to convert a thio group to a sulfonyl group.
**(g)** A compound of formula (III) is reacted with a compound of formula (IV) using similar conditions to those for reaction of a compound of the formula (III) with a compound of formula (V) described above. If not commercially available, the preparation of suitable starting materials of formulae (III) and (IV) is as described above, or by using analogous processes.

The removal of any protecting groups, the formation of a pharmaceutically-acceptable salt are within the skill of an ordinary organic chemist using standard techniques.

When an optically active form of a compound of the formula (I) is required, it may be obtained by carrying out one of the above procedures using an optically active starting material (formed, for example, by asymmetric induction of a suitable reaction step), or by resolution of a racemic form of the compound or intermediate using a standard procedure, or by chromatographic separation of diastereoisomers (when produced). Enzymatic techniques may also be useful for the preparation of optically active compounds and/or intermediates.

Similarly, when a pure regioisomer of a compound of the formula (I) is required, it may be obtained by carrying out one of the above procedures using a pure regioisomer as a starting material, or by resolution of a mixture of the regioisomers or intermediates using a standard procedure.

According to a further feature of the invention there is provided a compound of the formula (I), or a pharmaceutically-acceptable salt thereof for use in a method of treatment of the human or animal body by therapy.

According to a further feature of the present invention there is provided a method for producing an antibacterial effect in a warm blooded animal, such as man, in need of such treatment, which comprises administering to said animal an effective amount of a compound of the present invention, or a pharmaceutically-acceptable salt thereof.

The invention also provides a compound of the formula (I), or a pharmaceutically-acceptable salt, or in-vivo hydrolysable ester thereof, for use as a medicament; and the use of a compound of the formula (I) of the present invention, or a pharmaceutically-acceptable salt thereof, in the manufacture of a medicament for use in the production of an antibacterial effect in a warm blooded animal, such as man.

In order to use a compound of the formula (I) or a pharmaceutically-acceptable salt thereof (hereinafter in this section relating to pharmaceutical composition "a compound of this invention") for the therapeutic (including prophylactic) treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula (I) or a pharmaceutically-acceptable salt thereof, and a pharmaceutically-acceptable diluent or carrier.

The pharmaceutical compositions of this invention may be administered in standard manner for the disease condition that it is desired to treat, for example by oral, rectal or parenteral administration. For these purposes the compounds of this invention may be formulated by means known in the art into the form of, for example, tablets, capsules, aqueous or oily solutions or suspensions, (lipid) emulsions, dispersible powders, suppositories, ointments, creams, aerosols (or sprays), drops and sterile injectable aqueous or oily solutions or suspensions.

In addition to the compounds of the present invention the pharmaceutical composition of this invention may also contain or be co-administered (simultaneously, sequentially or separately) with one or more known drugs selected from other clinically useful antibacterial agents (for example, β-lactams or aminoglycosides) and/or other anti-infective agents (for example, an antifungal triazole or amphotericin). These may include carbapenems, for example meropenem or imipenem, to broaden the therapeutic effectiveness. Compounds of this invention may also contain or be co-administered with bactericidal/permeability-increasing protein (BPI) products or efflux pump inhibitors to improve activity against gram negative bacteria and bacteria resistant to antimicrobial agents.

A suitable pharmaceutical composition of this invention is one suitable for oral administration in unit dosage form, for example a tablet or capsule which contains between 1mg and 1g of a compound of this invention, preferably between 100mg and 1g of a compound. Especially preferred is a tablet or capsule which contains between 50mg and 800mg of a compound of this invention, particularly in the range 100mg to 500mg.

In another aspect a pharmaceutical composition of the invention is one suitable for intravenous, subcutaneous or intramuscular injection, for example an injection which contains between 0.1% w/v and 50% w/v (between 1mg/ml and 500mg/ml) of a compound of this invention.

Each patient may receive, for example, a daily intravenous, subcutaneous or intramuscular dose of 0.5 mgkg-¹ to 20 mgkg-¹ of a compound of this invention, the composition being administered 1 to 4 times per day. In another embodiment a daily dose of 5 mgkg-¹ to 20 mgkg-¹ of a compound of this invention is administered. The intravenous, subcutaneous and intramuscular dose may be given by means of a bolus injection. Alternatively the intravenous dose may be given by continuous infusion over a period of time. Alternatively each patient may receive a daily oral dose which may be approximately equivalent to the daily parenteral dose, the composition being administered 1 to 4 times per day.

A pharmaceutical composition to be dosed intravenously may contain advantageously (for example to enhance stability) a suitable bactericide, antioxidant or reducing agent, or a suitable sequestering agent.

In the above other, pharmaceutical composition, process, method, use and medicament manufacture features, the alternative and preferred embodiments of the compounds of the invention described herein also apply.

### Antibacterial Activity:

The pharmaceutically-acceptable compounds of the present invention are useful antibacterial agents having a good spectrum of activity in vitro against standard Gram-positive organisms, which are used to screen for activity against pathogenic bacteria. Notably, the pharmaceutically-acceptable compounds of the present invention show activity against enterococci, pneumococci and methicillin resistant strains of S.aureus and coagulase negative staphylococci. The antibacterial spectrum and potency of a particular compound may be determined in a standard test system.

The (antibacterial) properties of the compounds of the invention may also be demonstrated and assessed in-vivo in conventional tests, for example by oral and/or intravenous dosing of a compound to a warm-blooded mammal using standard techniques.

The following results were obtained on a standard in-vitro test system. The activity is described in terms of the minimum inhibitory concentration (MIC) determined by the agar-dilution technique with an inoculum size of 10⁴ CFU/spot. Typically, compounds are active in the range 0.01 to 256 µg/ml.

Staphylococci were tested on agar, using an inoculum of 10⁴ CFU/spot and an incubation temperature of 37°C for 24 hours - standard test conditions for the expression of methicillin resistance.

Streptococci and enterococci were tested on agar supplemented with 5% defibrinated horse blood, an inoculum of 10⁴ CFU/spot and an incubation temperature of 37°C in an atmosphere of 5% carbon dioxide for 48 hours - blood is required for the growth of some of the test organisms.

| Organism | | MIC (µg/ml) |
|---|---|---|
| | | Example 6 |
| Staphylococcus aureus: | | |
| | Oxford | 0.5 |
| | Novb. Res | 2.0 |
| | MRQR | 1.0 |

| Coagulase Negative Staphylococci | | |
|---|---|---|
| | MS | 0.5 |
| | MR | 1.0 |

| Streptococcus pyogenes | | |
|---|---|---|
| | C203 | 2.0 |
| Enterococcus faecalis | | 2.0 |
| Bacillus subtilis | | 0.5 |
| Novb. Res = Novobiocin resistant | | |
| MRQR = methicillin resistant quinolone resistant | | |
| MR = methicillin resistant | | |
| MS = methicillin sensitive | | |

The invention is now illustrated but not limited by the following Examples in which unless otherwise stated :-
i) evaporations were carried out by rotary evaporation in vacuo and work-up procedures were carried out after removal of residual solids by filtration;
(ii) operations were carried out at ambient temperature, that is typically in the range 18-26°C and in air unless otherwise stated, or unless the skilled person would otherwise work under an inert atmosphere;
(iii) column chromatography (by the flash procedure) was used to purify compounds and was performed on Merck Kieselgel silica (Art. 9385) unless otherwise stated;
(iv) yields are given for illustration only and are not necessarily the maximum attainable;
(v) the structure of the end-products of the formula (I) were generally confirmed by NMR and mass spectral techniques [proton magnetic resonance spectra were generally determined in DMSO-D6 unless otherwise stated using a Varian Gemini 2000 spectrometer operating at a field strength of 300 MHz, or a Bruker AM250 spectrometer operating at a field strength of 250 MHz; chemical shifts are reported in parts per million downfield from tetramethysilane as an internal standard (δ scale) and peak multiplicities are shown thus: s, singlet; d, doublet; AB or dd, doublet of doublets; t, triplet, m, multiplet; fast-atom bombardment (FAB) mass spectral data were generally obtained using a Platform spectrometer (supplied by Micromass) run in electrospray and, where appropriate, either positive ion data or negative ion data were collected];
(vi) intermediates were not generally fully characterised and purity was in general assessed by thin layer chromatographic, infra-red (IR), mass spectral (MS) or NMR analysis; and
(vii) in which the following abbreviations may be used :-
   ® is a Trademark; DMF is N,N-dimethylformamide; DMA is N,N-dimethylacetamide; TLC is thin layer chromatography; HPLC is high pressure liquid chromatography; MPLC is medium pressure liquid chromatography; DMSO is dimethylsulfoxide; CDCl₃ is deuterated chloroform; MS is mass spectroscopy; ESP is electrospray; THF is tetrahydrofuran; TFA is trifluoroacetic acid; NMP is N-methylpyrrolidone; HOBT is 1-hydroxy-benzotriazole; EtOAc is ethyl acetate; MeOH is methanol; phosphoryl is (HO)₂-P(O)-O-; phosphiryl is (HO)₂-P-O-; EDC is 1-(3-dimeihylaminopropyl)-3-ethylcarbodiimide (hydrochloride); PTSA is paratoluenesulfonic acid.

### Reference Example 1: 5(R)-Hydroxymethyl-3-(4-(4-(5-cyanopyrid-2-yl)piperazin-1-yl)-3-fluorophenyl)oxazolidin-2-one

5(R)-Hydroxymethyl-3-(3-fluoro-4-(4-*t*-butoxycarbonylpiperazin-1-yl)phenyl)-oxazolidin-2-one (International Patent A pplication Publication WO 93/23384, 43.1 g, 0.11 M) was suspended by stirring in ethanol (1000 ml) under nitrogen. An ethanol solution of hydrogen chloride (3.8 M, 400 ml) was added slowly, and the mixture was stirred at ambient temperature for 18 hours. The resulting precipitate was filtered, washed with diethyl ether (3 x 250 ml), and dried, to give 5(R)-hydroxymethyl-3-(3-fluoro-4-(piperazin-1-yl)phenyl)oxazolidin-2-one hydrochloride. A further crop was obtained by evaporation of the mother liquors to give a total yield of 38.7 g.
¹H-NMR (300MHz, DMSO-D6) δ: 3.17 (m, 8H); 3.53 (dd, 1H); 3.64 (dd, 1H); 3.79 (dd, 1H); 4.03 (t, 1H); 4.66 (m, 1H); 7.10 (t, 1H); 7.21 (dd, 1H); 7.52 (dd, 1H); 9.39 (br s, 2H). MS (ESP): 296 (MH⁺) for C₁₄H₁₈FN₃O₃
5(R)-Hydroxymethyl-3-(3-fluoro-4-(piperazin-1-yl)phenyl)oxazolidin-2-one hydrochloride (25 g, 75.4 mM) was suspended by stirring in acetonitrile (700 ml) under nitrogen, and triethylamine (16.8 g, 166 mM) added. The mixture was stirred for 10 minutes and then 2-chloro-5-cyanopyridine (10.3 g, 75.4 mmol) added, and the mixture heated under reflux for 18 hours. After cooling, the resultant solid was filtered, washed with water (3 x 500 ml) and diethyl ether (2 x 500 ml) to give 5(R)-hydroxymethyl-3-(4-(4-(5-cyanopyrid-2-yl)piperazin-1-yl)-3-fluorophenyl)-oxazolidin-2-one. A further crop was obtained by evaporation of the mother liquors to give a total yield of 23.2 g. MS (ESP): 398 (MH⁺) for C₂₀H₂₀FN₅O₃
¹H-NMR (300MHz, DMSO-D6) δ: 3.03 (t, 4H); 3.54 (m, 1H); 3.63(m, 1H); 3.78 (t overlapping m, 5H); 4.03 (t, 1H); 4.66 (m, 1H); 5.18 (t, 1H); 6.97 (d, 1H); 7.07 (t, 1H); 7.20 (dd, 1H); 7.53 (dd, 1H); 7.85 (dd, 1H); 8.49 (d, 1H).

### Example 1: 5(R)-Pyrid-2-yloxymethyl-3-(4-(4-(5-cyanopyrid-2-yl)piperazin-1-yl)-3-fluorophenyl)oxazolidin-2-one

5(R)-Hydroxymethyl-3-(4-(4-(5-cyanopyrid-2-yl)piperazin-1-yl)-3-fluorophenyl)-oxazolidin-2-one (397 mg, 1 mM), 2-hydroxypyridine (104 mg, 1.1 mM) and polymer bound triphenylphosphine (3 mM/g, 416 mg, 1.25 mM), were suspended with stirring in dry tetrahydrofuran (10 ml). Diisopropylazodicarboxylate (242 mg, 1.2 mM) was added dropwise by syringe, and the mixture stirred at ambient temperature for 1 hour. The reaction mixture was filtered, evaporated to dryness, dissolved in ethyl acetate, and applied to a 10 g silica Mega Bond Elut® column, eluting with a mixture of ethyl acetate and isohexane (1:1). Relevant fractions were combined and evaporated to give the title compound (45 mg). MS (ESP): 475 (MH⁺) for C₂₅H₂₃FN₆O₃
¹H-NMR (300MHz,DMSO-D6) δ: 3.03 (t, 4H); 3.81 (t, 4H); 3.89 (dd, 1H); 4.17 (t, 1H); 4.47 (dd, 1H); 4.55 (dd, 1H); 5.03 (m, 1H); 6.82 (d, 1H); 6.99 (t overlapping dd, 2H); 7.08 (t, 1H); 7.21 (dd, 1H); 7.52 (dd, 1H); 7.70 (td, 1H); 7.85 (dd, 1H); 8.15 (dd, 1H); 8.48 (d, 1H).

### Example 2 : 5(R)-(6-Chloropyridazin-3-yloxymethyl)-3-(4-(4-(5-cyanopyrid-2-yl)piperazin-1-yl)-3-fluorophenyl)oxazolidin-2-one:

### Example 3 : 5(R)-Pyrazin-2-yloxymethyl-3-(4-(4-(5-cyanopyrid-2-yl)piperazin-1-yl)-3-fluorophenyl)oxazolidin-2-one

5(R)-Hydroxymethyl-3-(4-(4-(5-cyanopyrid-2-yl)piperazin-1-yl)-3-fluorophenyl)-oxazolidin-2-one (240 mg, 0.6 mM), was added in portions to a stirred suspension of sodium hydride (60% in oil, 26 mg, 0.65 mM) in dry *N,N*-dimethylformamide under nitrogen. The mixture was stirred at ambient temperature for 25 minutes, the appropriate chloroheterocycle (0.6 mM) added, and stirring continued for 18 hours. The mixture was diluted with water (20 ml), and the resulting precipitate filtered, washed with diethyl ether, and dried to give the title compounds.

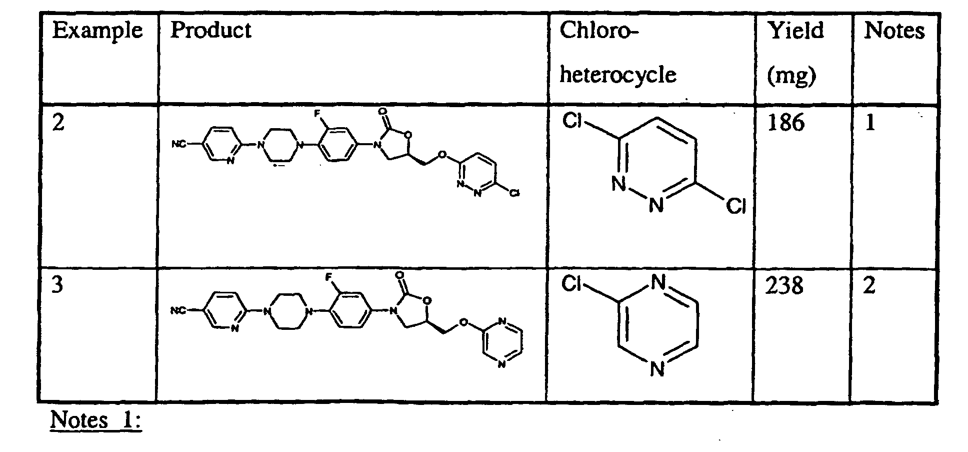

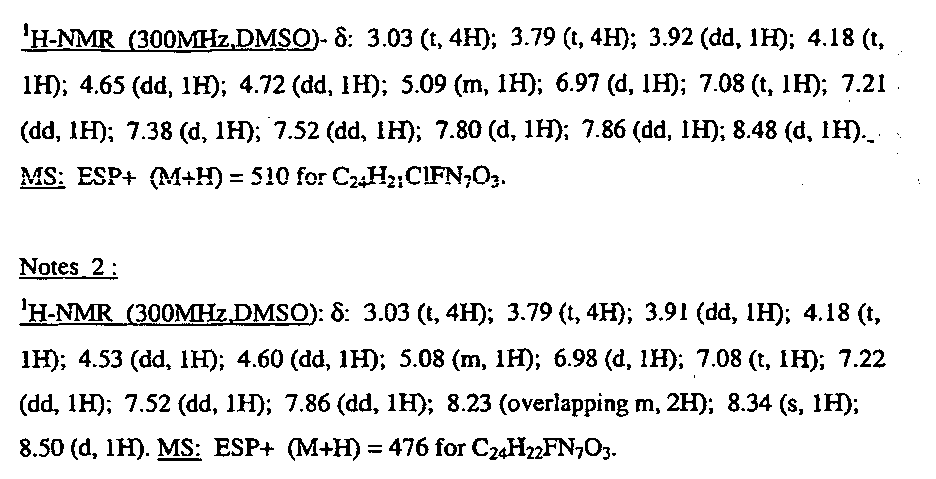

### Example 4 : 5(R)-Pyrimidin-4-yloxymethyl-3-(4-(4-(5-cyanopyrid-2-yl)piperazin-1-yl)-3-fluorophenyl)oxazolidin-2-one

5(R)-Hydroxymethyl-3-(4-(4-(5-cyanopyrid-2-yl)piperazin-1-yl)-3-fluorophenyl)-oxazolidin-2-one (397 mg, I mM), 4-hydroxypyrimidine (106 mg, 1.1 mM) and triphenylphosphine (327 mg, 1.25 mM), were suspended with stirring in dry tetrahydrofuran (10 ml). Di*iso*propylazodicarboxylate (242 mg, 1.2 mM) was added dropwise by syringe, and the mixture stirred at ambient temperature for 30 minutes. The reaction mixture was evaporated to dryness, dissolved in ethyl acetate / *iso*hexane (3:1), and applied to a 20 g silica Mega Bond Elut® column, eluting with a gradient increasing in polarity from 3:1 ethyl acetate in isohexane to pure ethyl acetate. Relevant fractions were combined and evaporated to give the title compound (240 mg). MS (ESP): 476 (MH⁺) for C₂₄H₂₂FN₇O_{3.}
¹H-NMR (300MHz,DMSO): δ: 3.03 (t, 4H); 3.79 (t, 4H); 3.89 (dd, 1H); 4.17 (t, 1H); 4.56 (dd, 1H); 4.61 (dd, 1H); 5.06 (m, 1H); 6.96 (overlapping m, 2H); 7.08 (t, 1H); 7.20 (dd, 1H); 7.51 (dd, 1H); 7.86 (dd, 1H); 8.50 (d, 1H); 8.50 (d, 1H); 8.79 (s, 1H).

### Example 5 : 5(R)-Pyridazin-3-yloxymethyl-3-(4-(4-(5-cyanopyrid-2-yl)piperazin-1-yl)-3-fluorophenyl)oxazolidin-2-one

5(R)-(6-Chloropyridazin-3-yloxymethyl)-3-(4-(4-(5-cyanopyrid-2-yl)piperazin-1-yl)-3-fluorophenyl)oxazolidin-2-one (176 mg, 0.35 mM) was dissolved in *N,N*-dimethylformamide (20 ml) and triethylamine (71 mg, 0.7 mM) and palladium on charcoal (5%, 30 mg) added with stirring. The atmosphere in the vessel was replaced with hydrogen under a balloon, and the mixture stirred 18 hours at ambient temperature. Catalyst was filtered off through celite, solvent evaporated, and the residue dissolved in dichloromethane, and applied to a 20 g silica Mega Bond Elut column, eluting with a gradient increasing in polarity from pure dichloromethane to 19:1 dichloromethane in methanol. Relevant fractions were combined and evaporated to give the title compound (22 mg). MS (ES): 476 (MH⁺) for C₂₄H₂₂FN₇O₃
NMR (DMSO-D6) δ: 3.04 (t, 4H); 3.81 (t, 4H); 3.94 (dd, 1H); 4.19 (t, 1H); 4.70 (overlapping m, 2H); 5.11 (m, 1H); 6.99 (d, 1H); 7.09 (t, 1H); 7.23 (overlapping m, 2H); 7.53 (dd, 1H); 7.61 (m, 1H); 7.86 (dd, 1H); 8.50 (d, 1H); 8.91 (dd, 1H).

### Example 6: 5(R)-Pyrid-2-yloxymethyl-3-(3-fluoro-4-(3,6-dihydro-(2H)-pyran-4-yl)phenyl)oxazolidin-2-one

2-Hydroxypyridine (108mg, 1.14mmol) was added portionwise, at ambient temperature, to a stirred suspension of sodium hydride (48mg, 1.2mmol of a 60% dispersion in oil) in DMF (5ml) under an atmosphere of nitrogen. The mixture was stirred for 30 minutes then 5(R)-methylsulphonyloxymethyl-3-(3-fluoro-4-(3,6-dihydro-(2H)-pyran-4-yl)phenyl)oxazolidin-2-one (International Patent Application Publication WO97/09328, 400mg, 1.08mmol) was added in one go. The reaction was stirred and heated at 60°C for 5 hours, then quenched in water and extracted with ethyl acetate. The extract was washed twice with water and once with saturated brine, dried over magnesium sulphate and evaporated to give an oil. The oil was purified by flash chromatography (Merck 9385 silica, 2.5% methanol / dichloromethane eluant) to give the title product (60mg, 15%) as a crystalline solid. MS: ESP⁺ (M+H)⁺= 371.
¹H-NMR (300MHz, CDCl₃): d = 2.50 (m, 2H), 3.94 (t, 2H), 3.98 (dd, 1H), 4.15 (t, 1H), 4.34 (m, 2H), 4.60 (d, 2H), 5.05 (m, 1H), 6.04 (m, 1H), 6.77 (d, 1H), 6.92 (dd, 1H), 7.26 (m, 2H), 7.42 (dd, 1H), 7.60 (m, 1H), 8.14 (dd, 1H).

### Example 7: 5(R)-(4-Methylpyrid-2-yloxymethyl)-3-(3-fluoro-4-(3,6-dihydro-(2H)-pyran-4-yl)phenyl)oxazolidin-2-one

Diisopropylazodicarboxytate (290mg, 1.43mmol) was added dropwise, at ambient temperature, to a stirred solution of 5(R)-hydroxymethyl-3-(3-fluoro-4-(3,6-dihydro(2H)-pyran-4-yl)phenyl)oxazolidin-2-one (International Patent Application Publication WO 97/09328, 300mg, 1.02mmol), 2-hydroxy-4-methylpyridine (167mg, 1.53mmol) and triphenylphosphine (402mg, 1.53mmol) in THF (8ml). The resulting solution was stirred at ambient temperature for 30 minutes before evaporating the solvent to give an oil. The oil was purified by flash chromatography (Merck 9385 silica, ethyl acetate / isohexane(3:2) eluant) to give the title product (181mg, 46%) as a crystalline solid.
¹H-NMR (300MHz, CDCl₃): d = 2.52 (m, 2H), 3.92 (t, 2H), 3.97 (dd, 1H), 4.12 (t, 1H), 4.32 (m, 2H), 4.59 (d, 2H), 5.02 (m, 1H), 6.06 (m, 1H), 6.59 (s, 1H), 6.75 (d, 1H), 7.26 (m, 2H), 7.41 (dd, 1H), 7.99 (d, 1H). MS: ESP⁺ (M+H)⁺= 385.

### Example 8: 5(R)-(3-Methylpyrid-2-yloxymethyl)-3-(3-fluoro-4-(3,6-dihydro-(2H)-pyran-4-yl)phenyl)oxazolidin-2-one

The title product was prepared by the general method of Example 2, using the same oxazolidinone starting material as Example 7 (300mg, 1.02mmol), sodium hydride (95mg, 2.37mmol of a 60% dispersion in oil) and 2-fluoro-3-methylpyridine (130mg, 1.17mmol) in DMF (3ml). The resultant reaction product was purified by flash chromatography (Merck 9385 silica, ethyl acetate / isohexane (7:3) eluant) to give the title product (50mg, 13%) as a crystalline solid. MS: ESP⁺ (M+H)⁺= 385.
¹H-NMR 9300MHz, CDCl₃): d = 2.12 (s, 3H), 2.51 (m, 2H), 3.92 (t, 2H), 3.99 (dd, 1H), 4.17 (t, 1H), 4.32 (m, 2H), 4.61 (m, 2H), 5.05 (m, 1H), 6.07 (m, 1H), 6.84 (dd, 1H), 7.27 (m, 2H), 7.35-7.44 (m, 2H), 7.06 (d, 1H).

### Example 9: 5(R)-(6-Chloropyridazin-3-yloxymethyl)-3-(3-fluoro-4-(3,6-dihydro(2H)-pyran-4-yl)phenyl)oxazolidin-2-one

The title product was prepared by the general method of Example 8 using the same oxazolidinone starting material (600mg, 2.05mmol), sodium hydride (90mg, 2.25mmol of a 60% dispersion in oil) and 3,6-dichloropyridazine (350mg, 2.35mmol) in DMF (6ml). The mixture was stirred at ambient temperature for 18 hours then at 60°C for 6 hours, and the resultant product purified by flash chromatography (Merck 9385 silica, ethyl acetate / isohexane (7:3) eluant) to give the title product (178mg, 21 %) as a crystalline solid. MS: ESP⁺ (M+H)⁺= 406/408.
¹H-NMR (300MHz, CDCl₃): d = 2.52 (m, 2H), 3.86-4.00 (m, 3H), 4.22 (m, 1H), 4.32 (m, 2H), 4.70-4.90 (m, 2H), 5.10 (m, 1H), 6.06 (m, 1H), 7.06 (dd, 2H), 7.18-7.33 (m, 2H), 7.44 (m, 2H).

### Example 10: 5(R)-Pyridazin-3-yloxymethyl-3-(3-fluoro-4-(3,6-dihydro-(2H)-pyran-4-yl)phenyl)oxazolidin-2-one

30% Palladium on carbon (20mg) was added to a stirred suspension of Example 9 (240mg, 0.59mmol) and ammonium formate (112mg, 1.78mmol) in ethanol (10ml) under an atmosphere of nitrogen. The reaction was stirred at ambient temperature for 4 hours then filtered through celite. The filtrate was evaporated under reduced pressure to give a gum which was taken up in methanol (20ml) and stirred with sodium carbonate (500mg) for 30 minutes. The mixture was then filtered and the filtrate evaporated to give a gum, which was purified by flash chromatography (Merck 9385 silica, 3% methanol / dichloromethane eluant) to give the title product (91mg, 41 %) as a crystalline solid.
¹H-NMR (300MHz, CDCl₃): d = 2.52 (m, 2H), 3.92 (t, 2H), 3.97 (dd, 1H), 4.20 (t, 1H), 4.31 (m, 2H), 4.79 (dd, 1H), 4.88 (dd, 1H), 5.10 (m, 1H), 6.06 (m, 1H), 7.05 (d, 1H), 7.28 (m, 2H), 7.42 (m, 2H), 8.90 (d, 1H). MS: ESP⁺ (M+H)⁺= 372.

### Example 11: 5(R)-Pyrimidin-4-yloxymethyl-3-(3-fluoro-4-(3,6-dihydro-(2H)-pyran-4-yl)phenyl)oxazolidin-2-one

The title product was prepared by the general method of Example 7, using the same oxazolidinone starting material (300mg, 1.02mmol), 4-hydroxypyrimidine (147mg, 1.53mmol), diisopropylazodicarboxylate (289mg, 1.43mmol) and triphenylphosphine (402mg, 1.53mmol) in THF (8ml). The resultant reaction product was purified by flash chromatography (Merck 9385 silica, 2.5% methanol / dichloromethane eluant) to give the title product (186mg, 49%) as a crystalline solid.
¹H-NMR (300MHz, CDCl₃): d = 2.50 (m, 2H), 3.91-3.98 (m, 3H), 4.17 (t, 1H), 4.32 (m, 2H), 4.64 (dd, 1H), 4.72 (dd, 1H), 5.04 (m, 1H), 6.06 (m, 1H), 6.79 (d, 1H), 7.28 (m, 2H), 7.42 (dd, 1H), 8.49 (d, 1H), 8.78 (s, 1H). MS: ESP⁺ (M+H)⁺= 372.

### Example 12: 5(R)-Pyrazin-2-yloxymethyl-3-(3-fluoro-4-(3,6-dihydro-(2H)-pyran-4-yl)phenyl)oxazolidin-2-one

The title product was prepared by the general method of Example 8 using the same oxazolidinone starting material (300mg, 1.02mmol), sodium hydride (45mg, 1.12mmol of a 60% dispersion in oil) and 2-chloropyrazine (130mg, 1.13mmol) in DMF (3ml). The resultant reaction product was purified by flash chromatography (Merck 9385 silica, 2.5% methanol / dichloromethane eluant) to give the title product (171mg, 45%) as a crystalline solid.
¹H-NMR (300MHz, CDCl₃): d = 2.51 (m, 2H), 3.92 (t, 2H), 3.97 (dd, 1H), 4.08 (t, 1H), 4.32 (m, 2H), 4.62 (m, 2H), 5.08 (m, 1H), 6.06 (m, 1H), 7.28 (m, 2H), 7.43 (dd, 1H), 8.10 (t, 1H), 8.21 (d, 1H), 8.29 (d, 1H). MS: ESP⁺ (M+H)⁺= 372.

### Example 13: 5(R)-(5-Chloropyridin-2-yloxymethyl)-3-(3-fluoro-4-(3,6-dihydro(2H)-pyran-4-yl)phenyl)oxazolidin-2-one

The title product was prepared by the general method of Example 7 using the same oxazolidinone starting material (300mg, 1.02mmol), 3-chloro-6-hydroxypyridine (146mg, 1.13mmol), diisopropylazodicarboxylate (227mg, 1.12mmol) and triphenylphosphine (305mg, 1.16mmol) in THF (5ml). The resultant reaction product was purified by flash chromatography (Merck 9385 silica, ethyl acetate / isohexane (3:2) eluant) to give the title product (197mg, 48%) as a crystalline solid.
¹H-NMR (300MHz, CDCl₃): d = 2.51 (m, 2H), 3.90-4.00 (m, 3H), 4.14 (t, 1H), 4.30 (m, 2H), 4.56 (m, 2H), 5.02 (m, 1H), 6.05 (m, 1H), 6.74 (d, 1H), 7.20-7.30 (m, 2H), 7.40 (d, 1H), 7.55 (dd, 1H), 8.10 (d, 1H). MS: ESP⁺ (M+H)⁺= 405/407.

### Example 14: 5(R)-Pyrid-2-yloxymethyl-3-(4-acetylphenyl)-oxazolidin-2-one

5(R)-Hydroxymethyl-3-(4-acetylphenyl)-oxazolidin-2-one (C.L.J.Wang et al, Tetrahedron, Vol.45, 1323, (1989); 300mg, 1.28mmol) was added portionwise, at room temperature, to a stirred suspension of sodium hydride (56mg, 1.4mmol of a 60% dispersion in oil) in DMF (3ml) under an atmosphere of nitrogen. The mixture was stirred for an additional 15 min. then 2-fluoropyridine (148mg, 1.53mmol) added. The reaction was stirred for 18 hr. before quenching in water and extracting with ethyl acetate. The extract was washed with water (3x) and saturated brine (1x) then evaporated to give an orange gum. Purified by flash chromatography (Merck 9385 silica, ethyl acetate / isohexane(7:3)) to give the title compound (27mg, 7%) as a colourless crystalline solid. MS: ESP⁺ (M+H)⁺= 313.
¹H-NMR (300MHz, CDCl₃): δ = 2.60 (s, 3H), 4.07 (dd, 1H), 4.22 (t, 1H), 4.62 (d, 2H), 5.08 (m, 1H), 6.77 (d, 1H), 6.93 (dd, 1H), 7.59 (m, 1H), 7.68 (d, 2H), 7.99 (d, 2H), 8.14 (d, 1H).

### Example 15: 5(R)-Pyrid-2-yloxymethyl-3-(4-(4-bromoimidazol-1-yl)-3-fluorophenyl)-oxazolidin-2-one

Prepared by the general method of Example 7 using 5(R)-hydroxymethyl-3-(4-(4-bromoimidazol-1-yl)-3-fluoro)phenyl)-oxazolidin-2-one (WO97/31917; 300mg, 0.84mmol), 2-hydroxypyridine (96mg, 1.01 mmol), diisopropylazodicarboxylate (204mg, 1.012 mmol) and triphenylphosphine (270mg, 1.03mmol) in THF (5ml). Purified by flash chromatography (Merck 9385 silica, 2.5% methanol / dichloromethane) to give the title compound (156mg, 43%) as a colourless crystalline solid.
¹H-NMR (300MHz, CDCl₃): δ = 4.06 (dd, 1H), 4.18 (t, 1H), 4.62 (d, 2H), 5.10 (m, 1H), 6.78 (d, 1H), 6.94 ( m, 1H), 7.20 (d, 1H), 7.37 (m, 2H), 7.55-7.67 (m, 2H), 7.72 (d, 1H), 8.14 (d, 1H). MS: ESP⁺ (M+H)⁺= 433/435.

### Example 16: 5(R)-Pyrid-2-yloxymethyl-3-(4-methylthiophenyl)-oxazolidin-2-one

Prepared by the general method of Example 7 using 5(R)-hydroxymethyl-3-(4-methylthiophenyl)-oxazolidin-2-one (prepared from the reaction of 4-methylthioaniline and (R)-glycidyl butyrate), 200mg, 0.84mmol), 2-hydroxypyridine (90mg, 0.95mmol), diisopropylazodicarboxylate (190mg, 0.94mmol) and triphenylphosphine (252mg, 0.96mmol) in THF (5ml). Purified by flash chromatography (Merck 9385 silica, ethyl acetate / isohexane (3:2)) to give the title compound (123mg, 46%) as a colourless crystalline solid.
¹H-NMR (300MHz, CDCl₃): δ = 2.49 (s, 3H), 3.97 (dd, 1H), 4.13 (t, 1H), 4.60 (d, 2H), 5.03 (m, 1H), 6.77 (d, 1H), 6.92 (dd, 1H), 7.30 (d, 2H), 7.51 (d, 2H), 7.58 (m, 1H), 8.13 (d, 1H). MS: ESP⁺ (M+H)⁺= 317.

### Example 17: 5(R)-Pyrid-3-yloxymethyl-3-(3-fluoro-4-(3,6-dihydro-(2H)-pyran-4-yl)phenyl)oxazolidin-2-one

Prepared by the general method of Example 6 using the same starting material, (400mg, 1.08mmol), sodium hydride (48mg, 1.2mmol of a 60% dispersion) and 3-hydroxypyridine (108mg, 1.14mmol) in DMF (5ml). Purified by flash chromatography (Merck 9385 silica, 3% methanol / dichloromethane) to give the title compound (231mg, 58%) as a colourless solid.
¹H-NMR (300MHz, CDCl₃): δ = 2.50 (m, 2H), 3.92 (t, 2H), 4.08 (dd, 1H), 4.21 (t, 1H), 4.26-4.34 (m, 4H), 5.03 (m, 1H), 6.06 (m, 1H), 7.20-7.34 (m, 4H), 7.44 (d, 1H), 8.30 (m, 1H), 8.34 (m, 1H). MS: ESP⁺ (M+H)⁺= 371.

There is no Example 18.

### Example 19: 5(R)-(6-Methylpyrid-2-yloxymethyl)-3-(3-fluoro-4-(3,6-dihydro(2H)-pyran-4-yl)phenyl)oxazolidin-2-one

Prepared by the general method of Example 7 the same starting material (300mg, 1.02mmol), 2-hydroxy-6-methylpyridine (167mg, 1.53mmol), diisopropylazodicarboxylate (290mg, 1.43mmol) and triphenylphosphine (402mg, 1.53mmol) in THF (8ml). Purified by flash chromatography (Merck 9385 silica, 40% ethyl acetate / isohexane) to give the title compound (197mg, 50%) as a crystalline solid. MS: ESP⁺ (M+H)⁺= 385.
¹H-NMR (300MHz, CDCl₃): δ = 2.44 (s, 3H), 2.50 (m, 2H), 3.91 (t, 2H), 4.00 (dd, 1H), 4.12 (t, 1H), 4.31 (m, 2H), 4.52-4.67 (m, 2H), 5.03 (m, 1H), 6.05 (m, 1H), 6.54 (d, 2H), 6.74 (d, 2H), 7.21-7.32 (m, 2H), 7.42 (d, 1H), 7.48 (t, 1H).

### Example 20: 5(R)-(Pyrid-4-yloxymethyl)-3-(3-fluoro-4-(3,6-dihydro-(2H)-pyran-4-yl)phenyl)oxazolidin-2-one

Prepared by the general method of Example 19 using the same starting material (300mg, 1.02mmol), 4 hydroxypyridine (107mg, 1.13mmol), diisopropylazodicarboxylate (227mg, 1.12mmol) and triphenylphosphine (305mg, 1.16mmol) in THF (5ml). Purified by flash chromatography (Merck 9385 silica, 5% methanol / dichloromethane) to give the title compound (150mg, 40%) as a crystalline solid.
¹H-NMR (300MHz, CDCl₃): δ = 2.51 (m, 2H), 3.92 (t, 2H), 4.04 (dd, 1H), 4.21 (t, 1H), 4.26-4.35 (m, 4H), 5.02 (m, 1H), 6.07 (m, 1H), 6.83 (d, 2H), 7.22-7.32 (m, 2H), 7.42 (d, 1H), 8.48 (d, 2H). MS: ESP⁺ (M+H)⁺= 371.

### Example 21: (5R)- Pyrazin-2-yloxymethyl 3-(4-(4-(6-Cyano-pyridazin-3-yl)piperazin-1-yl)-3-fluorophenyl)-oxazolidin-2-one

Essentially the method of Examples 2 & 3 was used starting from 3-(4-(4-(6-cyanopyridazin-3-yl)piperazin-1-yl)-3-fluorophenyl)-5(R)-hydroxymethyloxazolidin-2-one (prepared by analogy to Reference Example 1; 240 mg, 0.6 mM) and 2-chloropyrazine (68.7 mg, 0.6 mM). Crude material was precipitated and purified by chromatography on a 20 g silica Mega Bond Elut® column, eluting with a gradient increasing in polarity from 0 to 2.5% methanol in dichloromethane. Relevant fractions were combined and evaporated to give the title compound (151 mg).
MS (ESP): 477 (MH⁺) for C₂₃H₂₁FN₈O₃
NMR (DMSO-d₆) δ: 3.08 (t, 4H); 3.90 (t overlapping m, 5H); 4.18 (t, 1H); 4.53 (dd, 1H); 4.61 (dd, 1H); 5.08 (m, 1H); 7.11 (t, 1H); 7.23 (dd, 1H); 7.40 (d, 1H); 7.53 (dd, 1H); 7.87 (d, 1H); 8.24 (overlapping m, 2H); 8.33 (s, 1H).

### Example 22: (5R)- Pyridin-2-yloxymethyl-3-(4-(4-(6-Cyano-pyridazin-3-yl)piperazin-1-yl)-3-fluorophenyl)-oxazolidin-2-one

Essentially the method of Example 21 was used starting from 3-(4-(4-(6-cyanopyridazin-3-yl)piperazin-1-yl)-3-fluorophenyl)-5(R)-hydroxymethyloxazolidin-2-one (240 mg, 0.6 mM) and 2-fluoropyridine (58.2 mg, 0.6 mM), to give the title compound (54 mg). MS (ESP): 476 (MH⁺) for C₂₄H₂₂FN₇O₃
NMR (DMSO-d₆) δ: 3.08 (t, 4H); 3.90 (t overlapping m, 5H); 4.16 (t, 1H); 4.47 (dd, 1H); 4.54 (dd, 1H); 5.04 (m, 1H); 6.82 (d, 1H); 7.10 (t, 1H); 7.22 (t, 1H); 7.40 (d, 1H); 7.53 (dd, 1H); 7.87 (d, 1H); 8.24 (overlapping m, 2H); 8.33 (s, 1H).

### Example 23: (5R)-(6-Cyanopyridazin-3-yl)oxymethyl-3-(4-(4-(6-cyanopyridazin-3-yl)piperazin-1-yl)-3-fluorophenyl)-oxazolidin-2-one

Essentially the method of Example 21 was used starting from 3-(4-(4-(6-cyanopyridazin-3-yl)piperazin-1-yl)-3-fluorophenyl)-5(R)-hydroxymethyloxazolidin-2-one (240 mg, 0.6 mM) and 3-chloro-6-cyanopyridazine (84 mg, 0.7 mM), to give the title compound (60 mg). MS (ESP): 502 (MH⁺) for C₂₄H₂₀FN₉O₃
NMR (DMSO-d₆) δ: 3.09 (t, 4H); 3.92 (t, 4H); 3.96 (dd, 1H); 4.20 (t, 1H); 4.78 (dd, 1H); 4.85 (dd, 1H); 5.12 (m, 1H); 7.11 (t, 1H); 7.22 (dd, 1H); 7.40 (d, 1H); 7.53 (overlapping m, 2H); 7.88 (d, 1H); 8.24 (d, 1H).

### Example 24

The following illustrate representative pharmaceutical dosage forms containing a compound of the formula (I), an in-vivo hydrolysable ester or a pharmaceutically-acceptable salt thereof, including a pharmaceutically-acceptable salt of an in-vivo hydrolysable ester, (hereafter compound X), for therapeutic or prophylactic use in humans:

| (a) | **Tablet I** | mg/tablet |
|---|---|---|
| | Compound X | 500 |
| | Lactose Ph.Eur | 430 |
| | Croscarmellose sodium | 40 |
| | Polyvinylpyrrolidone | 20 |
| | Magnesium stearate | 10 |

| (b) | **Tablet II** | mg/tablet |
|---|---|---|
| | Compound X | 100 |
| | Lactose Ph.Eur | 179 |
| | Croscarmellose sodium | 12 |
| | Polyvinylpyrrolidone | 6 |
| | Magnesium stearate | 3 |

| (c) | **Tablet III** | mg/tablet |
|---|---|---|
| | Compound X | 50 |
| | Lactose Ph.Eur | 229 |
| | Croscarmellose sodium | 12 |
| | Polyvinylpyrrolidone | 6 |
| | Magnesium stearate | 3 |

| (d) | **Tablet IV** | mg/tablet |
|---|---|---|
| | Compound X1 | |
| | Lactose Ph.Eur | 92 |
| | Croscarmellose sodium | 4 |
| | Polyvinylpyrrolidone..... | 2 |
| | Magnesium stearate1 | |

| (e) | **Capsule** | mg/capsule |
|---|---|---|
| | Compound X | 10 |
| | Lactose Ph.Eur | 389 |
| | Croscarmellose sodium | 100 |
| | Magnesium stearate | 1 |

| (f) | **Injection I** | |
|---|---|---|
| | Compound X | 50% w/v |
| | Isotonic aqueous solution | to 100% |

| (g) | **Injection II (e.g. bolus)** | |
|---|---|---|
| | Compound X | 10% w/v |
| | Isotonic aqueous solution | to 100% |

| (h) | **Injection III** | |
|---|---|---|
| | Compound X | 5% w/v |
| | Isotonic aqueous solution | to 100% |

| (i) | **Injection IV (e.g. infusion)** | |
|---|---|---|
| | Compound X | 1% w/v |
| | Isotonic aqueous solution | to 100% |

Buffers, pharmaceutically-acceptable surfactants, oils or cosolvents such as polyethylene glycol, polypropylene glycol, glycerol or ethanol, glidants (such as silicon dioxide) or complexing agents such as a cyclodextrin (for example, hydroxypropyl β-cyclodextrin or sulfo-butyl-ether β-cyclodextrin) may be used to aid formulation. Also, improvements in aqueous solubility, if desired, may be achieved, for example, by conjugation of a compound of formula (I) with a phospholipid (such as a (phospho)choline derivative) to form a micellar emulsion. Note : The above formulations may be obtained by conventional procedures well known in the pharmaceutical art, for example as described in "Remington : The Science & Practice of Pharmacy" Vols. I & II (Ed. A.R.Gennaro (Chairman) et al; Publisher : Mack Publishing Company, Easton, Pennsylvania; 19th Edition - 1995) and "Pharmaceutics - The Science of Dosage Form Design" (Ed. M.E.Aulton; Publisher : Churchill Livingstone; first published 1988). The tablets (a)-(d) may be (polymer) coated by conventional means, for example to provide an enteric coating of cellulose acetate phthalate.

## Claims

1. A compound of the formula (I), or a pharmaceutically-acceptable salt thereof, 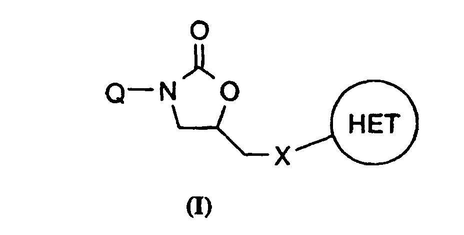 wherein X is -O- or -S-;
HET is a C-linked 6-membered heteroaryl ring containing 1 or 2 N, which ring is optionally substituted on any available C atom (provided that when the N atom is adjacent to the X-link, there is no substitution on any C atom that is adjacent to this N atom) by 1, 2 or 3 substituents independently selected from (1-4C)alkyl, amino, (1-4C)alkylamino, (1-4C)alkoxy, (1-4C)alkoxycarbonyl and halogen;
Q is Q1 :- 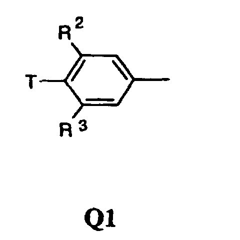 wherein R² and R³ are independently hydrogen or fluoro;
wherein T is selected from the groups in (TA) to (TC) below (wherein AR1, AR2, AR2a, AR2b, CY1 and CY2 are defined hereinbelow);
(**TA**) T is selected from the following groups :-
(**TAf**) an N-linked (fully unsaturated) 5-membered heteroaryl ring system containing 1, 2 or 3 nitrogen atoms selected from a group of formula (TAf1) to (TAf6) :- 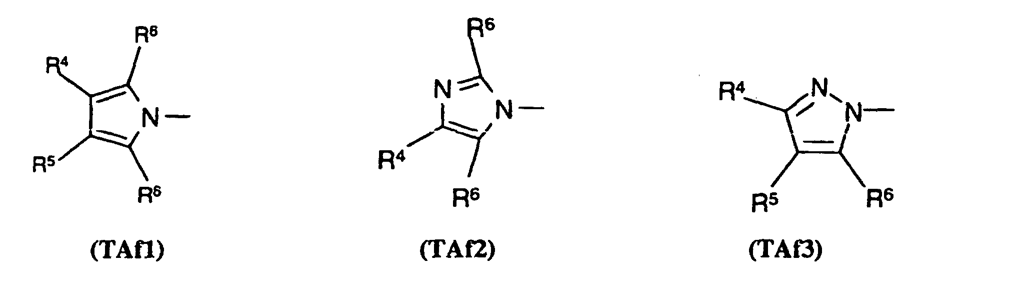 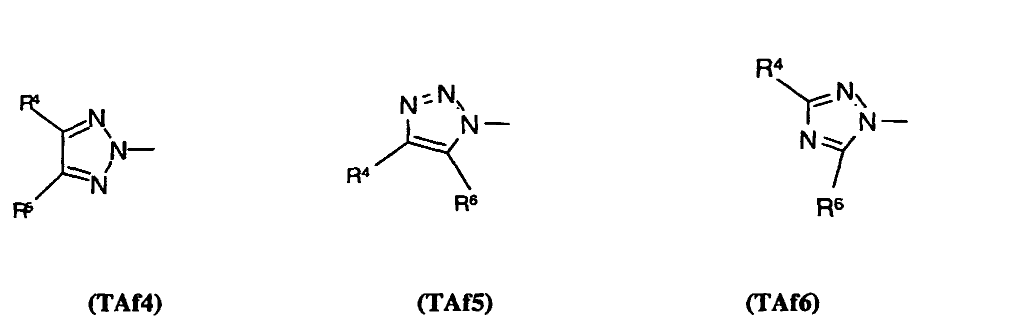
wherein :
R⁶ is selected (independently where appropriate) from hydrogen, (1-4C)alkyl, (1-4C)alkoxycarbonyl, (1-4C)alkanoyl, carbamoyl and cyano;
R⁴ and R⁵ are independently selected from hydrogen, halo, trifluoromethyl, cyano, nitro, (1-4C)alkoxy, (1-4C)alkylS(O)_{q}- (q is 0, 1 or 2), (1-4C)alkanoyl, (1-4C)alkoxycarbonyl, (2-4C)alkanoyloxy-(1-4C)alkyl, benzoxy-(1-4C)alkyl, (2-4C)alkanoylamino, -CONRvRw, -NRvRw and (1-4C)alkyl {optionally substituted by hydroxy, trifluoromethyl, cyano, nitro, (1-4C)alkoxy, (1-4C)alkylS(O)_{q}- (q is 0, 1 or 2), (1-4C)alkoxycarbonyl, (1-4C)alkanoylamino, - CONRvRw, -NRvRw; wherein RvRw is hydrogen or (1-4C)alkyl; Rw is hydrogen or (1-4C)alkyl}; or
**(TB)** T is selected from the following groups :-
**(TBa)** halo or (1-4C)alkyl
{optionally substituted by one or more groups each independently selected from hydroxy, (1-4C)alkoxy, (1-4C)alkanoyl, cyano, halo, trifluoromethyl, (1-4C)alkoxycarbonyl, -NRvRw, (1-6C)alkanoylamino, (1-4C)alkoxycarbonylamino, N-(1-4C)alkyl-N-(1-6C)alkanoylamino, (1-4C)alkylS(O)_{q}- (q is 0, 1 or 2), CY1, CY2 or AR1};
**(TBb)** -NRv¹Rw¹ ;
**(TBc)** ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl;
**(TBd)** R¹⁰CO- , R¹⁰S(O)_{q}- (q is 0, 1 or 2) or R¹⁰CS-
wherein R¹⁰ is selected from the following groups :-
***(TBda)*** CY1 or CY2;
***(TBdb)*** hydrogen, (1-4C)alkoxycarbonyl, trifluoromethyl, -NRvRw, ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl or 2-(AR2)ethenyl; or
***(TBdc)*** (1-4C)alkyl {optionally substituted as defined in (TBa) above, or by (1-4C)alkylS(O)ₚNH- or (1-4C)alkylS(O)ₚ-((1-4C)alkyl)N- (p is 1 or 2)};
wherein Rv is hydrogen or (1-4C)alkyl; Rw is hydrogen or (1-4C)alkyl; Rv¹ is hydrogen, (1-4C)alkyl or (3-8C)cycloalkyl; Rw¹ is hydrogen, (1-4C)alkyl, (3-8C)cycloalkyl, (1-4C)alkyl-CO- or (1-4C)alkylS(O)_{q}- (q is 1 or 2); or
**(TC)** T is selected from the following groups (TC1) to (TC4):- 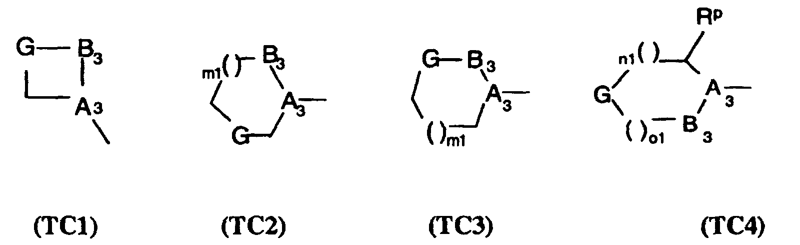 wherein in (TC1) : >A₃-B₃- is >C(Rq)-CH(Rr)- and G is -O-, -S-, -SO-, -SO₂- or >N(Rc);
wherein in (TC2) : m1 is 0, 1 or 2; >A₃-B₃- is >C=C(Rr)- or >C(Rq)-CH(Rr)- and G is -O-, - S-, -SO-, -SO₂- or >N(Rc);
wherein in (TC3) : m1 is 0, 1 or 2; >A₃-B₃- is >C(Rq)-CH(Rr)- (other than when Rq and Rr are both together hydrogen) and G is -O-, -S-, -SO-, -SO₂- or >N(Rc);
wherein in (TC4) : n1 is 1 or 2; o1 is 1 or 2 and n1 + o1 = 2 or 3; >A₃-B₃- is >C=C(Rr)- or >C(Rq)-CH(Rr)- or >N-CH₂- and G is -O-, -S-, -SO-, -SO₂- or >N(Rc); Rp is hydrogen, (1-4C)alkyl (other than when such substitution is defined by >A₃-B₃-), hydroxy, (1-4C)alkoxy or (1-4C)alkanoyloxy;
wherein in (TC1), (TC2) and (TC4); m1, n1 and o1 are as defined hereinbefore :
>A₃-B₃- is >N-CH₂- and G is >C(R¹¹)(R¹²), >C=O, >C-OH, >C-(1-4C)alkoxy, >C=N-OH, >C=N-(1-4C)alkoxy, >C=N-NH-(1-4C)alkyl, >C=N-N((1-4C)alkyl)₂ (the last two (1-4C)alkyl groups above in G being optionally substituted by hydroxy) or >C=N-N-CO-(1-4C)alkoxy; wherein > represents two single bonds;
Rq is hydrogen, hydroxy, halo, (1-4C)alkyl or (1-4C)alkanoyloxy;
Rr is (independently where appropriate) hydrogen or (1-4C)alkyl;
R¹¹ is hydrogen, (1-4C)alkyl, fluoro(1-4C)alkyl, (1-4C)alkyl-thio-(1-4C)alkyl or hydroxy-(1-4C)alkyl and R¹² is -[C(Rr)(Rr)]ₘ₂-N(Rr)(Rc) wherein m2 is 0, 1 or 2;
and, other than the ring substitution defined by G, >A₃-B₃- and Rp, each ring system may be optionally further substituted on a carbon atom not adjacent to the link at >A₃- by up to two substituents independently selected from (1-4C)alkyl, fluoro(1-4C)alkyl (including trifluoromethyl), (1-4C)alkyl-thio-(1-4C)alkyl, hydroxy-(1-4C)alkyl, amino, amino-(1-4C)alkyl, (1-4C)alkanoylamino, (1-4C)alkanoylamino-(1-4C)alkyl, carboxy, (1-4C)alkoxycarbonyl, AR-oxymethyl, AR-thiomethyl, oxo (=O) (other than when G is >N-Rc and Rc is group (Rc2)) or independently selected from Rc; and also hydroxy or halo (the last two optional substituents only when G is -O- or -S-);
wherein
AR is optionally substituted phenyl; optionally substituted phenyl(1-4C)alkyl; optionally substituted naphthyl; optionally substituted 5- or 6-membered heteroaryl, being a 5- or 6-membered aryl ring wherein 1, 2 or 3 of the ring atoms are selected from nitrogen, oxygen and sulfur and the rings are fully aromatic; optionally substituted 5/6 or 6/6 bicyclic heteroaryl ring system being an aromatic bicyclic ring system comprising a 6-membered ring fused to either a 5 membered ring or another 6 membered ring, the bicyclic ring system containing 1 to 4 heteroatoms selected from nitrogen, oxygen and sulfur in which the bicyclic heteroaryl ring systems may be linked via an atom in either of the rings comprising the bicyclic system, and wherein both the mono- and bicyclic heteroaryl ring systems are linked via a ring carbon atom and may be (partially) hydrogenated;
wherein AR is optionally mono- or di-substituted by halo, (1-4C)alkyl , hydroxy, nitro, carbamoyl, (1-4C)alkylcarbamoyl, di-((1-4C)alkyl)carbamoyl, cyano, trifluoromethyl, trifluoromethoxy, amino, (1-4C)alkylamino, di((1-4C)alkyl)amino, (1-4C)alkyl S(O)_{q}- (q is 0, 1 or 2), carboxy, (1-4C)alkoxycarbonyl, (2-4C)alkenyl, (2-4C)alkynyl, (1-4C)alkanoyl, (1-4C)alkoxy, (1-4C)alkylS(O)₂amino, (1-4C)alkanoylamino, benzoylamino, benzoyl, phenyl (optionally substituted by up to three substituents selected from halo, (1-4C)alkoxy or cyano), furan, pyrrole, pyrazole, imidazole, triazole, pyrimidine, pyridazine, pyridine, isoxazole, oxazole, isothiazole, thiazole, thiophene, hydroxyimino(1-4C)alkyl, (1-4C)alkoxyimino(1-4C)alkyl, hydroxy-(1-4C)alkyl, halo-(1-4C)alkyl, nitro(1-4C)alkyl, amino(1-4C)alkyl, cyano(1-4C)alkyl, (1-4C)alkanesulfonamido, aminosulfonyl, (1-4C)alkylaminosulfonyl and di-((1-4C)alkyl)aminosulfonyl;
wherein Rc is selected from groups (Rc1) to (Rc5) :-
***(Rc1)*** (1-6C)alkyl {optionally substituted by one or more (1-4C)alkanoyl groups (including geminal disubstitution) and/or optionally monosubstituted by cyano, (1-4C)alkoxy, trifluoromethyl, (1-4C)alkoxycarbonyl, phenyl (optionally substituted as for AR1 defined hereinafter), (1-4C)alkylS(O)_{q}- (q is 0, 1 or 2); or, on any but the first carbon atom of the (1-6C)alkyl chain, optionally substituted by one or more groups (including geminal disubstitution) each independently selected from hydroxy and fluoro, and/or optionally monosubstituted by oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is 1 or 2)};
***(Rc2)*** R¹³CO- , R¹³SO₂- or R¹³CS-
wherein R¹³ is selected from (Rc2a) to (Rc2e) :-
***(Rc2a)*** AR1, AR2, AR2a, AR2b, CY1, CY2;
***(Rc2b)*** hydrogen, (1-4C)alkoxycarbonyl, trifluoromethyl, -NRvRw [wherein Rv is hydrogen or (1-4C)alkyl; Rw is hydrogen or (1-4C)alkyl], ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl,
2-((1-4C)alkoxycarbonyl)ethenyl, 2-(AR1)ethenyl, 2-(AR2)ethenyl, 2-(AR2a)ethenyl;
***(Rc2c)*** (1-10C)alkyl
{optionally substituted by one or more groups (including geminal disubstitution) each independently selected from hydroxy, (1-10C)alkoxy, (1-4C)alkoxy-(1-4C)alkoxy, (I-4C)alkoxy-(1-4C)alkoxy-(1-4C)alkoxy, (1-4C)alkanoyl and amino; and/or optionally substituted by one group selected from carboxy, phosphonate [phosphono, -P(O)(OH)₂, and mono- and di-(1-4C)alkoxy derivatives thereof], phosphinate [-P(OH)₂ and mono- and di-(1-4C)alkoxy derivatives thereof], phosphoryl [-O-P(O)(OH)₂, and mono- and di-(1-4C)alkoxy derivatives thereof], phosphiryl [-O-P(OH)₂ and mono- and di-(1-4C)alkoxy derivatives thereof], cyano, halo, trifluoromethyl, (1-4C)alkoxycarbonyl, (1-4C)alkoxy-(1-4C)alkoxycarbonyl, (1-4C)alkoxy-(1-4C)alkoxy-(1-4C)alkoxycarbonyl, (1-4C)alkylamino, di((1-4C)alkyl)amino, (1-6C)alkanoylamino, (1-4C)alkoxycarbonylamino, N-(1-4C)alkyl-N-(1-6C)alkanoylamino, (1-4C)alkylaminocarbonyl, di((1-4C)alkyl)aminocarbonyl, (1-4C)alkylS(O)ₚNH-, (1-4C)alkylS(O)ₚ-((1-4C)alkyl)N-, fluoro(1-4C)alkylS(O)ₚNH-, fluoro(1-4C)alkylS(O)ₚ((1-4C)alkyl)N-, (1-4C)alkylS(O)_{q}-, CY1, CY2, AR1, AR2, AR1-O-, AR2-O-, AR1-S(O)_{q}- , AR2-S(O)_{q}- , AR1-NH-, AR2-NH-, (p is 1 or 2 and q is 0, 1 or 2), and also AR2a, AR2b, versions of AR2 containing groups};
***(Rc2d)*** R¹⁴C(O)O(1-6C)alkyl wherein R¹⁴ is AR1, AR2, (1-4C)alkylamino, benzyloxy(1-4C)alkyl or (1-10C)alkyl {optionally substituted as defined for (Rc2c)};
***(Rc2e)*** R¹⁵O- wherein R¹⁵ is benzyl, (1-6C)alkyl {optionally substituted as defined for (Rc2c)}, CY1, CY2 or AR2b;
***(Rc4)*** trityl, AR1, AR2, AR2a, AR2b;
wherein
**AR1** is an optionally substituted phenyl or optionally substituted naphthyl;
**AR2** is selected from furan, pyrrole, thiophene, pyrazole, imidazole, pyridine, pyrimidine, pyrazine, pyridazine, 1,2,3- & 1,2,4-triazole, tetrazole, oxazole, isoxazole, oxazine, thiazole, isothiazole, 1,2,4- & 1,3,4-thiadiazole;
**AR2a** is selected from dihydropyrrole and tetrahydropyridine;
**AR2b** is selected from tetrahydrofuran, pyrrolidine, morpholine, thiomorpholine, piperazine, imidazoline, piperidine, 1,3-dioxolan-4-yl, 1,3-dioxan-4-yl, 1,3-dioxan-5-yl and 1,4-dioxan-2-yl;
**CY1** is an optionally substituted cyclobutyl, cyclopentyl or cyclohexyl ring;
**CY2** is an optionally substituted cyclopentenyl or cyclohexenyl ring;
wherein :
optional substituents on AR1, AR2, AR2a, AR2b, CY1 and CY2 are (on an available carbon atom) up to three substituents independently selected from (1-4C)alkyl {optionally substituted by (preferably one) substituents selected independently from hydroxy, trifluoromethyl, (1-4C)alkyl S(O)_{q}- (q is 0, 1 or 2) (this last substituent preferably on AR1 only), (1-4C)alkoxy, (1-4C)alkoxycarbonyl, cyano, nitro, (1-4C)alkanoylamino, -CONRvRw or -NRvRw}, trifluoromethyl, hydroxy, halo, nitro, cyano, thiol, (1-4C)alkoxy, (1-4C)alkanoyloxy, dimethylaminomethyleneaminocarbonyl, di(N-(1-4C)alkyl)aminomethylimino, carboxy, (1-4C)alkoxycarbonyl, (1-4C)alkanoyl, (1-4C)alkylSO₂amino, (2-4C)alkenyl {optionally substituted by carboxy or (1-4C)alkoxycarbonyl}, (2-4C)alkynyl, (1-4C)alkanoylamino, oxo (=O), thioxo (=S), (1-4C)alkanoylamino (the (1-4C)alkanoyl group being optionally substituted by hydroxy}, (1-4C)alkyl S(O)_{q}- (q is 0, 1 or 2) {the (1-4C)alkyl group being optionally substituted by one or more groups independently selected from cyano, hydroxy and (1-4C)alkoxy}, -CONRvRw or -NRvRw [wherein Rv is hydrogen or (1-4C)alkyl; Rw is hydrogen or (1-4C)alkyl].

2. A compound of the formula (I), or a pharmaceutically-acceptable salt thereof, as claimed in claim 1 wherein T is selected from the groups (TAf) and (TC) as defined in claim 1.

3. A compound of the formula (I), or a pharmaceutically-acceptable salt thereof, as claimed in any one of claims 1 to 2 wherein the groups in (TC) are defined by formulae (TC5) to (TC11) :- 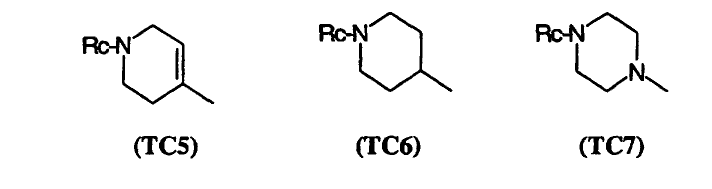 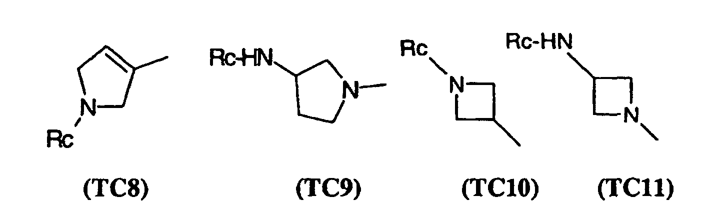 wherein Rc is as defined in claim 1.

4. A compound of the formula (I), or a pharmaceutically-acceptable salt thereof , as claimed in any one of claims 1 to 3 in which
the compound is the pure enantiomer depicted in formula (IA).

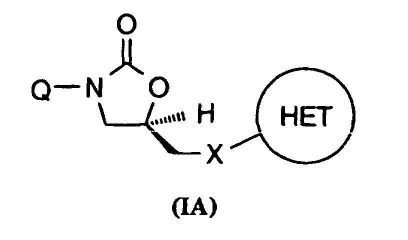

5. A compound of the formula (I) as claimed in claim 1, being a compound of the formula (IB), or a pharmaceutically-acceptable salt thereof 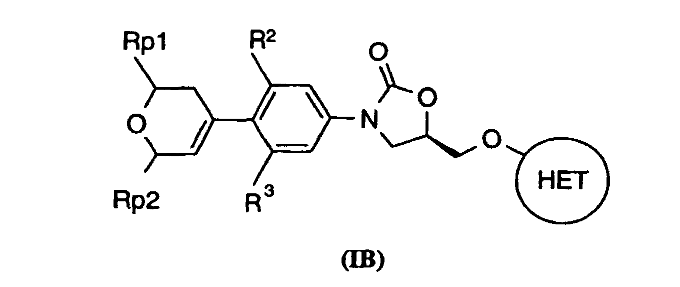 wherein HET is pyridin-2-yl or pyrazin-2-yl (especially pyridin-2-yl); R² and R³ are independently hydrogen or fluoro; and Rp1 and Rp2 are independently hydrogen, hydroxy, bromo, (1-4C)alkyl, carboxy, (1-4C)alkoxycarbonyl, hydroxymethyl, (1-4C)alkoxymethyl or carbamoyl .

6. A compound as claimed in claim 1, being :
5(R)-Pyrid-2-yloxymethyl-3-(4-(4-(5-cyanopyrid-2-yl)piperazin-1-yl)-3-fluorophenyl)oxazolidin-2-one;
5(R)-(6-Chloropyridazin-3-yloxymethyl)-3-(4-(4-(5-cyanopyrid-2-yl)piperazin-1-yl)-3-fluorophenyl)oxazolidin-2-one;
5(R)-Pyrazin-2-yloxymethyl-3-(4-(4-(5-cyanopyrid-2-yl)piperazin-1-yl)-3-fluorophenyl)oxazolidin-2-one;
5(R)-Pyrimidin-4-yloxymethyl-3-(4-(4-(5-cyanopyrid-2-yl)piperazin-1-yl)-3-fluorophenyl)oxazolidin-2-one;
5(R)-Pyridazin-3-yloxymethyl-3-(4-(4-(5-cyanopyrid-2-yl)piperazin-1-yl)-3-fluorophenyl)oxazolidin-2-one;
5(R)-Pyrid-2-yloxymethyl-3-(3-fluoro-4-(3,6-dihydro-(2H)-pyran-4-yl)phenyl)oxazolidin-2-one;
5(R)-(4-Methylpyrid-2-yloxymethyl)-3-(3-fluoro-4-(3,6-dihydro-(2H)-pyran-4-yl)phenyl)oxazolidin-2-one;
5(R)-(3-Methylpyrid-2-yloxymethyl)-3-(3-fluoro-4-(3,6-dihydro-(2H)-pyran-4-yl)phenyl)oxazolidin-2-one;
5(R)-(6-Chloropyridazin-3-yloxymethyl)-3-(3-fluoro-4-(3,6-dihydro-(2H)-pyran-4-yl)phenyl)oxazolidin-2-one;
5(R)-Pyridazin-3-yloxymethyl-3-(3-fluoro-4-(3,6-dihydro-(2H)-pyran-4-yl)phenyl)oxazolidin-2-one;
5(R)-Pyrimidin-4-yloxymethyl-3-(3-fluoro-4-(3,6-dihydro-(2H)-pyran-4-yl)phenyl)oxazolidin-2-one;
5(R)-Pyrazin-2-yloxymethyl-3-(3-fluoro-4-(3,6-dihydro-(2H)-pyran-4-yl)phenyl)oxazolidin-2-one;
5(R)-(5-Chloropyridin-2-yloxymethyl)-3-(3-fluoro-4-(3,6-dihydro-(2H)-pyran-4-yl)phenyl)oxazolidin-2-one; or pharmaceutically-acceptable salts thereof.

7. A compound as claimed in claim 1 being:
5(R)-Pyrid-2-yloxymethyl-3-(3-fluoro-4-(3,6-dihydro-(2H)-pyran-4-yl)phenyl)oxazolidin-2-one;
5(R)-Pyrazin-2-yloxymethyl-3-(3-fluoro-4-(3,6-dihydro-(2H)-pyran-4-yl)phenyl)oxazolidin-2-one; or pharmaceutically-acceptable salts thereof.

8. A process for the preparation of a compound of the formula (I) as claimed in claim 1 or pharmaceutically-acceptable salts thereof, which process comprises of **(a)** to **(f):-**
**(a)** modifying a substituent in or introducing a substituent into another compound of formula (I);
**(b)** reacting a compound of formula (II) 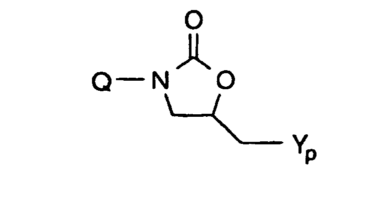 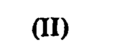 wherein Yp is hydroxy with a compound of the formula (b1) HET-OH or (b2) HET-Lg, wherein Lg is a suitable leaving group;
**(c)** reacting a compound of formula (II) wherein Yp is a leaving group with a metal alkoxide compound of the formula HET-OM where M is an alkali metal, or another metal known to promote O-alkylation;
**(d)** reacting a compound of the formula Q-Zp wherein Zp is an isocyanate or amine group with an epoxide of the formula CH₂(O)CH-CH₂O-HET;
**(e)** when X is -S- using a process analogous to process (c) using (e1) a metal thioxide compound of the formula HET-SM where M is an alkali metal, or another metal known to promote S-alkylation; or using (e2) HET-SH and a compound of formula (II) in which Yp is a suitable leaving group;
**(f)** reacting a urethane compound of formula (III) with a compound of formula (IV) 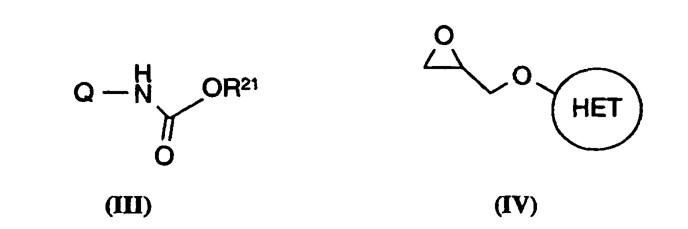
wherein R²¹ is (1-6C)alkyl or benzyl; and thereafter if necessary :
(i) removing any protecting groups; (ii) forming a pharmaceutically-acceptable salt.

9. A compound of the formula (I) as claimed in claims 1 to 7, or a pharmaceutically-acceptable salt thereof, for use as a medicament.

10. The use of a compound of the formula (I) as claimed in claims 1 to 7, or a pharmaceutically-acceptable salt thereof, in the manufacture of a medicament for use in the production of an antibacterial effect in a warm blooded animal.

11. A pharmaceutical composition which comprises a compound of the formula (I) as claimed in claims 1 to 7, or a phannaceutically-acceptable salt thereof, and a pharmaceutically-acceptable diluent or carrier.

## Patentansprüche

1. Verbindung der Formel (I) oder ein pharmazeutisch unbedenkliches Salz davon: 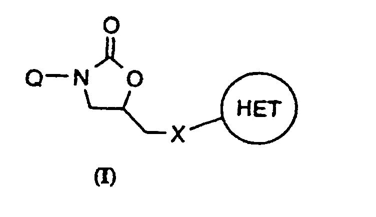 worin X für -O- oder -S- steht;
HET für einen C-verknüpften 6-gliedrigen Heteroarylring mit 1 oder 2 N steht, der gegebenenfalls an jedem beliebigen verfügbaren C-Atom (mit der Maßgabe, daß in dem Fall, daß das N-Atom zur X-Verknüpfung benachbart ist, ein diesem N-Atom benachbartes C-Atom nicht substituiert ist) durch 1, 2 oder 3, unabhängig voneinander unter C₁₋₄-Alkyl, Amino, C₁₋₄-Alkylamino, C₁₋₄-Alkoxy, C₁₋₄-Alkoxycarbonyl und Halogen ausgewählten Substituenten substituiert ist;
Q für Q1 steht: 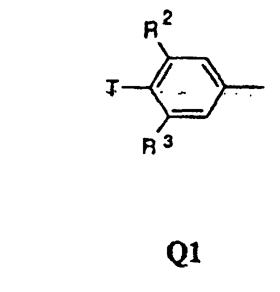 worin R² und R³ unabhängig voneinander für Wasserstoff oder Fluor stehen und
T unter den nachstehend in (TA) bis (TC) aufgeführten Gruppen ausgewählt ist (wobei AR1, AR2, AR2a, AR2b, CY1 und CY2 die nachstehend angegebene Bedeutung besitzen):
(TA) T unter den folgenden Gruppen ausgewählt ist:
(TAf) ein N-verknüpftes (vollständig ungesättigtes) 5-gliedriges Heteroarylringsystem mit 1, 2 oder 3 Stickstoffatomen, ausgewählt unter einer Gruppe der Formel (TAf1) bis (TAf6): 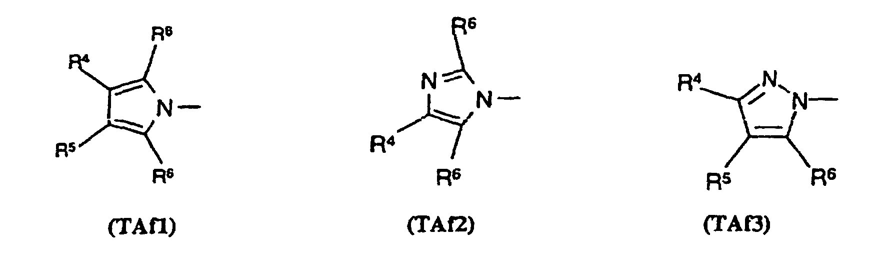 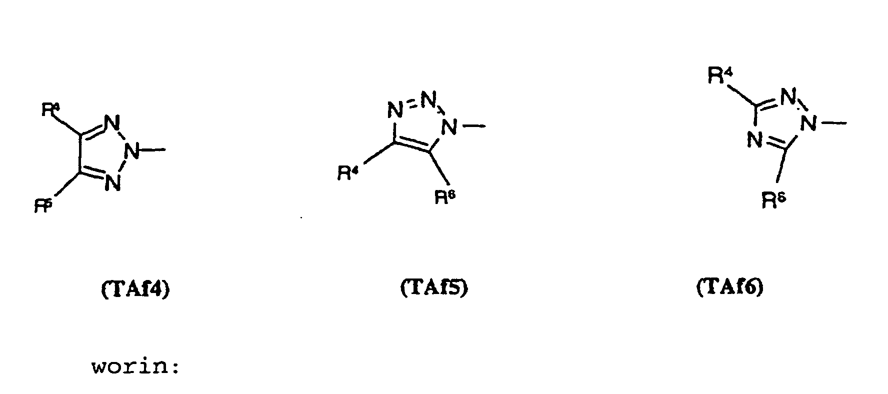 R⁶ (falls zutreffend, unabhängig) unter Wasserstoff, C₁₋₄-Alkyl, C₁₋₄-Alkoxycarbonyl, C₁₋₄-Alkanoyl, Carbamoyl und Cyano ausgewählt ist; R⁴ und R⁵ unabhängig voneinander unter Wasserstoff, Halogen, Trifluormethyl, Cyano, Nitro, C₁₋₄-Alkoxy, C₁₋₄-Alkyl-S(O)_{q}- (wobei q 0, 1 oder 2 bedeutet), C₁₋₄-Alkanoyl, C₁₋₄-Alkoxycarbonyl, C₂₋₄-Alkanoyloxy-C₁₋₄-alkyl, Benzoxy-C₁₋₄-alkyl, C₂₋₄-Alkanoylamino, -CONRvRw, -NRvRw und C₁₋₄-Alkyl {gegebenenfalls substituiert durch Hydroxy, Trifluormethyl, Cyano, Nitro, C₁₋₄-Alkoxy, C₁₋₄-Alkyl-S(O)_{q}- (wobei q 0, 1 oder 2 bedeutet), C₁₋₄-Alkoxycarbonyl, C₁₋₄-Alkanoylamino, -CONRvRw, -NRvRw; worin Rv für Wasserstoff oder C₁₋₄-Alkyl steht und Rw für Wasserstoff oder C₁₋₄-Alkyl steht} ausgewählt sind; oder
(TB) T unter den folgenden Gruppen ausgewählt ist:
(TBa) Halogen oder C₁₋₄-Alkyl
{gegebenenfalls substituiert durch eine oder mehrere, jeweils unabhängig voneinander unter Hydroxy, C₁₋₄-Alkoxy, C₁₋₄-Alkanoyl, Cyano, Halogen, Trifluormethyl, C₁₋₄-Alkoxycarbonyl, -NRvRw, C₁₋₆-Alkanoylamino, C₁₋₄-Alkoxycarbonylamino, N-C₁₋₄-Alkyl-N-C₁₋₆-alkanoylamino, C₁₋₄-Alkyl-S(O)_{q}- (wobei q 0, 1 oder 2 bedeutet), CY1, CY2 oder CY3 ausgewählte Gruppen);
(TBb) -NRv¹Rw¹;
(TBc) Ethenyl, 2-C₁₋₄-Alkylethenyl, 2-Cyanoethenyl, 2-Cyano-2-(C₁₋₄-alkyl)ethenyl, 2-Nitroethenyl, 2-Nitro-2-(C₁₋₄-alkyl)ethenyl, 2-(C₁₋₄-Alkylaminocarbonyl)ethenyl, 2-(C₁₋₄-Alkoxycarbonyl)ethenyl, 2-(AR1)-Ethenyl, 2-(AR2)-Ethenyl;
(TBd) R¹⁰CO-, R¹⁰S(O)_{q}- (worin q 0, 1 oder 2 bedeutet) oder R¹⁰CS-,
worin R¹⁰ unter den folgenden Gruppen ausgewählt ist:
(TBda) CY1 oder CY2;
(TBdb) Wasserstoff, C₁₋₄-Alkoxycarbonyl, Trifluormethyl, -NRvRw, Ethenyl, 2-C₁₋₄-Alkylethenyl, 2-Cyanoethenyl, 2-Cyano-2-(C₁₋₄-alkyl)ethenyl, 2-Nitroethenyl, 2-Nitro-2-(C₁₋₄-alkyl)ethenyl, 2-(C₁₋₄-Alkylaminocarbonyl)ethenyl, 2-(C₁₋₄-Alkoxycarbonyl)ethenyl, 2-(AR1)-Ethenyl oder 2-(AR2)-Ethenyl; oder
(TBdc) C₁₋₄-Alkyl {gegebenenfalls entsprechend der oben in (TBa) angegebenen Definition oder durch C₁₋₄-Alkyl-S(O)ₚNH- oder C₁₋₄-Alkyl-S(O)ₚ-(C₁₋₄alkyl)N- (wobei p 1 oder 2 bedeutet) substituiert};
worin Rv für Wasserstoff oder C₁₋₄-Alkyl steht; Rw für Wasserstoff oder C₁₋₄-Alkyl steht; Rv¹ für Wasserstoff, C₁₋₄-Alkyl oder C₃₋₈-Cycloalkyl steht; Rw¹ für Wasserstoff, C₁₋₄-Alkyl, C₃₋₈-Cycloalkyl, C₁₋₄-Alkyl-CO oder C₁₋₄-Alkyl-S(O)_{q}- (wobei q 1 oder 2 bedeutet), steht; oder
(TC) T unter den folgenden Gruppen (TC1) bis (TC4) ausgewählt ist: 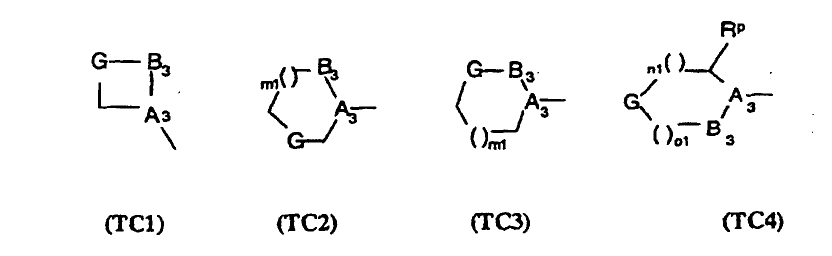 wobei in (TC1): >A₃-B₃- für >C (Rq) -CH (Rr) - steht und G für -O-, -S-, -SO-, -SO₂- oder >N(Rc) steht;
wobei in (TC2): m1 für 0, 1 oder 2 steht, >A₃-B₃für >C=C(Rr)- oder >C(Rq)-CH(Rr)- steht und G für -O-, -S-, -SO-, -SO₂- oder >N(Rc) steht;
wobei in (TC3): m1 für 0, 1 oder 2 steht, >A₃-B₃für >C=C(Rr)- oder >C(Rq)-CH(Rr)- steht (außer wenn Rq und Rr beide zusammen Wasserstoff bedeuten) und G für -O-, -S-, -SO-, -SO₂- oder >N(Rc) steht;
wobei in (TC4): n1 für 1 oder 2 steht; o1 für 1 oder 2 steht und die Summe n1 + o1 gleich 2 oder 3 ist; >A₃-B₃- für >C=C(Rr)- oder >C(Rq)-CH(Rr)- oder >N-CH₂- steht und G für -O-, -S-, -SO-, -SO₂- oder >N(Rc) steht; Rp für Wasserstoff, C₁₋₄-Alkyl (außer wenn eine derartige Substitution durch >A₃-B₃definiert ist), Hydroxy, C₁₋₄-Alkoxy oder C₁₋₄-Alkanoyloxy steht;
wobei in (TC1), (TC2) und (TC4): m1, n1 und o1 die oben angegebene Bedeutung besitzen; >A₃-B₃- für >N-CH₂- steht und G für >C(R¹¹)(R¹²), >C=O, >C-OH, >C₁₋₄-Alkoxy, >C=N-OH, >C=N-C₁₋₄-Alkoxy, >C=N-NH-C₁₋₄-Alkyl, >C=N-N(C₁₋₄-Alkyl)₂ (wobei die beiden letzten C₁₋₄-Alkylgruppen oben in G gegebenenfalls durch Hydroxy substituiert sein können) oder >C=N-N-CO-C₁₋₄-Alkoxy steht; wobei > für zwei Einfachbindungen steht;
Rq für Wasserstoff, Hydroxy, Halogen, C₁₋₄-Alkyl oder C₁₋₄-Alkanoyloxy steht;
Rr (falls zutreffend, unabhängig) für Wasserstoff oder C₁₋₄-Alkyl steht;
R¹¹ für Wasserstoff, C₁₋₄-Alkyl, Fluor-C₁₋₄-alkyl, C₁₋₄-Alkylthio-C₁₋₄-alkyl oder Hydroxy-C₁₋₄-alkyl steht und R¹² für -[C(Rr)(Rr)]ₘ₂-N(Rr)(Rc), worin m2 0, 1 oder 2 bedeutet, steht;
und jedes Ringsystem abgesehen von durch G, >A₃-B₃und Rp definierten Ringsubstitution gegebenenfalls ferner an einem nicht der Verknüpfung an >A₃benachbarten Kohlenstoffatom durch bis zu zwei, unabhängig voneinander unter C₁₋₄-Alkyl, Fluor-C₁₋₄alkyl (einschließlich Trifluormethyl), C₁₋₄-Alkylthio-C₁₋₄-alkyl, Hydroxy-C₁₋₄-alkyl, Amino, Amino-C₁₋₄-alkyl, C₁₋₄-Alkanoylamino, C₁₋₄-Alkanoylamino-C₁₋₄-alkyl, Carboxy, C₁₋₄-Alkoxycarbonyl, AR-Oxymethyl, AR-Thiomethyl, Oxo (=O) (außer wenn G für >N-Rc und Rc für eine Gruppe (Rc2) steht) oder unabhängig voneinander unter Rc und auch Hydroxy oder Halogen (die letzten beiden fakultativen Substituenten nur dann, wenn G für -O- oder -Ssteht) ausgewählte Substituenten substituiert sein kann;
worin
AR für gegebenenfalls substituiertes Phenyl; gegebenenfalls substituiertes Phenyl-C₁₋₄-alkyl; gegebenenfalls substituiertes 5- oder 6-gliedriges Heteroaryl, bei dem es sich um einen 5- oder 6-gliedrigen Arylring handelt, in dem 1, 2 oder 3 der Ringatome unter Stickstoff, Sauerstoff und Schwefel ausgewählt sind und die Ringe vollaromatisch sind; ein gegebenenfalls substituiertes 5/6- oder 6/6-bicyclisches Heteroarylringsystem, bei dem es sich um ein aromatisches bicyclisches Ringsystem handelt, das einen 6-gliedrigen Ring, der entweder mit einem 5-gliedrigen Ring oder einem anderen 6-gliedrigen Ring anelliert ist, und 1 bis 4, unter Stickstoff, Sauerstoff und Schwefel ausgewählte Heteroatome enthält, worin die bicyclischen Heteroarylringsysteme über ein Atom in einem der beiden das bicyclische System bildenden Ringe verknüpft sein können und sowohl die monocyclischen als auch die bicylischen Heteroarylringsysteme über ein Ringkohlenstoffatom verknüpft sind und gegebenenfalls (partiell) hydriert sein können, steht;
worin AR gegebenenfalls ein- oder zweifach durch Halogen, C₁₋₄-Alkyl, Hydroxy, Nitro, Carbamoyl, C₁₋₄-Alkylcarbamoyl, Di(C₁₋₄-alkyl)carbamoyl, Cyano, Trifluormethyl, Trifluormethoxy, Amino, C₁₋₄-Alkylamino, Di(C₁₋₄-alkyl)amino, C₁₋₄-Alkyl-S(O)_{q}- (wobei q 0, 1 oder 2 bedeutet), Carboxy, C₁₋₄-Alkoxycarbonyl, C₂₋₄-Alkenyl, C₂₋₄-Alkinyl, C₁₋₄-Alkanoyl, C₁₋₄-Alkoxy, C₁₋₄-Alkyl-S(O)₂-amino, C₁₋₄-Alkanoylamino, Benzoylamino, Benzoyl, Phenyl (gegebenenfalls substituiert durch bis zu drei unter Halogen, C₁₋₄-Alkoxy oder Cyano ausgewählte Substituenten), Furan, Pyrrol, Pyrazol, Imidazol, Triazol, Pyrimidin, Pyridazin, Pyridin, Isoxazol, Oxazol, Isothiazol, Thiazol, Thiophen, Hydroxyimino-C₁₋₄-alkyl, C₁₋₄-Alkoxyimino-C₁₋₄-alkyl, Hydroxy-C₁₋₄-alkyl, Halogen-C₁₋₄-alkyl, Nitro-C₁₋₄alkyl, Amino-C₁₋₄-alkyl, Cyano-C₁₋₄-alkyl, C₁₋₄-Alkansulfonamido, Aminosulfonyl, C₁₋₄-Alkylaminosulfonyl und Di(C₁₋₄-alkyl)aminosulfonyl substituiert sein kann;
worin Rc unter den folgenden Gruppen (Rc1) bis (Rc5) ausgewählt ist:
(Rc1) C₁₋₆-Alkyl {gegebenenfalls substituiert durch eine oder mehrere C₁₋₄-Alkanoylgruppen (einschließlich geminaler Disubstitution) und/oder gegebenenfalls einfach substituiert durch Cyano, C₁₋₄-Alkoxy, Tri f luormethyl, C₁₋₄-Alkoxycarbonyl, Phenyl (gegebenenfalls entsprechend der nachstehend für AR1 angegebenen Definition substituiert), C₁₋₄-Alkyl-S(O)_{q}- (wobei q 0, 1 oder 2 bedeutet); oder an jedem außer dem ersten Kohlenstoffatom der C₁₋₆-Alkylkette gegebenenfalls substituiert durch eine oder mehrere, unabhängig voneinander unter Hydroxy und Fluor ausgewählte Gruppen (einschließlich geminaler Disubstitution) und/oder gegebenenfalls einfach substituiert durch Oxo, -NRvRw [worin Rv für Wasserstoff oder C₁₋₄-Alkyl steht; Rw für Wasserstoff oder C₁₋₄-Alkyl steht], C₁₋₆-Alkanoylamino, C₁₋₄-Alkoxycarbonylamino, N-C₁₋₄-Alkyl-N-C₁₋₆-alkanoylamino, C₁₋₄-Alkyl-S(O)ₚ-NH oder C₁₋₄-Alkyl-S(O)ₚ-(C₁₋₄-alkyl)N- (wobei p 1 oder 2 bedeutet)};
(Rc2) R¹³CO-, R¹³SO₂- oder R¹³CS-,
worin R¹³ unter (Rc2a) bis (Rc2e) ausgewählt ist:
(R2ca) AR1, AR2, AR2a, AR2b, CY1, CY2;
(R2cb) Wasserstoff, C₁₋₄-Alkoxycarbonyl, Trifluormethyl, -NRvRw [worin Rv für Wasserstoff oder C₁₋₄-Alkyl steht; Rw für Wasserstoff oder C₁₋₄-Alkyl steht], Ethenyl, 2-C₁₋₄-Alkylethenyl, 2-Cyanoethenyl, 2-Cyano-2-(C₁₋₄-alkyl)ethenyl, 2-Nitroethenyl, 2-Nitro-2-(C₁₋₄-alkyl)ethenyl, 2-(C₁₋₄-Alkylaminocarbonyl)ethenyl, 2-(C₁₋₄-Alkoxycarbonyl)ethenyl, 2-(AR1)-Ethenyl, 2-(AR2)-Ethenyl, 2-(AR2a)-Ethenyl;
(Rc2c) C₁₋₁₀-Alkyl
{gegebenenfalls substituiert durch eine oder mehrere, jeweils unabhängig voneinander unter Hydroxy, C₁₋₁₀-Alkoxy, C₁₋₄-Alkoxy-C₁₋₄-alkoxy, C₁₋₄-Alkoxy-C₁₋₄-alkoxy-C₁₋₄-alkoxy, C₁₋₄-Alkanoyl und Amino ausgewählte Gruppen (einschließlich geminaler Disubstitution) und/oder gegebenenfalls substituiert durch eine, unter Carboxy, Phosphonat [Phosphono, -P(O)(OH)₂ und Mono- und Di-C₁₋₄-alkoxyderivate davon], Phosphinat [-P(OH)₂ und Mono- und Di-C₁₋₄-alkoxyderivate davon], Phosphoryl [-O-P(O)(OH)₂ und Mono- und Di-C₁₋₄-alkoxyderivate davon], Phosphiryl [-O-P(OH)₂ und Mono- und Di-C₁₋₄alkoxyderivate davon], Cyano, Halogen, Trifluormethyl, C₁₋₄-Alkoxycarbonyl, C₁₋₄-Alkoxy-C₁₋₄-alkoxycarbonyl, C₁₋₄-Alkoxyl-C₁₋₄-alkoxy-C₁₋₄-alkoxycarbonyl, C₁₋₄-Alkylamino, Di-(C₁₋₄-alkyl)amino, C₁₋₆-Alkanoylamino, C₁₋₄-Alkoxycarbonylamino, N-C₁₋₄-Alkyl-N-C₁₋₆-alkanoylamino, C₁₋₄-Alkylaminocarbonyl, Di-(C₁₋₄-alkyl)aminocarbonyl, C₁₋₄-Alkyl-S(O)ₚNH-, C₁₋₄-Alkyl-S(O)ₚ-(C₁₋₄-alkyl)N-, Fluor-C₁₋₄-alkyl-S(O)ₚNH-, Fluor-C₁₋₄-alkyl-S(O)ₚ-(C₁₋₄-alkyl)N-, C₁₋₄-Alkyl-S(O)_{q}-, CY1, CY2, AR1, AR2, AR1-O-, AR2-O-, AR1-S(O)_{q}-, AR2-S(O)_{q}-, AR1-NH-, AR2-NH- (wobei p 1 oder 2 bedeutet und 9 0, 1 oder 2 bedeutet) sowie AR2a- und AR2b-Varianten von AR2 enthaltenden Gruppen ausgewählte Gruppe};
(Rc2d) R¹⁴C(O)O-C₁₋₆-alkyl, worin R¹⁴ für AR1, AR2, C₁₋₄-Alkylamino, Benzyloxy-C₁₋₄-alkyl oder C₁₋₁₀-Alkyl {gegebenenfalls entsprechend der für (Rc2c) angegebenen Definition substituiert};
(Rc2e) R¹⁵O-, worin R¹⁵ für Benzyl, C₁₋₆-Alkyl gegebenenfalls entsprechend der für (Rc2c) angegebenen Definition substituiert}, CY1, CY2 oder AR2b steht;
(Rc4) Trityl, AR1, AR2, AR2a, AR2b;
worin
AR1 für gegebenenfalls substituiertes Phenyl oder gegebenenfalls substituiertes Naphthyl steht;
AR2 unter Furan, Pyrrol, Thiophen, Pyrazol, Imidazol, Pyridin, Pyrimidin, Pyrazin, Pyridazin, 1,2,3- und 1,2,4-Triazol, Tetrazol, Oxazol, Isoxazol, Oxazin, Thiazol, Isothiazol, 1,2,4- und 1,3,4-Thiadiazol ausgewählt ist;
AR2a unter Dihydropyrrol und Tetrahydropyridin ausgewählt ist;
AR2b unter Tetrahydrofuran, Pyrrolidin, Morpholin, Thiomorpholin, Piperazin, Imidazolin, Piperidin, 1,3-Dioxolan-4-yl, 1,3-Dioxan-4-yl, 1,3-Dioxan-5-yl und 1,4-Dioxan-2-yl;
CY1 für einen gegebenenfalls substituierten Cyclobutyl-, Cyclopentyl- oder Cyclohexylring steht;
CY2 für einen gegebenenfalls substituierten Cyclopentenyl- oder Cyclohexenylring steht;
worin:
AR1, AR2, AR2a, AR2b, CY1 und CY2 gegebenenfalls (an einem verfügbaren Kohlenstoffatom) bis zu drei, unabhängig voneinander unter C₁₋₄-Alkyl (gegebenenfalls substitutiert durch unabhängig voneinander unter Hydroxy, Trifluormethyl, C₁₋₄-Alkyl-S(O)_{q}- (wobei q 0, 1 oder 2 bedeutet) (wobei dieser letzte Substituent vorzugsweise nur an AR1 steht), C₁₋₄-Alkoxy, C₁₋₄-Alkoxycarbonyl, Cyano, Nitro, C₁₋₄-Alkanoylamino, -CONRvRw oder -NRvRw ausgewählte Substituenten (vorzugsweise einen Substituenten)}, Trifluormethyl, Hydroxy, Halogen, Nitro, Cyano, Thiol, C₁₋₄-Alkyoxy, C₁₋₄-Alkanoyloxy, Dimethylaminomethylenaminocarbonyl, Di-(N-C₁₋₄alkyl)aminomethylimino, Carboxy, C₁₋₄-Alkoxycarbonyl, C₁₋₄-Alkanoyl, C₁₋₄-Alkyl-SO₂-amino, C₂₋₄-Alkenyl {gegebenenfalls substituiert durch Carboxy oder C₁₋₄-Alkoxycarbonyl}, C₂₋₄-Alkinyl, C₁₋₄-Alkanoylamino, Oxo (=O), Thioxo (=S), C₁₋₄-Alkanoylamino {wobei die C₁₋₄-Alkanoylgruppe gegebenenfalls durch Hydroxy substituiert sein kann}, C₁₋₄-Alkyl-S(O)_{q}- (wobei q 0, 1 oder 2 bedeutet) {wobei die C₁₋₄-Alkylgruppe gegebenenfalls durch eine oder mehrere, unabhängig voneinander unter Cyano, Hydroxy und C₁₋₄-Alkoxy ausgewählte Gruppen substituiert sein kann}, -CONRvRw oder -NRvRw [worin Rv für Wasserstoff oder C₁₋₄-Alkyl steht; Rw für Wasserstoff oder C₁₋₄-Alkyl steht] ausgewählte Substituenten tragen können.

2. Verbindung der Formel (I) oder ein pharmazeutisch unbedenkliches Salz davon nach Anspruch 1, worin T unter den Gruppen (TAf) und (TC) gemäß der in Anspruch 1 angegebenen Definition ausgewählt ist.

3. Verbindung der Formel (I) oder ein pharmazeutisch unbedenkliches Salz davon nach Anspruch 1 oder 2, worin die Gruppen in (TC) durch die Formeln (TC5) bis (TC11) definiert sind: 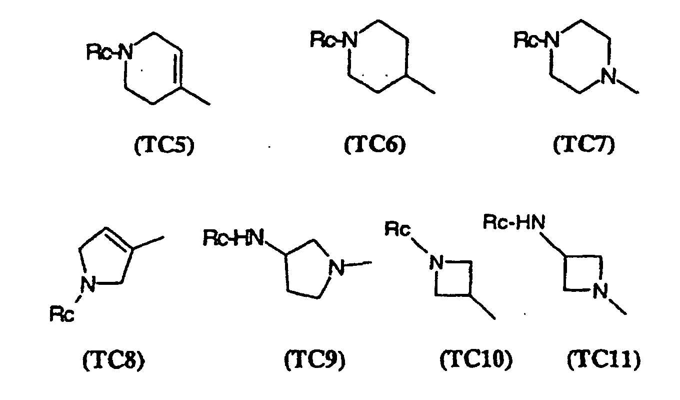 worin Rc die in Anspruch 1 angegebene Bedeutung besitzt.

4. Verbindung der Formel (I) oder ein pharmazeutisch unbedenkliches Salz davon nach einem der Ansprüche 1 bis 3, wobei es sich bei der Verbindung um das in Formel (IA) dargestellte reine Enantiomer handelt:

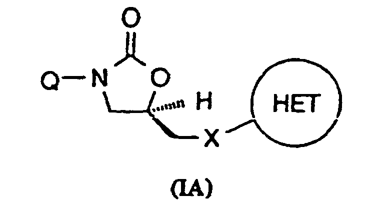

5. Verbindung der Formel (I) nach Anspruch 1, bei der es sich um eine Verbindung der Formel (IB) oder ein pharmazeutisch unbedenkliches Salz davon handelt: 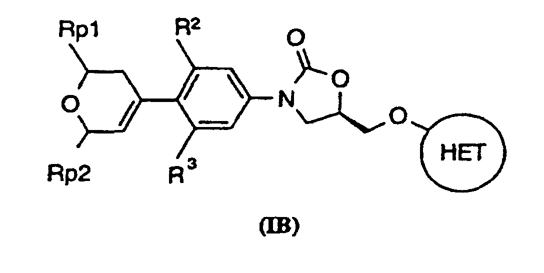 worin HET für Pyridin-2-yl oder Pyrazin-2-yl (insbesondere Pyridin-2-yl) steht; R² und R³ unabhängig voneinander für Wasserstoff oder Fluor stehen und Rp1 und Rp2 unabhängig voneinander für Wasserstoff, Hydroxy, Brom, C₁₋₄-Alkyl, Carboxy, C₁₋₄-Alkoxycarbonyl, Hydroxymethyl, C₁₋₄-Alkoxymethyl oder Carbamoyl stehen.

6. Verbindung nach Anspruch 1, bei der es sich um 5(R)-Pyrid-2-yloxymethyl-3-(4-(4-(5-cyanopyrid-2-yl)piperazin-1-yl)-3-fluorphenyl)oxazolidin-2-on;
5(R)-(6-Chlorpyridazin-3-yloxymethyl)-3-(4-(4-(5-cyanopyrid-2-yl)piperazin-1-yl)-3-fluorphenyl)-oxazolidin-2-on;
5(R)-Pyrazin-2-yloxymethyl-3-(4-(4-(5-cyanopyrid-2-yl)piperazin-1-yl)-3-fluorphenyl)oxazolidin-2-on;
5(R)-Pyrimidin-4-yloxymethyl-3-(4-(4-(5-cyanopyrid-2-yl)piperazin-1-yl)-3-fluorphenyl)-oxazolidin-2-on;
5(R)-Pyridazin-3-yloxymethyl-3-(4-(4-(5-cyanopyrid-2-yl)piperazin-1-yl)-3-fluorphenyl)-oxazolidin-2-on;
5(R)-Pyrid-2-yloxymethyl-3-(3-fluor-4-(3,6-dihydro-(2H)-pyran-4-yl)phenyl)oxazolidin-2-on;
5(R)-(4-Methylpyrid-2-yloxymethyl-3-(3-fluor-4-(3,6-dihydro-(2H)-pyran-4-yl)phenyl)oxazolidin-2-on;
5(R)-(3-Methylpyrid-2-yloxymethyl-3-(3-fluor-4-(3,6-dihydro-(2H)-pyran-4-yl)phenyl)oxazolidin-2-on;
5(R)-(6-Chlorpyridazin-3-yloxymethyl-3-(3-fluor-4-(3,6-dihydro-(2H)-pyran-4-yl)phenyl)oxazolidin-2-on;
5(R)-Pyridazin-3-yloxymethyl-3-(3-fluor-4-(3,6-dihydro-(2H)-pyran-4-yl)phenyl)oxazolidin-2-on;
5(R)-Pyrimidin-4-yloxymethyl-3-(3-fluor-4-(3,6-dihydro-(2H)-pyran-4-yl)phenyl)oxazolidin-2-on;
5(R)-Pyrazin-2-yloxymethyl-3-(3-fluor-4-(3,6-dihydro-(2H)-pyran-4-yl)phenyl)oxazolidin-2-on;
5(R)-(5-Chlorpyridin-2-yloxymethyl)-3-(3-fluor-4-(3,6-dihydro-(2H)-pyran-4-yl)phenyl)oxazolidin-2-on; oder pharmazeutisch unbedenkliche Salze davon handelt.

7. Verbindung nach Anspruch 1, bei der es sich um
5(R)-Pyrid-2-yloxymethyl-3-(3-fluor-4-(3,6-dihydro-(2H)-pyran-4-yl)phenyl)oxazolidin-2-on;
5(R)-Pyrazin-2-yloxymethyl-3-(3-fluor-4-(3,6-dihydro-(2H)-pyran-4-yl)phenyl)oxazolidin-2-on; oder pharmazeutisch unbedenkliche Salze davon handelt.

8. Verfahren zur Herstellung einer Verbindung der Formel (I) nach Anspruch 1 oder pharmazeutisch unbedenklichen Salzen davon, bei dem man
(a) in einer anderen Verbindung der Formel (I) einen Substituenten modifiziert oder in eine andere Verbindung der Formel (I) einen Substituenten einführt;
(b) eine Verbindung der Formel (II) 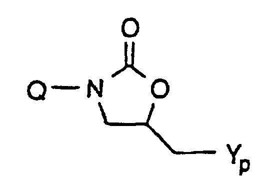 worin Yp für Hydroxy steht, mit einer Verbindung der Formel (b1) HET-OH oder (b2) HET-Lg, worin Lg für eine geeignete Abgangsgruppe steht, umsetzt;
(c) eine Verbindung der Formel (II), worin Yp für eine Abgangsgruppe steht, mit einer Metallalkoholatverbindung der Formel HET-OM, worin M für ein Alkalimetall oder ein anderes, bekanntlich die O-Alkylierung förderndes Metall steht;
(d) eine Verbindung der Formel Q-Zp, worin Zp für eine Isocyanat- oder Amingruppe steht, mit einem Epoxid der Formel CH₂(O)CH-CH₂O-HET umsetzt;
(e) wenn X für -S- steht, in Analogie zu Verfahren (c) vorgeht und dabei (e1) eine Metallthiolatverbindung der Formel HET-SM, worin M für ein Alkalimetall oder ein anderes, bekanntlich die S-Alkylierung förderndes Metall steht, oder (e2) HET-SH und eine Verbindung der Formel (II), worin Yp für eine geeignete Abgangsgruppe steht, verwendet;
(f) eine Urethanverbindung der Formel (III) mit einer Verbindung der Formel (IV) 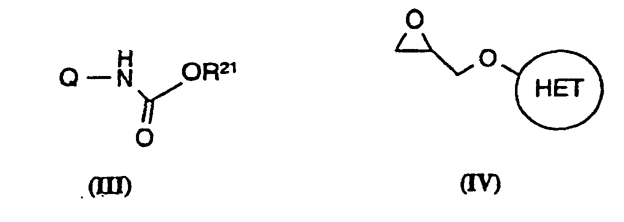
worin R²¹ für C₁₋₆-Alkyl oder Benzyl steht, umsetzt; und danach gegebenenfalls:
(i) Schutzgruppen abspaltet und (ii) ein pharmazeutisch unbedenkliches Salz bildet.

9. Verbindung der Formel I nach den Ansprüchen 1 bis 7 oder ein pharmazeutisch unbedenkliches Salz davon zur Verwendung als Arzneimittel.

10. Verwendung einer Verbindung der Formel (I) nach den Ansprüchen 1 bis 7 oder eines pharmazeutisch unbedenklichen Salzes davon bei der Herstellung eines Arzneimittels zur Verwendung bei der Hervorrufung einer antibakteriellen Wirkung bei einem Warmblüter.

11. Pharmazeutische Zusammensetzung, die eine Verbindung der Formel (I) nach den Ansprüchen 1 bis 7 oder ein pharmazeutisch unbedenkliches Salz davon und ein pharmazeutisch unbedenkliches Verdünnungsmittel oder einen pharmazeutisch unbedenklichen Träger enthält.

## Revendications

1. Composé de formule (I), ou un sel pharmaceutiquement acceptable de celui-ci : 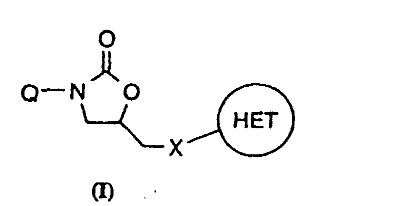 dans laquelle X est -O- ou -S- ;
HET est un cycle hétéroaryle à 6 chaînons lié par C, contenant 1 ou 2 N, lequel cycle est éventuellement substitué sur tout atome de C disponible (à condition que lorsque l'atome de N est adjacent au lien X, il n'y a pas de substitution sur un atome de C quelconque qui est adjacent à cet atome de N) par 1, 2 ou 3 substituants choisis indépendamment parmi un (1-4C)alkyle, un amino, un (1-4C)alkylamino, un (1-4C)alcoxy, un (1-4C)alcoxycarbonyle et un halogène ;
Q est Q1 : 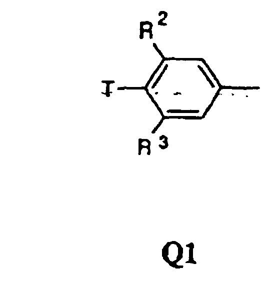 où R² et R³ sont indépendamment l'hydrogène ou un f luoro ;
où T est choisi parmi les groupes dans (TA) à (TC) ci-dessous (où AR1, AR2, AR2a, AR2b, CY1 et CY2 sont définis ci-après) :
(**TA**) T est choisi parmi les groupes suivants :
(**TAf**) un système de cycle hétéroaryle à 5 chaînons lié par N (totalement insaturé), contenant 1, 2 ou 3 atomes d'azote choisis dans un groupe de formule (TAf1) à (TAf6) : 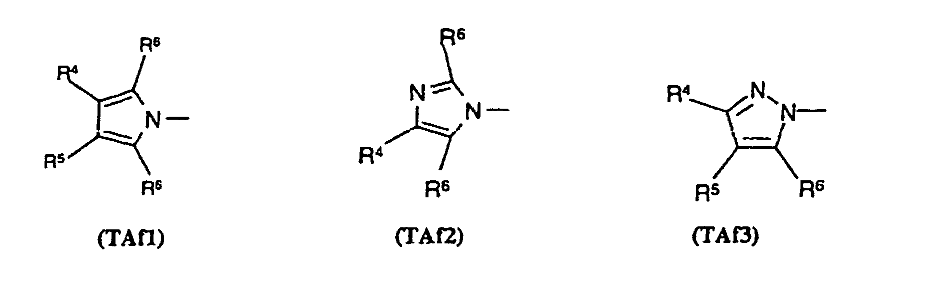 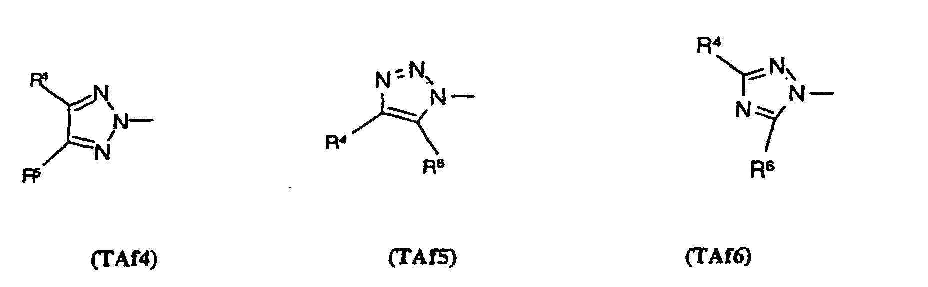
dans lesquelles :
R⁶ est choisi (indépendamment le cas échéant) parmi l'hydrogène, un (1-4C)alkyle, un (1-4C)alcoxycarbonyle, un (1-4C)alcanoyle, un carbamoyle et un cyano ;
R⁴ et R⁵ sont choisis indépendamment parmi l'hydrogène, un halogéno, un trifluorométhyle, un cyano, un nitro, un (1-4C)alcoxy, un (1-4C)alkyl-S(O)_{q}- (q est 0, 1 ou 2), un (1-4C)alcanoyle, un (1-4C)alcoxycarbonyle, un (2-4C)alcanoyloxy(1-4C)alkyle, un benzoxy-(1-4C)alkyle, un (2-4C)alcanoylamino, -CONRvRw, -NRvRw et un (1-4C)alkyle (éventuellement substitué par un hydroxy, un trifluorométhyle, un cyano, un nitro, un (1-4C)alcoxy, un (1-4C)alkylS(O)_{q}- (q est 0, 1 ou 2), un (1-4C)alcoxycarbonyle, un (1-4C)alcanoylamino, -CONRvRw, -NRvRw ; où RvRw est l'hydrogène ou un (1-4C)alkyle ; Rw est l'hydrogène ou un (1-4C)alkyle} ; ou
(**TB**) T est choisi parmi les groupes suivants :
(**TBa**) un halogéno ou un (1-4C)alkyle
{éventuellement substitué par un ou plusieurs groupes choisis chacun indépendamment parmi un hydroxy, un (1-4C)alcoxy, un (1-4C)alcanoyle, un cyano, un halogéno, un trifluorométhyle, un (1-4C)alcoxycarbonyle, -NRvRw, un (1-6C)alcanoylamino, un (1-4C)alcoxycarbonylamino, un N-(1-4C)alkyl-N-(1-6C)alcanoylamino, un (1-4C)alkylS(O)_{q}- (q est 0, 1 ou 2), CY1, CY2 ou AR1} ;
**(TBb)** -NRv¹Rw¹ ;
**(TBc)** un éthényle, un 2-(1-4C)alkyléthényle, un 2-cyanoéthényle, un 2-cyano-2-((1-4C)alkyl)éthényle, un 2-nitroéthényle, un 2-nitro-2-((1-4C)-alkyl)éthényle, un 2-((1-4C)alkylaminocarbonyl)-éthényle, un 2-((1-4C)alcoxycarbonyl)éthényle, un 2-(AR1)éthényle, un 2-(AR2)éthényle ;
(**TBd**) R¹⁰CO-, R¹⁰S(O)_{q}- (q est 0, 1 ou 2) ou R¹⁰CS- où R¹⁰ est choisi parmi les groupes suivants :
(***TBda***) CY1 ou CY2 ;
(***TBdb***) l'hydrogène, un (1-4C)alcoxycarbonyle, un trifluorométhyle, -NRvRw, un éthényle, un 2-(1-4C)alkyléthényle, un 2-cyanoéthényle, un 2-cyano-2-((1-4C)alkyl)éthényle, un 2-nitroéthényle, un 2-nitro-2-((1-4C)alkyl)éthényle, un 2-((1-4C)alkylaminocarbonyl)éthényle, un 2-((1-4C)-alcoxycarbonyl)éthényle, un 2-(AR1)éthényle ou un 2-(AR2)éthényle ; ou
(***TBdc***) un (1-4C)alkyle {éventuellement substitué comme défini dans (TBa) ci-desssus, ou par un (1-4C)alkylS(O)ₚNH- ou un (1-4C)alkylS(O)ₚ- ((1-4C)alkyl)N- (p est 1 ou 2)} ;
où Rv est l'hydrogène ou un (1-4C)alkyle ; Rw est l'hydrogène ou un (1-4C)alkyle ; Rv¹ est l'hydrogène, un (1-4C)alkyle ou un (3-8C)-cycloalkyle ; Rw¹ est l'hydrogène, un (1-4C)alkyle, un (3-8C)cycloalkyle, un (1-4C)alkylCO- ou un (1-4C)alkylS(O)_{q}- (q est 1 ou 2) ; ou
(TC) T est choisi dans les groupes (TC1) à (TC4) suivants : 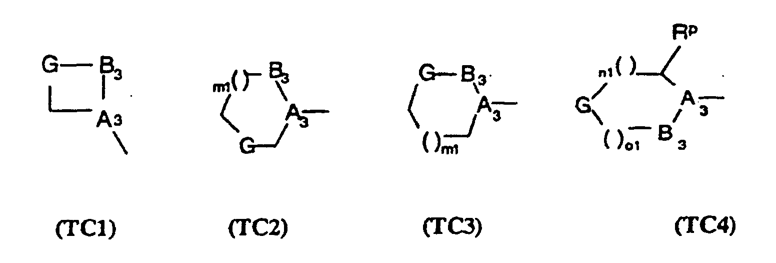 où dans (TC1) : >A₃-B₃- est >C (Rq) -CH (Rr) - et G est -O-, -S-, -SO-, -SO₂- ou >N(Rc) ;
où dans (TC2) : m1 est 0, 1 ou 2 ; >A₃-B₃- est >C=C(Rr)- ou >C(Rq)-CH(Rr)- et G est -O-, -S-, -SO-, -SO₂- ou >N(Rc) ;
où dans (TC3) : m1 est 0, 1 ou 2 ; >A₃-B₃- est >C(Rq)-CH(Rr)- (autre que lorsque Rq et Rr sont tous deux l'hydrogène) et G est -O-, -S-, -SO-, -SO₂- ou >N(Rc) ;
où dans (TC4) : n1 est 1 ou 2 ; ol est 1 ou 2 et n1 + ol = 2 ou 3 ; >A₃-B₃- est >C=C(Rr)- ou >C(Rq)-CH(Rr)- ou >N-CH₂- et G est -O-, -S-, -SO-, -SO₂-
ou >N(Rc) ; Rp est l'hydrogène, un (1-4C)alkyle (autre que lorsqu'une telle substitution est définie par >A₃-B₃-), un hydroxy, un (1-4C)alcoxy ou un (1-4C)alcanoyloxy ;
où dans (TC1), (TC2) et (TC4) : m1, n1 et ol sont tels que définis précédemment :
>A₃-B₃- est >N-CH₂- et G est >C(R¹¹)(R¹²), >C=O, >C-OH, >C- (1-4C)alcoxy, >C=N-OH, >C=N-(1-4C)alcoxy, >C=N-NH-(1-4C)alkyle, >C=N-N((1-4C)alkyle)₂ (les deux derniers groupes (1-4C)alkyle ci-dessus dans G étant éventuellement substitués par un hydroxy) ou >C=N-N-CO-(1-4C)alcoxy ; où > représente deux simples liaisons ;
Rq est l'hydrogène, un hydroxy, un halogéno, un (1-4C)alkyle ou un (1-4C)alcanoyloxy ;
Rr est (indépendamment le cas échéant) l'hydrogène ou un (1-4C)alkyle ;
R¹¹ est l'hydrogène, un (1-4C)alkyle, un fluoro(1-4C)alkyle, un (1-4C)alkylthio(1-4C)alkyle ou un hydroxy-(1-4C)alkyle et R¹² est - [C(Rr)(Rr)]ₘ₂-N(Rr)(Rc) où m2 est 0, 1 ou 2 ;
et, autre que la substitution sur le cycle définie par G, >A₃-B₃- et Rp, chaque système de cycle peut être éventuellement encore substitué sur un atome de carbone non adjacent au lien à >A₃- par jusqu'à deux substituants choisis indépendamment parmi un (1-4C)alkyle, un fluoro(1-4C)alkyle (dont un trifluorométhyle), un (1-4C)alkylthio(1-4C)alkyle, un hydroxy-(1-4C)alkyle, un amino, un amino-(1-4C)alkyle, un (1-4C)alcanoylamino, un (1-4C)alcanoylamino-(1-4C)alkyle, un carboxy, un (1-4C)alcoxycarbonyle, un AR-oxyméthyle, un AR-thiométhyle, un oxo(=O) (autre que lorsque G est >N-Rc et Rc est le groupe (Rc2)) ou choisi indépendamment parmi Rc ; et également un hydroxy
ou un halogéno (les deux derniers substituants éventuels seulement lorsque G est -O- ou -S-) ;
où
AR est un phényle éventuellement substitué ; un phényl(1-4C)alkyle éventuellement substitué ; un naphtyle éventuellement substitué ; un hétéroaryle à 5 ou 6 chaînons éventuellement substitué, étant un cycle aryle à 5 ou 6 chaînons où 1, 2 ou 3 des atomes du cycle sont choisis parmi l'azote, l'oxygène et le soufre et les cycles sont totalement aromatiques ; le système de cycle hétéroaryle bicyclique 5/6 ou 6/6 éventuellement substitué étant un système de cycle bicyclique aromatique comprenant un cycle à 6 chaînons condensé sur soit un cycle à 5 chaînons soit un autre cycle à 6 chaînons, le système de cycle bicyclique contenant de 1 à 4 hétéroatomes choisis parmi l'azote, l'oxygène et le soufre où les systèmes de cycle hétéroaryle bicycliques peuvent être liés par un atome dans l'un ou l'autre des cycles comprenant le système bicyclique, et où les deux systèmes de cycle hétéroaryle mono- et bicycliques sont liés par l'intermédiaire d'un atome de carbone du cycle et peuvent être (partiellement) hydrogénés ;
où AR est éventuellement mono- ou disubstitué par un halogéno, un (1-4C)alkyle, un hydroxy, un nitro, un carbamoyle, un (1-4C)alkylcarbamoyle, un di((1-4C)alkyl)carbamoyle, un cyano, un trifluorométhyle, un trifluorométhoxy, un amino, un (1-4C)alkylamino, un di((1-4C)alkyl)amino, un (1-4C)alkylS(O)_{q}- (q est 0, 1 ou 2), un carboxy, un (1-4C)alcoxycarbonyle, un (2-4C)alcényle, un (2-4C)alcynyle, un (1-4C)alcanoyle, un (1-4C)alcoxy, un (1-4C)alkylS(O)₂amino, un (1-4C)alcanoylamino, un benzoylamino, un benzoyle, un phényle (éventuellement substitué par jusqu'à trois substituants choisis parmi un halogéno, un (1-4C)alcoxy ou un cyano), un furane, un pyrrole, un pyrazole, un imidazole, un triazole, une pyrimidine, un pyridazine, une pyridine, un isoxazole, un oxazole, un isothiazole, un thiazole, un thiophène, un hydroxyimino(1-4C)alkyle, un (1-4C)alcoxyimino(1-4C)-alkyle, un hydroxy(1-4C)alkyle, un halogéno(1-4C)-alkyle, un nitro(1-4C)alkyle, un amino(1-4C)-alkyle, un cyano(1-4C)alkyle, un (1-4C)-alcanesulfonamido, un aminosulfonyle, un (1-4C)-alkylaminosulfonyle et un di((1-4C)alkyl)-aminosulfonyle ;
où Rc est choisi parmi les groupes (Rc1) à (Rc5) :
(***Rc1***) un (1-6C)alkyle {éventuellement substitué par un ou plusieurs groupes (1-4C)alcanoyle (dont une disubstitution géminée) et/ou éventuellement monosubstitué par un cyano, un (1-4C)alcoxy, un trifluorométhyle, un (1-4C)alcoxycarbonyle, un phényle (éventuellement substitué comme pour AR1 défini ci-après), un (1-4C)alkylS(O)_{q}- (q est 0, 1 ou 2) ; ou, sur tout sauf le premier atome de carbone de la chaîne (1-6C)alkyle, éventuellement substitué par un ou plusieurs groupes (dont une disubstitution géminée) choisis chacun indépendamment parmi un hydroxy et un f luoro, et/ou éventuellement monosubstitué par un oxo, -NRvRw [où Rv est l'hydrogène ou un (1-4C)alkyle ; Rw est l'hydrogène ou un (1-4C)alkyle], un (1-6C)alcanoylamino, un (1-4C)alcoxycarbonylamino, un N-(1-4C)alkyl-N-(1-6C)alcanoylamino, un (1-4C)alkylS(O)ₚNH- ou un (1-4C)alkylS(O)ₚ-((1-4C)alkyl)N- (p est 1 ou 2)} ;
(***RC2***) R¹³CO-, R¹³SO₂- ou R¹³CS-,
où R¹³ est choisi parmi (Rc2a) à (Rc2e) :
(***R2ca***) AR1, AR2, AR2a, AR2b, CY1, CY2 ;
(***R2cb***) l'hydrogène, un (1-4C)alcoxycarbonyle, un trifluorométhyle, -NRvRw [où Rv est l'hydrogène ou un (1-4C)alkyle ; Rw est l'hydrogène ou un (1-4C)-alkyle], un éthényle, un 2-(1-4C)alkyléthényle, un 2-cyanoéthényle, un 2-cyano-2-((1-4C)alkyl)-éthényle, un 2-nitroéthényle, un 2-nitro-2-((1-4C)alkyl)éthényle, un 2-((1-4C)alkylaminocarbonyl)éthényle, un 2-((1-4C)alcoxycarbonyl)-éthényle, un 2-(AR1)éthényle, un 2-(AR2)éthényle, un 2-(AR2a)éthényle ;
***(Rc2c)*** un (1-10C)alkyle
{éventuellement substitué par un ou plusieurs groupes (dont une disubstitution géminée) choisis chacun indépendamment parmi un hydroxy, un (1-10C)alcoxy, un (1-4C)alcoxy-(1-4C)alcoxy, un (1-4C)alcoxy-(1-4C)alcoxy-(1-4C)alcoxy, un (1-4C)-alcanoyle et un amino ; et/ou éventuellement substitué par un groupe choisi parmi un carboxy, un phosphonate, [un phosphono, -P(O)(OH)₂, et les dérivés mono- et di-(1-4C)alcoxy de celui-ci], un phosphinate [-P(OH)₂ et les dérivés mono- et di(1-4C)alcoxy de celui-ci], un phosphoryle [-0-P(O)(OH)₂ et les dérivés mono- et di-(1-4C)alcoxy de celui-ci], un phosphiryle [-O-P(OH)₂ et les dérivés mono- et di-(1-4C)alcoxy de celui-ci], un cyano, un halogéno, un trifluorométhyle, un (1-4C)alcoxycarbonyle, un (1-4C)alcoxy-(1-4C)alcoxycarbonyle, un (1-4C)alcoxy-(1-4C)alcoxy-(1-4C)-alcoxycarbonyle, un (1-4C)alkylamino, un di((1-4C)alkyl)amino, un (1-6C)alcanoylamino, un (1-4C)alcoxycarbonylamino, un N-(1-4C)alkyl-N-(1-6C)alcanoylamino, un (1-4C)alkylaminocarbonyle, un di-((1-4C)alkyl)aminocarbonyle, un (1-4C)-alkylS(O)ₚNH-, un (1-4C)alkylS(O)ₚ-((1-4C)alkyl)N-, un fluoro(1-4C)alkylS(O)ₚNH-, un fluoro(1-4C)-alkylS(O)ₚ((1-4C)alkyl)N-, un (1-4C)alkylS(O)_{q}-, CY1, CY2, AR1, AR2, AR1-O-, AR2-O-, AR1-S(O)_{q}-, AR2-S(O)_{q}-, AR1-NH-, AR2-NH- (p est 1 ou 2 et q est 0, 1 ou 2), et également AR2a, AR2b, des versions de groupes contenant AR2} ;
***(Rc2d)*** un R¹⁴C(O)O(1-6C)alkyle où R¹⁴ est AR1, AR2, un (1-4C)alkylamino, un benzyloxy(1-4C)alkyle ou un (1-10C)alkyle {éventuellement substitué comme défini pour (Rc2c)} ;
(***Rc2e***) R¹⁵O- où R¹⁵ est un benzyle, un (1-6C)alkyle {éventuellement substitué comme défini pour (Rc2c)}, CY1, CY2 ou AR2b ;
(***Rc4***) un trityle, AR1, AR2, AR2a, AR2b ;
où
**AR1** est un phényle éventuellement substitué ou un naphtyle éventuellement substitué ;
**AR2** est choisi parmi un furane, un pyrrole, un thiophène, un pyrazole, un imidazole, une pyridine, une pyrimidine, une pyrazine, une pyridazine, un 1,2,3- et 1,2,4-triazole, un tétrazole, un oxazole, un isoxazole, une oxazine, un thiazole, un isothiazole, un 1,2,4- et 1,3,4-thiadiazole ;
**AR2a** est choisi parmi un dihydropyrrole et une tétrahydropyridine ;
AR2b est choisi parmi un tétrahydrofurane, une pyrrolidine, une morpholine, une thiomorpholine, une pipérazine, une imidazoline, une pipéridine, un 1,3-dioxolan-4-yle, un 1,3-dioxan-4-yle, un 1,3-dioxan-5-yle et un 1,4-dioxan-2-yle ;
**CY1** est un cycle cyclobutyle, cyclopentyle ou cyclohexyle éventuellement substitué ;
CY2 est un cycle cyclopentényle ou cyclohéxényle éventuellement substitué ;
où :
les substituants éventuels sur AR1, AR2, AR2a, AR2b, CY1 et CY2 sont (sur un atome de carbone disponible) jusqu'à trois substituants choisis indépendamment parmi un (1-4C)alkyle {éventuellement substitué par des (de préférence un) substituants choisis indépendamment parmi un hydroxy, un trifluorométhyle, un (1-4C)alkylS(O)_{q}- (q est 0, 1 ou 2) (ce dernier substituant de préférence sur AR1 uniquement), un (1-4C)alcoxy, un (1-4C)alcoxycarbonyle, un cyano, un nitro, un (1-4C)alcanoylamino, -CONRvRw ou -NRvRw), un trifluorométhyle, un hydroxy, un halogéno, un nitro, un cyano, un thiol, un (1-4C)alcoxy, un (1-4C)alcanoyloxy, un diméthylaminométhylèneaminocarbonyle, un di(N-(1-4C)alkyl)aminométhylimino, un carboxy, un (1-4C)alcoxycarbonyle, un (1-4C)alcanoyle, un (1-4C)alkylSO₂amino, un (2-4C)alcényle {éventuellement substitué par un carboxy ou un (1-4C)alcoxycarbonyle), un (2-4C)alcynyle, un (1-4C)alcanoylamino, un oxo (=O), un thioxo (=S), un (1-4C)alcanoylamino (le groupe (1-4C)alcanoyle étant éventuellement substitué par un hydroxy), un (1-4C)alkylS(O)_{q}- (q est 0, 1 ou 2) {le groupe (1-4C)alkyle étant éventuellement substitué par un ou plusieurs groupes choisis indépendamment parmi un cyano, un hydroxy, et un (1-4C)alcoxy), -CONRvRw ou -NRvRw [où Rv est l'hydrogène ou un (1-4C)alkyle ; Rw est l'hydrogène ou un (1-4C)alkyle].

2. Composé de formule (I), ou un sel pharmaceutiquement acceptable de celui-ci, selon la revendication 1, **caractérisé en ce que** T est choisi dans les groupes (TAf) et (TC) tels que définis dans la revendication 1.

3. Composé de formule (I), ou un sel pharmaceutiquement acceptable de celui-ci, selon l'une quelconque des revendications 1 à 2, **caractérisé en ce que** les groupes dans (TC) sont définis par les formules (TC5) à (TC11) : 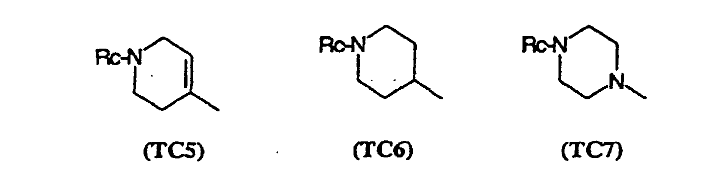 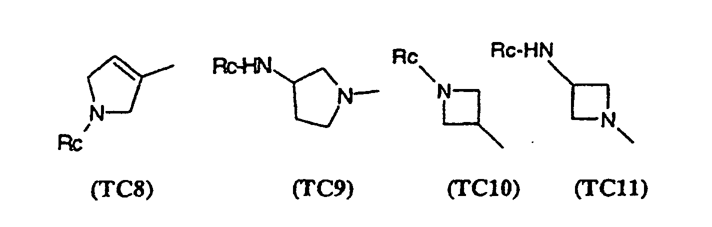 dans lesquelles Rc est tel que défini dans la revendication 1.

4. Composé de formule (I), ou un sel pharmaceutiquement acceptable de celui-ci, selon l'une quelconque des revendications 1 à 3,
**caractérisé en ce que** le composé est l'énantiomère pur représenté dans la formule (IA).

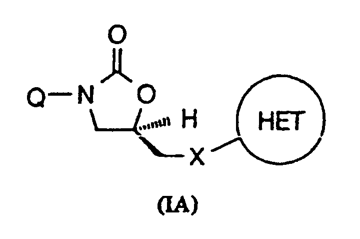

5. Composé de formule (I) selon la revendication 1, **caractérisé en ce qu'**il s'agit d'un composé de formule (IB), ou un sel pharmaceutiquement acceptable de celui-ci : 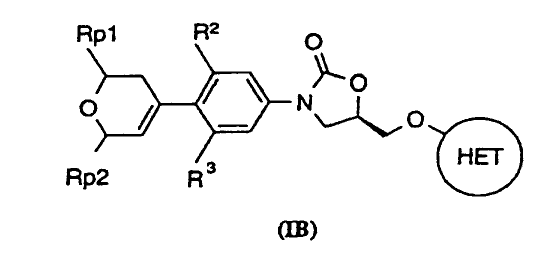 où HET est un pyridin-2-yle ou un pyrazin-2-yle (notamment un pyridin-2-yle) ; R² et R³ sont indépendamment l'hydrogène ou un fluoro ; et Rp1 et Rp2 sont indépendamment l'hydrogène, un hydroxy, un bromo, un (1-4C)alkyle, un carboxy, un (1-4C)alcoxycarbonyle, un hydroxyméthyle, un (1-4C)alcoxyméthyle ou un carbamoyle.

6. Composé selon la revendication 1, **caractérisé en ce qu'**il s'agit de :
5(R)-pyrid-2-yloxyméthyl-3-(4-(4-(5-cyanopyrid-2-yl)pipérazin-1-yl)-3-fluorophényl)oxazolidin-2-one ;
5(R)-(6-chloropyridazin-3-yloxyméthyl)-3-(4-(4-(5-cyanopyrid-2-yl)pipérazin-1-yl)-3-fluorophényl)-oxazolidin-2-one ;
5(R)-pyrazin-2-yloxyméthyl-3-(4-(4-(5-cyanopyrid-2-yl)pipérazin-1-yl)-3-fluorophényl)oxazolidin-2-one ;
5(R)-pyrimidin-4-yloxyméthyl-3-(4-(4-(5-cyanopyrid-2-yl)pipérazin-1-yl)-3-fluorophényl)-oxazolidin-2-one ;
5(R)-pyridazin-3-yloxyméthyl-3-(4-(4-(5-cyanopyrid-2-yl)pipérazin-1-yl)-3-fluorophényl)-oxazolidin-2-one ;
5(R)-pyrid-2-yloxyméthyl-3-(3-fluoro-4-(3,6-dihydro-(2H)-pyran-4-yl)phényl)oxazolidin-2-one ;
5(R)-(4-méthylpyrid-2-yloxyméthyl-3-(3-fluoro-4-(3,6-dihydro-(2H)-pyran-4-yl)phényl)oxazolidin-2-one ;
5(R)-(3-méthylpyrid-2-yloxyméthyl-3-(3-fluoro-4-(3,6-dihydro-(2H)-pyran-4-yl)phényl)oxazolidin-2-one ;
5(R)-(6-chloropyridazin-3-yloxyméthyl)-3-(3-fluoro-4-(3,6-dihydro-(2H)-pyran-4-yl)phényl)oxazolidin-2-one ;
5(R)-pyridazin-3-yloxyméthyl-3-(3-fluoro-4-(3,6-dihydro-(2H)-pyran-4-yl)phényl)oxazolidin-2-one ;
5(R)-pyrimidin-4-yloxyméthyl-3-(3-fluoro-4-(3,6-dihydro-(2H)-pyran-4-yl)phényl)oxazolidin-2-one ;
5(R)-pyrazin-2-yloxyméthyl-3-(3-fluoro-4-(3,6-dihydro-(2H)-pyran-4-yl)phényl)oxazolidin-2-one ;
5(R)-(5-chloropyridin-2-yloxyméthyl)-3-(3-fluoro-4-(3,6-dihydro-(2H)-pyran-4-yl)phényl)oxazolidin-2-one ; ou les sels pharmaceutiquement acceptables de celui-ci.

7. Composé selon la revendication 1, **caractérisé en ce qu'**il s'agit de :
5(R)-pyrid-2-yloxyméthyl-3-(3-fluoro-4-(3,6-dihydro-(2H)-pyran-4-yl)phényl)oxazolidin-2-one ;
5(R)-pyrazin-2-yloxyméthyl-3-(3-fluoro-4-(3,6-dihydro-(2H)-pyran-4-yl)phényl)oxazolidin-2-one ;
ou les sels pharmaceutiquement acceptables de celui-ci.

8. Procédé de préparation d'un composé de formule (I) selon la revendication 1 ou les sels pharmaceutiquement acceptables de celui-ci, lequel procédé comprend de **(a)** à **(f)** :
**(a)** la modification d'un substituant ou l'introduction d'un substituant dans un autre composé de formule (I) ;
**(b)** la réaction d'un composé de formule (II) 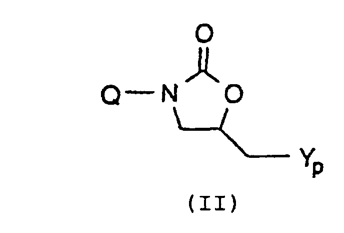 dans laquelle Yp est un hydroxy avec un composé de formule (b1) HET-OH ou (b2) HET-Lg, dans laquelle Lg est un groupe partant approprié ;
**(c)** la réaction d'un composé de formule (II) dans laquelle Yp est un groupe partant avec un composé d'alcoxyde métallique de formule HET-OM dans laquelle M est un métal alcalin, ou un autre métal connu pour favoriser la O-alkylation ;
**(d)** la réaction d'un composé de formule Q-Zp dans laquelle Zp est un groupe isocyanate ou amine avec un époxyde de formule CH₂(O)CH-CH₂O-HET ;
**(e)** lorsque X est -S- en utilisant un procédé analogue au procédé (c) en utilisant (e1) un composé de thioxyde métallique de formule HET-SM dans laquelle M est un métal alcalin, ou un autre métal connu pour favoriser la S-alkylation ; ou en utilisant (e2) HET-SH et un composé de formule (II) dans laquelle Yp est un groupe partant approprié ;
**(f)** la réaction d'un composé uréthane de formule (III) avec un composé de formule (IV) 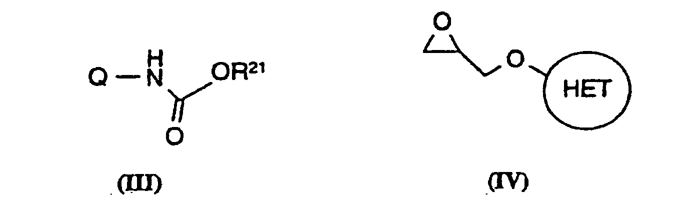
dans laquelle R²¹ est un (1-6C)alkyle ou un benzyle ; et ensuite le cas échéant :
(i) l'élimination de tout groupement protecteur ;
(ii) la formation d'un sel pharmaceutiquement acceptable.

9. Composé de formule (I) selon les revendications 1 à 7, ou un sel pharmaceutiquement acceptable de celui-ci, destiné à être utilisé comme médicament.

10. Utilisation d'un composé de formule (I) selon les revendications 1 à 7, ou un sel pharmaceutiquement acceptable de celui-ci, dans la fabrication d'un médicament destiné à être utilisé dans la production d'un effet antibactérien chez un animal à sang chaud.

11. Composition pharmaceutique comprenant un composé de formule (I) selon les revendications 1 à 7, ou un sel pharmaceutiquement acceptable de celui-ci, et un diluant ou un support pharmaceutiquement acceptable.
